# EUROPEAN PATENT SPECIFICATION

(11) **EP 4 379 725 B1**
(45) Date of publication and mention of the grant of the patent: **02.12.2026**
(21) Application number: 24152024.6
(22) Date of filing: 10.07.2015
(51) Int. Cl.: G16B 20/00, G16B 20/30, G16B 20/50, G16B 30/00, G16B 50/00, G01N 33/68

(54) **IMMUNE RECOGNITION MOTIFS**
IMMUNERKENNUNGSMOTIVE
MOTIFS DE RECONNAISSANCE IMMUNITAIRE

(30) Priority: 11.07.2014 US 201462023212 P; 08.09.2014 US 201462047385 P; 01.12.2014 US 201462085792 P; 01.12.2014 US 201462085773 P
(43) Date of publication of application: 05.06.2024
(62) Document number(s) of the earlier application(s) in accordance with Art. 76 EPC: 15818218.8 / 3 167 396
(73) Proprietor: Iogenetics, LLC., Madison, Wisconsin 53704 (US)
(72) Inventor: BREMEL, Robert D., Hillpoint, Wisconsin, 53937 (US); HOMAN, Jane, Hillpoint, Wisconsin, 53937 (US); IMBODEN, Michael, Madison, Wisconsin, 53711 (US)
(74) Representative: Algemeen Octrooi- en Merkenbureau B.V.

(56) References cited:
- C. KING ET AL: "Removing T-cell epitopes with computational protein design", PROCEEDINGS NATIONAL ACADEMY OF SCIENCES PNAS, vol. 111, no. 23, 19 May 2014 (2014-05-19), US, pages 8577 - 8582, XP055414871, ISSN: 0027-8424, DOI: 10.1073/pnas.1321126111
- A. PATRONOV ET AL: "T-cell epitope vaccine design by immunoinformatics", JOURNAL OF MEDICINAL CHEMISTRY, vol. 42, no. 22, 8 January 2013 (2013-01-08), pages 4650 - 120139, XP055256399, ISSN: 0022-2623, DOI: 10.1098/rsob.120139
- ANONYMOUS WILLIAM W. ET AL: "Frequency of epitope specific naïve CD4+ T cells correlates with immunodominance in the human memory repertoire - PMC", vol. 188, no. 6, 15 March 2012 (2012-03-15), US, pages 2537 - 2544, XP093188326, ISSN: 0022-1767, Retrieved from the Internet <URL:https://www.ncbi.nlm.nih.gov/pmc/articles/PMC3997369/> [retrieved on 20240722], DOI: 10.4049/jimmunol.1102190
- ANONYMOUS WILLIAM W. ET AL: "Frequency of epitope specific naïve CD4+ T cells correlates with immunodominance in the human memory repertoire - PMC", THE JOURNAL OF IMMUNOLOGY, vol. 188, no. 6, 15 March 2012 (2012-03-15), pages 2537 - 2544, XP93188326, ISSN: 0022-1767, Retrieved from the Internet <URL:https://www.ncbi.nlm.nih.gov/pmc/articles/PMC3997369/> DOI: 10.4049/jimmunol.1102190

## Description

### BACKGROUND OF THE INVENTION

The adaptive immune system is comprised of antibodies, generated by B-cells, and cell mediated immunity which depends on the recognition by T-cells of peptides bound in the major histocompatibility molecules and presented on the surface of antigen presenting cells (APCs). Many cell types may serve as APCs, but primarily they comprise dendritic cells, macrophages, and B-cells (the so called "professional" APCs). While each B-cell becomes dedicated to the production of one antibody sequence, that sequence is derived as the product of somatic hypermutation (SHM) of the genes in that B-cell that encode the immunoglobulin variable region. The presentation of peptides bound in MHC molecules is a function of cleavage of polypeptides by various endosomal peptidases, including but not limited to cathepsins, and the competitive binding of peptides to genetically defined MHC molecules. Hence, the presentation to antigens by the immune system is the combination of stochastic, innate, and genetically determined events.

The primary function of the adaptive immune system is to differentiate self from not-self and to allow the body to mount an appropriate response to molecules, once identified as self or as not-self. When a familiar self-antigen is recognized, the desired outcome is down-regulation of the immune response or tolerance. When an unfamiliar non-self-antigen is encountered, the appropriate outcome is usually a robust up-regulation to yield an immune response in which cytokine responses enlist additional cellular responses to remove the foreign stimulus and protect the integrity of the host.

The discrimination between self and not-self is largely dependent on the T-cell responses and is the combination of the host's genetically determined MHC molecules in combination with motifs comprised in peptides which are bound by MHC molecules and exposed to T-cells in the context of the MHC molecules.

There is a need to be able to predict peptide sequences which comprise motifs that are likely to be recognized by T-cells, and to identify those motifs which are likely to give rise to down-regulation or suppression of the immune response (sometimes termed "Tregitopes" [1]) and those which are likely to result in up-regulation or activation of the immune response (T-helper activity). Having the ability to identify T cell epitopes most likely to upregulate or downregulate an immune response then enables the design of a number of interventions including the design of vaccines with improved properties and the ability to specifically target T cell populations and to reduce or eliminate such populations.

Document D1 (C. KING ET AL, "Removing T-cell epitopes with computational protein design"): This document relates to a computational protein design method that reduces immunogenicity by identifying and eliminating T-cell epitopes while maintaining protein structure and function.

Document D2 (A. PATRONOV ET AL, "T-cell epitope vaccine design by immunoinformatics"): This document relates to a review of different sequence-based and structure-based immunoinformatics methods used for the in silico design and analysis of T-cell epitope vaccines.

Document D3 (William W. ET AL, "Frequency of epitope specific naïve CD4+ T cells correlates with immunodominance in the human memory repertoire"): This document relates to a study demonstrating that the frequency of epitope-specific naïve T-cells in unexposed individuals correlates with the immunodominance of those epitopes in the memory T-cell repertoire after vaccination.

### SUMMARY OF THE INVENTION

The present invention provides methods and systems for identifying and classifying epitopes and use of that information to analyze proteins and peptides within proteins, especially potential epitopes, and to use the information to design synthetic peptides and proteins, analyze biopharmaceutical proteins, and diagnose autoimmune conditions. Peptides which are bound in MHC grooves comprise two sets of amino acids: those that face inwards into the groove and determine the binding affinity to the MHC molecule (the groove exposed motifs or GEM) and those which do not interact with the groove but rather are on the obverse side exposed outwardly to the T-cells (the T-cell exposed Motifs or TCEM). The present invention utilizes information related to the identity and physiochemical characteristics of the GEM and TCEM as described in more detail below.

Using the methods described herein, it is possible to categorize any set of peptides, irrespective of whether their source is from antibody molecules, molecules from pathogens, or from biotherapeutic molecules, or any other protein of interest. Whether a peptide bound within a pMHC will be an up-regulating or down-regulating epitope, is a product of (a) its binding affinity (and hence dwell time) in the context of the host MHC allele, and (b) the frequency with which T-cells have been previously exposed to that motif. Common TCEMs with high affinity GEMs would be expected to overstimulate and lead to down-regulation, immunosuppression, or tolerance. By combining predictions of pMHC binding affinities and endosomal processing with databases of TCEM motif frequencies based on amino acid sequence, it is possible to create databases to cross reference molecules from different origins in order to make predictions as to their immunogenic potential as components of vaccines, and the role of the motifs as possible sources of auto-immune responses or allergic responses, or as suppressive T-cell responses.

Accordingly, the present invention provides methods for evaluating peptides derived from any source protein and for ranking peptides based on their likelihood of upregulating or downregulating the cellular and humoral immune response. It further provides compositions of peptides designed to elicit a particular immune response and also a means of specifically targeting T cells.

In some embodiments the application of the methods described herein provides a scoring system for ranking peptides based on their frequency of occurrence in a large reference database of peptide motifs. In some embodiments the reference database is that derived from immunoglobulin variable regions including both the germline and somatically hypermutated variable region sequences. Immunoglobulin constant regions provide another source of reference proteins. In yet other embodiments the reference database is that derived from the human proteome other than immunoglobulins. In alternative embodiments the reference database is made up of T cell receptor sequences. The present invention provides a method of categorizing TCEMs in target proteins by identifying TCEMS in such proteins by means of application of a computer algorithm and then categorizing such TCEMs relative to their frequency of occurrence in a reference database of the immunoglobulins or proteome. Another application is to categorize the TCEMs in target proteins relative to their frequency of occurrence in a database of comparable target proteins. Accordingly the invention provides for application of computer algorithms to categorize TCEMs relative to databases of allergens and microorganisms (including but not limited to pathogens and the commensal human microbiome and including but not limited to bacteria, viruses, fungi and parasites). The categorization approach allows for scoring of frequency of occurrence and determination of common and rare TCEMs. This scoring system provides an indicator of whether a given amino acid TCEM motif, when encountered in a protein of interest, is likely to give rise to upregulation or downregulation of the immune response provided certain other conditions are fulfilled. A preferred condition is whether the peptide has a high probability of being bound with high affinity to the MHC molecule of the alleles of the given host and in the competitive context of its protein of origin while at the same time displaying a specific motif for recognition by a T-cell (helper or Treg). A further preferred condition is whether the peptide of interest is likely to be excised by an endosomal peptidase to allow it to be bound by an MHC molecule. As a TCEM is the product of binding of a peptide in the groove of either an MHC I or MHC II molecule, it follows that the present invention provides a means of categorizing peptides and TCEMs bound to either type of MHC and thus resulting in binding to either CD4+ or CD8+ T cells. As the amino acid positions of peptides bound in MHC I and MHC II molecules are different, the invention provides for evaluation of TCEMs comprising different amino acid positions determined by the binding positions in the MHC molecular groove, including but not limited to those described by Rudolph *et al,* as described in further detail below. In particular embodiments the invention provides for scoring based on frequency classes, and metrics for determining the relative frequency of occurrence of any particular TCEM with respect to the reference database of interest.

Every TCEM has a complimentary groove exposed motif or GEM which determines the binding affinity of the peptide to the MHC. In another embodiment therefore the present invention allows determination of the binding affinity of the peptide for a particular MHC allele or set of alleles and hence addresses another key factor in determining the dwell time of the peptide in conjunction with the MHC. Having identified both GEM and TCEM motifs, it follows that the present invention enables manipulation of either.

By application of the scoring system for TCEMs, described herein, in a further embodiment, the invention provides a ranking system to identify immunosuppressive epitopes (sometimes called Tregitopes). In further embodiments, a set of T-cell exposed motifs (TCEM) are identified which occur with high frequency in immunoglobulin variable regions of both germline and mutated immunoglobulin variable regions. These are discontinuous amino acid motifs representing the positions exposed to T-cells when a peptide is bound in an MHC groove. These are motifs, which because of the frequency of their occurrence, in combination with their affinity, are likely to give rise to immunosuppressive responses when they are also bound by MHC molecules. In specific embodiments described herein, a number of peptides may be identified which are high frequency occurrences, and which also have a high frequency of high affinity binding across a broad array of MHC molecules and thus are examples of probable down-regulatory or suppressive epitopes. Some of these TCEM arise in immunoglobulin germline sequences. Yet other TCEM arise in high frequency in mutated immunoglobulin sequences. Within this group it is possible to further categorize into those most frequently found associated with GEMS that are of high, medium, or lower binding affinity for a permuted population of 65 MHC alleles.

In yet further embodiments, the invention described herein provides a method to apply the ranking system to identify epitopes which have a high probability of stimulating the immune response because they are rare in occurrence and which, provided the other conditions of MHC binding affinity and endopeptidase cleavage are fulfilled, have a high likelihood of generating a stimulatory rather than suppressive immune response.

In additional embodiments the invention provides for design and expression of synthetic proteins in which modifications are made to the TCEM by means of changing one or more amino acids to alter the frequency categorization of the TCEM, or similarly the changing of the affinity of MHC binding by changing of amino acids in the GEMs. A further modification which may be made in the course of designing and expressing a synthetic protein is to alter the amino acids flanking the MHC binding peptide which carries the TCEM, in order to provide a greater probability of excision by cathepsin necessary to allow presentation of the TCEM of interest by an antigen presenting cell. By these means the embodiments include introduction of amino acid sequences which do not normally occur in conjunction with a particular TCEM but which result in a particularly desired outcome.

A further source of TCEMs exposed to the immune response is the immunoglobulin constant region. Each class or isotype of immunoglobulin constant region (IgM, IgA, IgD, IgE, IgG and the subclasses thereof) comprises a different sequence and hence a different set of TCEM motifs. Within each class the sequences, and hence motifs, are highly conserved. As shown herein, the repertoire of motifs found in the constant regions are different from those found in the variable region. It appears therefore that motifs in the constant region are excluded from use in the variable region. Constant region motifs - as they are indeed constant - are the most frequent class of TCEM motifs derived from immunoglobulins. It follows therefore that when these motifs are also high binding affinity they may stimulate Tregulatory or suppressor responses. In one embodiment, therefore, the present invention enables identification of constant region TCEM motifs which have high predicted binding affinity to various MHC alleles and are predicted to have a Tregulatory function when bound by these MHC alleles.

In particular embodiments the invention provides for the construction of a non transitory computer readable medium in which the database developed to categorize TCEMs is stored and the application of computer algorithms and code to the manipulation of said database to compare the frequency of TCEMs in a target protein of interest. The invention similarly provides a system for analysis of target proteins by application of a process and memory into which target protein sequences are entered and algorithms executed to identify TCEMs and compare the frequency of these with the frequency of occurrence in a reference database of reference proteins. As described above, it follows that the databases of interest include immunoglobulins of both germline and mutated origin, immunoglobulin constant regions and human proteome proteins, and databases of target proteins including but not limited to allergens, microorganisms and indeed any group of target proteins of interest. The aforesaid algorithms provide means to analyze peptides binding to either MHC I or MHC II and the corresponding populations of binding T cells.

The terms upregulation and downregulation are used herein to describe the outcome of a T-cell pMHC-interaction, because any interaction between a T-cell and a pMHC that results in binding may be considered "immunostimulation". However, not all such events result in an active cascade of cytokines and cellular responses leading to up-regulation of the immune system and leading towards destruction of the target. Some binding interactions result in a cytokine response which diminishes the immune response and shutdown of the cellular responses or down-regulation; this appears to occur most when such immunostimulation is too frequent or too prolonged. Hence, when confronted with a self-protein the appropriate outcome is likely down-regulation (avoidance of self-destruction). An adverse reaction to a self-protein may therefore be by inappropriate upregulation or by the removal of down regulation. Furthermore, the presence in pathogen proteins of motifs which occur in high frequency in self proteins and which bind with high affinity in the protein of interest will tend to have a down regulating effect.

Autoimmune responses can arise when T-cell responses are stimulated by amino acid motifs occurring in pathogens and other environmental or "non self" or exogenous sources and result in the direction of the immune response to the same T-cell motifs where they also occur in self proteins, leading to a disequilibrium in the normal response to that self-protein. A further embodiment of the present invention is thus to provide a method of identifying motifs in non-self-proteins and scanning self-proteins to identify matching TCEM motifs which may become the target of autoimmune responses. This has application, including but not limited to, in the diagnosis and management of autoimmune diseases. In yet further embodiments, this also provides for a method to apply an important evaluative step to biotherapeutic and vaccine proteins to assist in avoidance of adverse reactions. In a further embodiment, therefore, the invention addresses the identification of epitope mimics which can give rise to adverse reactions including but not limited to autoimmune disease.

In a further embodiment of the present invention, characteristic patterns of T-cell motifs found in immunoglobulins are determined. In comparison with the pattern of frequency found in normal immunoglobulin variable regions, patterns of motif frequency are also identified which are associated with specific immunopathologies. In B-cell pathologies, the normal patterns of somatic hypermutation are disrupted and the balance of common and rare TCEM motifs changes. Hence, the application of the methods described herein, when applied to analyze the immunoglobulins of a patient, can provide assistance in the diagnosis of immunopathologies. Examples include, but are not limited to, immunopathologies such as lymphomas, leukemias, lupus erythematosus, multiple sclerosis and others. Further by identifying the mechanism in which the immunopathology arises, the analytical methods provided herein facilitate the design of interventions.

Overall therefore the invention described herein provides a means to collate and categorize the discontinuous amino acid motifs exposed to T-cells which may result in regulation and direction of the cellular immune response. Like the immune system itself, the methods described herein draw on the vast variety of motifs found in immunoglobulin variable regions as a reference database of motif frequency. In one particular embodiment therefore the invention provides for the assembly of databases which may be used as references for categorizing TCEMs and GEMs by extraction of such motifs from immunoglobulins, proteins of the proteome or groups of target proteins of interest, through the application of computer algorithms and assembly of a computer readable database.

The invention further provides for the design and expression of synthetic peptides and polypeptides which embody particular features in their TCEMs and GEMs and flanking cathepsin cleavage sites that add desired functionality and specificity of binding affinity or cleavage. Such features are determined by applications of the invention and used to design peptides and poly peptides for expression. In yet further embodiments the invention applied such properties to modify and design synthetic peptides which embody TCEMs identified in certain target proteins. In particular examples of these the target proteins of interest are tumor associated proteins, including but not limited to those associated with melanoma such as MART, MAGE and PMEL. Other proteins of interest in which TCEMs are identified and may be incorporated into synthetic peptides or polypeptides in which GEMs or flanking cathepsin cleavage sites have been modified to provide functionality, include myelin basic protein and the core protein of hepatitis B virus. Also in particular embodiments the modified synthetic peptides and poly peptides may be applied to patients to upregulate or down regulate the immune response to the TCEM of interest.

A further embodiment of the present invention is the application of peptides comprising TCEMs of determined characteristics in conjunction with synthetic MHC molecules linked to a further peptide of particular functionality. Thus a TCEM can be designed to bind to a given population of T cells and facilitate delivery of a toxin or a cytokine to such T cells. In a yet further embodiment the combination of chosen TCEM, MHC molecule and a further peptide enables the attachment of a pMHC combination as a means of binding a T cell population to a substrate such as but not limited to a purification matrix, a tissue culture plate or bead or to a cell or liposome.

In some embodiments the production of synthetic proteins comprising TCEMs comprising certain desired features is limited to a single TCEM. In yet other embodiments, the synthetic protein may comprise a multiplicity of TCEMs. In particular embodiments, the many TCEMs may be linked by linker sequences which may provide for the inclusion of cathepsin cleavage sites of particular functionality.

In a particular embodiment the production of synthetic polypeptides comprising TCEMs is directed to the production of compositions made up of only a limited number of amino acids. In yet further embodiments such synthetic polypeptides may be applied to the treatment of autoimmune diseases including but not limited to multiple sclerosis, systemic lupus erythematosis, myasthenia gravis, rheumatoid arthritis, Alzheimer's disease, diabetes, and Parkinson's disease. In such instances the synthetic polypeptide delivers TCEMs to the patient designed to down regulate the immune response or tolerize.

A number of biopharmaceutical products have been developed which have the ability to reduce the count of T-regulatory T cells. One example is ipilimumab, which binds to the CTLA4 receptors on T cells and inhibits T-regulatory T cell function. The effect is, however, directed to all Treg cells and does not discriminate those which are tumor specific. The invention described herein identifies Treg binding peptides which are specific to tumor proteins. In one example described above, these are TCEM motifs identified in HPV oncoproteins. In another embodiment, the TCEM motifs are MART or HBV associated motifs. However, it should be understood that these examples are non-limiting and the same approach applies to any oncoprotein in which a TCEM of interest is identified.

In some embodiments, the combination of a MHC molecule and a TCEM bearing peptide can preferably be configured as a target ("bait") to specifically bind to T-cells and allow their elimination by a cytotoxin or targeted energy. In some embodiments, suitable TCEMs are selected based on the analysis of high frequency motifs in a protein of interest, and by reference to a database of TCEM frequencies, as probable Tregs. In other preferred embodiments, the TCEMs are TCEMs which elicit a strong immunostimulatory upregulation. In some preferred embodiments, a MHC and TCEM-bearing peptide combination which results in the specific reduction of upregulating T cell responses, for instance in autoimmunity, is provided.

Accordingly, in some embodiments, the present invention provides a MHC-TCEM bearing peptide complex. In some preferred embodiments, the complex is attached to or includes a cytotoxin, for example radionuclide or positron emitter. The cytotoxin may be attached to, or included in, either the MHC molecule or the peptide which will be presented to a patient's T-cells bound as a pMHC so that the cytotoxin (e.g. radionuclide or positron emitter) will inflict lethal damage to the specifically binding T-cell. The following examples of substances with may be linked to the peptide or MHC to damage the T-cell should not be considered limiting. In some preferred embodiments, an alpha emitter or Auger electron emitter may be attached to or included in the peptide. Among the radionuclides of particular interest are alpha emitters, including but not limited to, Bismuth 213, Actinium 225 and Lead 212. Alpha emitters produce a high ionization density radiation with a short pathway length suitable to target single cells. Another non-limiting example is emitters of Auger particles such as Gadolinium-67, Technicium-99, Indium-111, lodine-123, lodine-125, and Tellurium-201. These examples, which are considered non-limiting, are appropriate because such low energy emissions result in a short distance of travel and thus confine cell damage to the immediate vicinity of the radiolabel, enabling targeting to a specific T cell. Incorporation of the radionuclide may be by inclusion in the peptide itself (e.g., iodine bound to tyrosine) or by addition of tags to the terminal amino acids of the peptide.

In yet further embodiments, the peptide or MHC may be labelled or tagged by a positron emitter. Among non-limiting examples of positron emitters which may be used to label peptides, and their half-lives, are Carbon-11 (20.4 minutes), Nitrogen 13 (10 minutes), Fluorine 18 (110 minutes), Iron 52 (8.3 hours), Iodine 124 (4.3 days), Yttrium86 (14.7 hours), Gadolium68 (68 minutes) and Arsenic 72 (26 hours) (http://www.bnl.gov/medical/RCIBI/Sub-links/PositronEmitterTable.asp, http://www.kernchemie.uni-mainz.de/radiopharmazie-roesch/140_ENG_HTML.php)

In further embodiments, the peptide of interest may be labeled with a metallic particle such as gold or ferritin. When mounted in the MHC corresponding to the HLA of a patient and administered *in vivo,* the metallic particle may be targeted by an electromagnetic field as a means of destruction of the bound T cell.

In alternative embodiments, instead of a radionuclide, a non-radioactive cytotoxin is attached to the MHC molecule or to the peptide bearing the TCEM of interest. Among examples of such non-radioactive cytotoxins are RNAses, phospholipase, membrane active peptides such as cercropin, and diphtheria toxin.

In a particularly preferred embodiment, a cytotoxin or radionuclide tagged peptide which comprises a TCEM of high frequency, selected by reference to a reference database, is mutated to obtain a higher affinity of binding for a particular MHC. Such a mutated peptide is preferably assembled into a MHC molecule corresponding to the HLA allele of a specific patient and administered to that patient to bring about a desired reduction of a specific T cell population.

In further embodiments, a peptide of interest bearing a TCEM-bearing peptide is labeled with a radionuclide or with a non-radioactive label, such as but not limited to, colorimetric tags such as streptavidin or fluorescein. When assembled as a pMHC with MHC molecules corresponding to those of a specific patient the addition *in vitro* of PBMCs from that patient to the tagged pMHC can serve as an index or enumerator of the specific T cells in that patient. A preferred embodiment of this method comprises monitoring the presence of specific Tregs in a patient as an index of growth or metastasis of a tumor.

The production of soluble MHC molecules is known to those skilled in the art. In preferred embodiments, the MHC molecules are provided in a form suitable for patient administration. In some embodiments, soluble MHC are expressed in CHO cells as described herein.

In particularly preferred embodiments, secreted HLA molecules are constructed with the alpha or beta chain as a fusion with an immunoglobulin Fc region. The Fc region may be derived from IgG or IgA or IgM. In some embodiments, the cysteine cross bridges are removed to prevent assembly of the Fc regions as dimers. The presence of the Fc region enables detection using standard immunoassays (e.g., using an anti-human Fc detection antibody), it further provides a site for addition of tags, cytotoxins, and labels as described above.

Accordingly, the present invention provides methods for evaluating peptides derived from any source protein and for ranking the peptides within those proteins based on their likelihood of up-regulating or down-regulating the cellular immune response and various applications of such methods, including, but not limited to, in the evaluation of biopharmaceuticals, design of vaccines or immunotherapeutics, identification of epitope mimics, identification of autoimmune reactions and diagnosis of immunopathologies. It further provides compositions of peptides comprising characteristics most likely to result in various immunological outcomes, including those likely to lead to an immunosuppressive response. As a consequence the present invention provides for the modification of proteins of interest to remove immunosuppressive motifs or motifs predicted to cause excessive upregulation of the immune response. In yet further embodiments the present invention provides for the design of synthetic polypeptide and protein sequences which provide for interventions in a number of disease conditions.

In some embodiments, the present invention provides methods of designing an immunogen, comprising: categorizing the T-cell exposed motifs in a protein of interest according to their frequency of occurrence in reference to a reference database of reference proteins; determining the binding affinity of groove exposed motifs in the protein of interest and identifying those which have high binding affinity; identifying potential immunosuppressive peptides in the protein of interest; and synthesizing a synthetic isoform of the protein of interest in which the immunosuppressive peptide epitopes have been removed or modified by an amino acid substitution, deletion or other mutation to provide the immunogen. In some preferred, but not limiting, embodiments the protein of interest is an influenza protein or an Ebolavirus protein. In some embodiments, the Ebolavirus is selected from the group consisting of Zaire Ebolavirus, Sudan Ebolavirus, Cote D'lvoire Ebolavirus, Lake Victoria Marburg virus, and Guinea-Sierra Leone Ebolaviruses of 2014. In some embodiments, the Ebolavirus protein is selected from the group consisting of envelope glycoprotein, small secreted glycoprotein, VP24, and VP40 of an Ebolavirus. In some embodiments, the immunogen comprises a peptide of from 20-40 amino acids in length selected from the protein of interest. In some embodiments, the immunogen comprises less than 15 % of the amino acids of the wild type protein of interest. In some embodiments, the protein of interest is the Ebolavirus GP2 protein and said immunogen comprises a polypeptide of from 100 to 175 amino acids derived from said protein of interest. In some preferred embodiments, the protein of interest is an influenza hemagglutinin protein. In yet other embodiments the immunogen may be from another microbial pathogen, whether viral bacterial fungal or parasitic in origin, or a tumor related protein.

In some embodiments, the present invention provides a fusion protein. In some preferred embodiments the fusion protein comprises an Ebolavirus immunogen as described above. In some embodiments, the fusion protein comprises at least a portion of an immunoglobulin. In some embodiments, the fusion protein comprises a first polypeptide comprising a peptide of 20-40 amino acids derived from the protein of interest operatively linked to the N terminal of a portion of an immunoglobulin molecule. In some embodiments, the at least a portion of an immunoglobulin is linked to said immunogen peptide by a linker. In some embodiments, a first polypeptide comprising the immunogenic polypeptide derived from the protein of interest is operatively linked to the N terminal of an immunoglobulin heavy chain and a second polypeptide comprising the immunogenic polypeptide derived from the protein of interest is operatively linked to the N terminal of an immunoglobulin light chain. In some embodiments, three copies of a first polypeptide comprising the immunogenic polypeptide derived from said protein of interest are sequentially linked by a linker and the C terminal of the third copy of said immunogenic polypeptide is linked by a linker to the N terminal of an immunoglobulin heavy chain. In some embodiments, the linker comprises a plurality of glycines and serine residues. In some embodiments, the linker is from 4-10 amino acids in length. In some embodiments, the linker comprises a plurality of glycine and serine residues and is from 16-28 amino acids in length. In some embodiments, the linker is encoded by SEQ ID NO:435 or 436. In some embodiments, the immunoglobulin is murine immunoglobulin. In some embodiments, the immunoglobulin is human immunoglobulin. In some preferred embodiments, the fusion protein is encoded by a sequence selected from the group consisting of SEQ ID NOs:405 to 434. In yet other embodiments the fusion protein encodes a sequence derived from another microorganism of interest or a tumor related protein.

In some embodiments, the present invention provides a host cell expressing immunogen or fusion protein as described above.

In some embodiments, the present invention provides methods of making an immunotherapeutic comprising immunizing an animal with an immunogen or fusion protein as described above; harvesting cells and making hybridomas; extracting immunoglobulin variable region sequences from the hybridomas and constructing a murine-human chimeric antibody; and expressing the antibody in a host cell. The immunogen may be derived from a microorganism of interest including from the group comprising viruses, bacteria, fungi or parasites. In some embodiments, the present invention provides an immunotherapeutic made by the foregoing method. In some embodiments, the recombinant antibody is operatively linked to a microbiocide. In some embodiments, the microbiocide is virucidal. In some embodiments, the microbiocide is selected from the group consisting of defensins, cathelicidins, and phospolipases. In some embodiments, the immunotherapeutic neutralizes Ebola virus, in other cases the immunotherapeutic neutralizes another microorganism including from the group comprising viruses, bacteria, fungi or parasites.

In some embodiments, the present invention provides methods of treating a subject suspected of being infected with Ebolavirus by administering an immunotherapeutic as described above. In yet other embodiments the invention provides a method for treating a subject infected by another microorganism.

In some embodiments, the present invention provides a vaccine comprising an Ebolavirus immunogen or fusion protein as described above.

In some embodiments, the present invention provides a method of providing protection to a subject from infection by a microorganism. by immunization with a vaccine comprising an immunogen or fusion protein as described above. In some preferred embodiments the method is applied to protect the subject from Ebolavirus infection or influenza infection. In yet other embodiments protection is provided against infection by other microorganisms. In yet another embodiment the method provides a therapeutic benefit to an individual who is already infected by a microorganism or who is affected by cancer.

In some particularly preferred embodiments, the immunogen is from an influenza hemagglutinin. In some cases the T cell exposed motif in said influenza hemagglutinin is identified as occurring with high frequency in a reference database of reference proteins and the methods further comprise: identifying an alternate T-cell exposed motif in other similar hemagglutinins of the same hemagglutinin class wherein said alternate T cell exposed occurs with a low frequency in a reference database of reference proteins and substituting the high frequency motif with the low frequency motif. In some embodiments, the reference database of reference proteins comprises human immunoglobulin heavy and light chain variable regions. In some embodiments, the influenza hemagglutinin is from the group comprising H1, H2, H3, H5, and H7. In some embodiments, the alternate motif that is substituted occurs with a frequency of from 16 to 64-fold less frequently in the reference database of reference proteins as the motif which it substitutes. In some embodiments, the hemagglutinin with one or more substituted motifs is incorporated into a vaccine. In some embodiments, the hemagglutinin with one or more substituted motifs is expressed in a host cell. In some embodiments, the hemagglutinin with one or more substituted motifs is expressed in a host cell as a fusion peptide operatively linked with part or all of an immunoglobulin.

In some embodiments, the present invention provides methods to predict immune response to a peptide in a target protein comprising: establishing a reference database of T cell exposed motifs by assembling a database of reference proteins, extracting peptide sequences from the reference proteins, identifying T-cell exposed motifs in the peptide sequences by a computer implemented algorithm; and categorizing the T-cell exposed motifs based on the frequency of occurrence of the T-cell exposed motifs in the reference database of reference proteins; extracting peptide sequences from the target protein, identifying T-cell exposed motifs in the peptide sequences from the target protein by a computer implemented algorithm; and comparing T cell exposed motifs from the target protein to the frequency of occurrence of the same T cell motifs in the reference database.

In some embodiments, the database of reference proteins is selected from the group consisting of immunoglobulin variable regions, immunoglobulin constant regions, T cell receptor molecules, proteins of the human proteome other than immunoglobulins, allergens and microorganism proteins. In some embodiments, the database of reference proteins comprises at least 300 proteins. In some embodiments, the database of reference proteins comprises at least 5000 immunoglobulin variable region sequences. In some embodiments, the database of reference proteins comprising immunoglobulin variable regions comprises representatives of at least three immunoglobulin germline gene families. In some embodiments, the sequences in the database of reference proteins comprising immunoglobulin variable regions are identified as originating from germline or somatic mutated origin. In some embodiments, the sequences in the database of reference proteins comprising immunoglobulin variable regions are identified as originating from heavy chain or light chain origin.

In some embodiments, the configuration of amino acids in the T-cell exposed motifs comprise the 2,3,5,7,8 positions of a 15 amino acid peptide binding in a MHC-II groove which is numbered from -3 to +3. In some embodiments, the configuration of amino acids in the T-cell exposed motifs comprise the -1,3,5,7,8 positions of a 15 amino acid peptide binding in a MHC-II groove which is numbered from -3 to +3. In some embodiments, the configuration of amino acids in the T-cell exposed motifs comprise the 4,5,6,7,8 positions of a 9 amino acid peptide binding in a MHC-I groove.

In some embodiments, the categorizing further comprises determining whether the T-cell exposed motifs in the target protein occur with a frequency of greater than 1 in 8 in the reference database. In some embodiments, the categorizing further comprises determining whether the T-cell exposed motifs in the target protein occur with a frequency of greater than 1 in 64 in the reference database. In some embodiments, the categorizing further comprises determining whether the T-cell exposed motifs in the target protein occur with a frequency of greater than 1 in 1024 in the reference database. In some embodiments, the categorizing further comprises determining whether the T-cell exposed motifs in the target protein occur with a frequency of less than 1 in 1024 in the reference database. In some embodiments, the categorizing further comprises determining whether the T-cell exposed motifs in the target protein occur with a frequency of greater than the mean frequency of occurrence in the reference database. In some embodiments, the categorizing further comprises determining whether the T-cell exposed motifs in the target protein occur with a frequency of less than the mean frequency of occurrence in the reference database.

In some embodiments, the methods further comprise the step of characterizing the overall immunogenicity of the target protein by ranking of the frequency of occurrence of a plurality of the T-cell exposed motifs in the target protein. In some embodiments, the methods further comprise determining the predicted MHC binding affinity of the peptide sequences comprising the T-cell exposed motifs derived from the target protein by determining the MHC allele-specific binding of the groove exposed motifs corresponding to each T-cell exposed motif. In some embodiments, the methods further comprise identifying the peptide sequences within the target protein which have T-cell exposed motifs with a high frequency of occurrence in a reference database of immunoglobulin variable regions and which have groove exposed motifs associated with the T-cell exposed motifs that have a predicted high binding affinity to one or more MHC alleles. In some embodiments, the methods further comprise
peptide sequences within the target protein that are predicted immunosuppressive T-cell epitopes.. In some embodiments, the methods further comprise identifying the peptide sequences within the target protein which have T-cell exposed motifs with a low frequency of occurrence in a reference database immunoglobulin variable regions and which have groove exposed motifs associated with the T-cell exposed motifs that have a high predicted binding affinity. In some embodiments, the methods further comprise identifying the peptide sequences within the target protein that lead to upregulation of the immune response.. In some embodiments, the methods further comprise designing modifications in a peptide selected from the target protein wherein the modifications are selected to produce a result selected from the group consisting of (a) a lower or higher frequency of representation as compared to the reference database of reference proteins, and (b) increased or decreased MHC binding affinity of the selected peptide, and expressing a synthetic polypeptide comprising the modified peptide and purifying the synthetic polypeptide.. In some embodiments, the methods further comprise selecting one or more peptide sequences having a desired frequency of T-cell exposed motif representation as compared to the reference database of reference proteins from the target protein and synthesizing a nucleic acid construct comprising the one or more peptide sequences in operable association with one or more sequences that do not naturally occur with the one or more peptide sequences. In some embodiments, the methods further comprise expressing the protein product of the nucleic acid construct. In some embodiments, the methods further comprise purifying the protein encoded by the nucleic acid construct.

In some embodiments, the present invention provides methods to predict cross reaction of T-cell binding comprising: establishing a reference database of T cell exposed motifs by assembling a multiplicity of databases of reference proteins, extracting peptide sequences from the reference proteins, and identifying T-cell exposed motifs in the peptide sequences by a computer implemented algorithm; categorizing the T-cell exposed motifs based on the frequency of occurrence of the T-cell exposed motifs in the multiplicity of databases of reference proteins, and comparing the frequency of occurrence of the T cell exposed motifs in more than one of the multiplicity of databases of reference proteins.

In some embodiments, the present invention provides a non-transitory computer readable medium comprising: a database of peptides comprising T-cell exposed motifs categorized based on the frequency of occurrence of the T-cell exposed motifs in a reference database of reference proteins, wherein the database retains the identity of the peptide from which the T cell exposed motif is obtained in the reference database of reference proteins; computer executable code that upon execution allows entry of target protein sequences; computer executable code that upon execution extracts target peptide sequences from the target protein sequences and identifies T-cell exposed motifs in the target peptide sequences; and computer executable code that compares the T-cell exposed motifs in the target peptide sequences to the database of T-cell exposed motifs to determine the frequency of representation of the T-cell exposed motifs in the target peptide sequences as compared to the reference database of reference proteins.

In some embodiments, the database of reference proteins is selected from the group consisting of immunoglobulin variable regions, immunoglobulin constant regions, T cell receptor molecules, proteins of the human proteome other than immunoglobulins, allergens and microorganism proteins. In some embodiments, the reference database of reference proteins comprises at least 300 proteins. In some embodiments, the reference database of reference proteins comprises at least 5000 immunoglobulin variable region sequences. In some embodiments, the reference database of reference proteins comprising immunoglobulin variable regions comprises representatives of at least three immunoglobulin germline gene families. In some embodiments, the sequences in the reference database of reference proteins comprising immunoglobulin variable regions are identified as originating from germline or somatic mutated origin. In some embodiments, the sequences in the reference database of reference proteins comprising immunoglobulin variable regions are identified as originating from heavy or light chains. In some embodiments, the database of peptides comprising T cell exposed motifs is further categorized according to the probability of the peptide from which the T cell exposed motif is derived binding to a MHC allele. In some embodiments, the database of peptides comprising T cell exposed motifs is further categorized according to the predicted MHC binding affinity of the groove exposed motif comprised within the peptide from which the T cell exposed motif is derived.

In some embodiments, the configuration of amino acids in the T-cell exposed motifs comprise the 2,3,5,7,8 positions of a 15 amino acid peptide binding in a MHC-II groove which is numbered from -3 to +3. In some embodiments, the configuration of amino acids in the T-cell exposed motifs comprise the -1,3,5,7,8 positions of a 15 amino acid peptide binding in a MHC-II groove which is numbered from -3 to +3. In some embodiments, the configuration of amino acids in the T-cell exposed motifs comprise the 4,5,6,7,8 positions of a 9 amino acid peptide binding in a MHC-I groove.

In some embodiments, the target protein sequences are biopharmaceutical protein sequences. In some embodiments, the target protein sequences are microbial protein sequences. In some embodiments, the target protein sequences are mammalian protein sequences. In some embodiments, the target protein sequences are selected from the group consisting of non-mammalian protein sequences, arthropod protein sequences, and plant protein sequences.

In some embodiments, the present invention provides systems for analysis of target proteins comprising: a processor and memory, wherein the processor and memory are configured to (i) assemble a reference database of reference proteins, extract peptide sequences from the reference proteins; identify T-cell exposed motifs in the peptide sequences by a computer implemented algorithm; and categorize the T-cell exposed motifs based on the frequency of occurrence of the T-cell exposed motifs in the reference database of reference proteins so as to establish a database of peptides categorized by their constituent T cell exposed motif frequency; (ii) extract target peptide sequences from a target protein of interest; (iii) identify T-cell exposed motifs in the target peptide sequences from the target protein of interest by a computer implemented algorithm; and (iv) compare the T-cell exposed motifs in the target peptide sequences to the database of T-cell exposed motifs categorized based on the frequency of occurrence of the T-cell exposed motifs in the reference database of reference proteins to determine the frequency of representation of the T-cell exposed motifs in the target peptide sequences as compared to the reference database of reference proteins.

In some embodiments, the target protein sequences are selected from the group consisting of biopharmaceutical protein sequences, microbial protein sequences, mammalian protein sequences, non-mammalian protein sequences, arthropod protein sequences, and plant protein sequences. In some embodiments, the processor and memory are further configured to determine the affinity of binding of the target peptide sequences to one or more MHC alleles.

In some embodiments, the configuration of amino acids in the T-cell exposed motifs comprise the 2,3,5,7,8 positions of a 15 amino acid peptide binding in a MHC-II groove which is numbered from -3 to +3. In some embodiments, the configuration of amino acids in the T-cell exposed motifs comprise the -1,3,5,7,8 positions of a 15 amino acid peptide binding in a MHC-II groove which is numbered from -3 to +3. In some embodiments, the configuration of amino acids in the T-cell exposed motifs comprise the 4,5,6,7,8 positions of a 9 amino acid peptide binding in a MHC-I groove.

In some embodiments, the reference database of reference proteins is selected from the group consisting of immunoglobulin variable regions, immunoglobulin constant regions, T cell receptor molecules, proteins of the human proteome other than immunoglobulins, allergens and microorganism proteins. In some embodiments, the reference database of reference proteins comprises at least 300 proteins. In some embodiments, the reference database of reference proteins comprises at least 5000 immunoglobulin variable region sequences. In some embodiments, the reference database of reference proteins comprising immunoglobulin variable regions comprises representatives of at least three immunoglobulin germline gene families. In some embodiments, the database of reference proteins comprising immunoglobulin variable regions comprises sequences that are identified as originating from germline or somatic mutated origin. In some embodiments, the reference database of reference proteins comprising immunoglobulin variable regions comprises sequences that are identified as originating from heavy or light chain origin.

In some embodiments, the present invention provides methods of evaluating a target protein for peptide sequences with motifs that may induce adverse immune responses comprising: identifying T-cell exposed motifs in target peptide sequences in the target protein and determining the frequency of occurrence of the T-cell exposed motifs by comparison with a reference database of T-cell exposed motifs; identifying groove exposed motifs associated with T-cell exposed motifs in target peptide sequences in the target protein based on the frequency of representation in the reference database; determining the predicted binding affinity of the target peptide sequences comprising the groove exposed motifs and identifying MHC binding based on the predicted binding affinity to the target peptide sequences comprising the groove exposed motifs; and evaluating the combination of frequency and binding affinity as an indicator of potential to cause an adverse immune response.

In some embodiments, the peptide sequences in the target protein comprise a combination selected from the group consisting of (a) a T-cell exposed motif with a high frequency of representation in the reference database and a high predicted binding affinity to an MHC allele likely to induce immunosuppression, and (b) a T-cell exposed motif with low frequency of representation in the reference database and a high predicted binding affinity to an MHC allele likely to induce a strong upregulation of the immune response. In some embodiments, the target protein is selected from the group consisting of biopharmaceutical proteins and vaccines.

In some embodiments, the present invention provides methods of modifying the immune response to a protein of interest comprising applying the method of claim 55 to identify a protein comprising peptides with potential adverse motifs and modifying the protein of interest by changing one or more amino acids in the potential adverse motifs and further expressing the modified protein as a synthetic protein product.

In some embodiments, the present invention provides methods of identifying a potential epitope mimic in a protein of interest comprising: identifying the T-cell exposed motifs in target peptide sequences in a first protein of interest and determining the frequency of occurrence of the T-cell exposed motifs in a reference database to identify T-cell exposed motifs which are present at high frequency in the reference database; identifying groove exposed motifs associated with the T-cell exposed motifs in the first protein of interest that have a high frequency of representation in the reference database; determining the predicted binding affinity of the target peptide sequences from the first protein of interest comprising the groove exposed motifs; and repeating the foregoing steps on a second protein of interest to identify identical T-cell exposed motifs associated with a same or different groove exposed motif; wherein the first protein is an exogenous protein and the second protein is an endogenous or self-protein and wherein exposure to the first protein results in an adverse immune response directed to the second protein.

In some embodiments, the present invention provides methods of modifying the immune response to a protein of interest comprising applying the method described above to identify peptides which are potential epitope mimics based on the T-cell exposed motif and/or groove exposed motif compositions and modifying the motifs by changing one or more amino acids in the motifs in the protein of interest. In some embodiments, the epitope mimic is identified as an autoimmune disease target. In some embodiments, the methods further comprise using information from the process to inform a diagnosis for an autoimmune disease.

In some embodiments, the present invention provides methods of diagnosing an immunopathology comprising: obtaining a blood sample from a subject suspected of being affected by an immunopathology; determining the sequence of a multiplicity of immunoglobulin sequences in the blood sample; determining a frequency distribution pattern of T-cell exposed motifs in the immunoglobulins in the blood sample; comparing the frequency distribution of T cell exposed motifs with a reference frequency distribution characteristic an immunopathology; and providing a diagnosis based on the comparison. In some embodiments, the immunopathology is selected from the group consisting of lymphoma, leukemia, lupus erythematosus, and multiple sclerosis. In some embodiments, the reference database is compiled from immunoglobulins sequenced from individuals with an immunopathology.

In some embodiments, the present invention provides a synthetic polypeptide comprising a T-cell exposed motif that occurs with a desired frequency of representation as compared to the frequency of occurrence in a reference database of reference proteins and wherein the amino acids in the counterpart groove exposed motif of the T cell exposed motif are selected to provide a desired profile of binding affinity to at least one selected MHC allele.

In some embodiments, the present invention provides a synthetic polypeptide comprising a T-cell exposed motif that occurs with a desired frequency of representation as compared to a reference database of reference proteins and wherein the amino acids in the counterpart groove exposed motif are selected to provide high affinity binding to at least one selected MHC allele. In some embodiments, the present invention provides a synthetic polypeptide comprising a T-cell exposed motif that occurs with a desired frequency of representation as compared to a reference database of reference proteins and wherein the amino acids in the counterpart groove exposed motif are selected to provide low affinity binding to at least one selected MHC allele. In some embodiments, the present invention provides a synthetic polypeptide comprising a first peptide of 9-23 amino acids which comprises a T-cell exposed motif that occurs with a desired frequency in a reference database of reference proteins and wherein the first peptide containing a T-cell exposed motif is flanked on both sides by flanking peptide sequences selected to provide probability of >0.5 of excision of the first peptide by endosomal peptidases.

In some preferred embodiments, the synthetic polypeptide is operably associated with an exogenous sequence.

In some embodiments, the configuration of amino acids in the T-cell exposed motif comprises the 2,3,5,7,8 positions of a 15 amino acid peptide binding in a MHC-II groove which is numbered from -3 to +3. In some embodiments, the configuration of amino acids in the T-cell exposed motifs comprise the -1,3,5,7,8 positions of a 15 amino acid peptide binding in a MHC-II groove which is numbered from -3 to +3. In some embodiments, the configuration of amino acids in the T-cell exposed motifs comprise the 4,5,6,7,8 positions of a 9 amino acid peptide binding in a MHC-I groove. In some embodiments, the synthetic polypeptide has one or more mutations in a T cell exposed motif or groove exposed motif as compared to the corresponding wild-type T cell motif. In some embodiments, the one or more mutations is at a site selected from the group consisting of the 2,3,5,7,8 positions of a 15 amino acid peptide binding in a MHC-II groove which is numbered from -3 to +3, the - 1,3,5,7,8 positions of a 15 amino acid peptide binding in a MHC-II groove which is numbered from -3 to +3, the 4,5,6,7,8 positions of a 9 amino acid peptide binding in a MHC-I groove, the -3,2,-1,1,4,6,9,+1,+2,+3 positions of a 15 amino acid peptide binding in a MHC-II groove which is numbered from -3 to +3, the -3,2,1,2,4,6,9,+1,+2,+3 positions of a 15 amino acid peptide binding in a MHC-II groove which is numbered from -3 to +3, and the 1,2,3,9 positions of a 9 amino acid peptide binding in a MHC-I groove.

In some embodiments, the reference database of reference proteins is selected from the group consisting of immunoglobulin variable regions, immunoglobulin constant regions, T cell receptor molecules, proteins of the human proteome other than immunoglobulins, allergens and microorganism proteins. In some embodiments, the desired frequency of T-cell exposed motif occurrence in the reference database is greater than 1 in 1024 T-cell exposed motifs and the database comprises at least 5000 proteins. In some embodiments, the desired frequency of T-cell exposed motif occurrence is greater than the mean frequency in the reference database. In some embodiments, the desired frequency of T-cell exposed motif occurrence is less than the mean frequency in the reference database. In some embodiments, the binding to at least one selected MHC allele is to more than 4 alleles. In some embodiments, the flanking sequences comprise 4-10 amino acids.

In some embodiments, the synthetic polypeptide is immunosuppressive. In some embodiments, the synthetic polypeptide upregulates the immune response.

In some embodiments, the T-cell exposed motif is derived from a reference database of tumor associated antigens. In some embodiments, the tumor associated antigen is from the group comprising MART, MAGE antigens, PMEL. In some embodiments, the peptide comprising the T-cell exposed motif is selected from the group consisting of SEQ ID NOs: 119, 121, 123, 124, 126, 128, 130, 132 and 134, and wherein X represents any amino acid. In some embodiments, the peptide comprising the T-cell exposed motif is selected from the group consisting of SEQ ID NOs: 118, 120, 122, 125, 127, 129, 131 and 133.

In some embodiments, the T-cell exposed motif is derived from a viral protein. In some embodiments, the protein is selected from the group of viral proteins consisting hepatitis B core protein, and human papilloma virus proteins E6 and E7. In some embodiments, the peptide comprising the T-cell exposed motif is selected from the group consisting of SEQ ID NOs:237-285, and wherein X represents any amino acid. In some embodiments, the peptide comprising the T-cell exposed motif is selected from the group consisting of SEQ ID NOs: 259-278 and 467.

In some embodiments, the T cell exposed motif is derived from myelin basic protein. In some embodiments, the peptide comprising the T-cell exposed motif is selected from the group consisting of SEQ ID NOs: 136, 138 and 140, wherein X represents any amino acid. In some embodiments, the peptide comprising the T-cell exposed motif is selected from the group consisting of SEQ ID NOs: 135, 137 and 139.

In some embodiments, the present invention provides a purified biopharmaceutical protein preparation comprising a synthetic polypeptide as described above.

In some embodiments, the present invention provides a vaccine comprising a synthetic polypeptide as described above.

In some embodiments, the present invention provides methods of designing a synthetic polypeptide encoding an immunogen comprising: categorizing the T-cell exposed motifs in a protein of interest according to their frequency of occurrence in reference to a reference database of reference proteins; determining the binding affinity of groove exposed motifs in the protein of interest and identifying those which have high binding affinity; identifying potential immunosuppressive peptides in the protein of interest; and synthesizing a synthetic polypeptide comprising an isoform of the protein of interest in which the immunosuppressive peptide epitopes have been removed or modified by an amino acid substitution, deletion or other mutation.

In some embodiments, the potential immunosuppressive epitopes have been modified to reduce their immunosuppressive function by a modification selected from the group consisting of changing one or more amino acids in the groove exposed motif to reduce the binding affinity, changing one or more T-cell exposed motif amino acids to prevent binding to T cells, and changing one or more amino acids flanking within 4 amino acids of either side of the peptide to reduce the probability of excision. In some embodiments, a peptide sequence containing an immunosuppressive T-cell exposed motif is removed from the synthetic polypeptide comprising an isoform of the protein.

In some embodiments, the reference database of reference proteins is selected from the group consisting of immunoglobulin variable regions, immunoglobulin constant regions, T cell receptor molecules, proteins of the human proteome other than immunoglobulins, allergens and microorganism proteins.

In some embodiments, the immunogen is a component of a vaccine. In some embodiments, the immunogen comprises a peptide of from 20-40 amino acids in length selected from the protein of interest. In some embodiments, the immunogen comprises less than 15% of the amino acids of the wild type protein of interest. In some embodiments, the protein of interest is a tumor associated antigen. In some embodiments, the tumor associated antigen is from the group comprising MART, MAGE antigens, PMEL. In some embodiments, the protein of interest is a viral protein. In some embodiments, the viral protein is from the group comprising hepatitis B and human papillomavirus. In some embodiments, the potential immunosuppressive peptides in the protein of interest are selected from the group consisting of SEQ ID NOs: 119, 121, 123, 124, 126, 128, 130, 132, 134 and 237 to 285. In some embodiments, the protein of interest is an influenza hemagglutinin protein. In some embodiments, the T cell exposed motif in the influenza hemagglutinin is identified as occurring with high frequency in a reference database of reference proteins and further comprising: identifying an alternate T-cell exposed motif in other similar hemagglutinins of the same hemagglutinin class wherein the alternate T cell exposed occurs with a low frequency in a reference database of reference proteins; and substituting the high frequency motif with the low frequency motif. In some embodiments, the influenza hemagglutinin is from the group comprising H1, H2, H3, H5, and H7. In some embodiments, the alternate motif that is substituted occurs with a frequency of from 16 to 64-fold less frequently in the reference database of reference proteins as the motif which it substitutes. In some embodiments, the protein of interest is an Ebolavirus protein. In some embodiments, the Ebolavirus is selected from the group consisting of Zaire Ebolavirus, Sudan Ebolavirus, Cote D'lvoire Ebolavirus, Lake Victoria Marburg virus, and Guinea-Sierra Leone Ebolaviruses of 2014. In some embodiments, the Ebolavirus protein is selected from the group consisting of envelope glycoprotein, small secreted glycoprotein, VP24, and VP40 of an Ebolavirus. In some embodiments, the protein of interest is the Ebolavirus GP2 protein and the immunogen comprises a polypeptide of from 100 to 175 amino acids derived from the protein of interest.

In some embodiments, the present invention provides **a** synthetic polypeptide encoding an immunogen produced by the methods described above.

In some embodiments, the present invention provides a fusion protein comprising the synthetic polypeptide encoding an immunogen as described above. In preferred embodiments, the synthetic polypeptide is placed in fame or in operable association with a peptide sequence not naturally associated with the synthetic polypeptide.

In some embodiments, the fusion protein comprises at least a portion of an immunoglobulin. In some embodiments, the fusion protein comprises a first polypeptide comprising a peptide of 20-40 amino acids derived from the protein of interest operatively linked to the N terminal of a portion of an immunoglobulin molecule. In some embodiments, the at least a portion of an immunoglobulin is linked to the immunogen peptide by a linker. In some embodiments, a first polypeptide comprising the immunogenic polypeptide derived from the protein of interest is operatively linked to the N terminal of an immunoglobulin heavy chain and a second polypeptide comprising the immunogenic polypeptide derived from the protein of interest is operatively linked to the N terminal of an immunoglobulin light chain. In some embodiments, three copies of a first polypeptide comprising the immunogenic polypeptide derived from the protein of interest are sequentially linked by a linker and the C terminal of the third copy of the immunogenic polypeptide is linked by a linker to the N terminal of an immunoglobulin heavy chain. In some embodiments, the linker comprises a plurality of glycine and serine residues. In some embodiments, the linker is from 4-10 amino acids in length. In some embodiments, the linker comprises a plurality of glycine and serine residues and is from 16-28 amino acids in length. In some embodiments, the linker is encoded by SEQ ID NO:435 or 436.

In some embodiments, the present invention provides a vaccine comprising a fusion protein as described above.

In some embodiments, the fusion protein is encoded by a sequence selected from the group consisting of SEQ ID NOs:405-434.

In some embodiments, the present invention provides methods of providing protection to a subject from infection by Ebolavirus by immunization with a vaccine comprising an immunogen or fusion protein as defined above.

In some embodiments, the present invention provides a host cell expressing a synthetic polypeptide or fusion protein as described above.

In some embodiments, the present invention provides methods of making an immunotherapeutic comprising immunizing an animal with the synthetic polypeptide encoding an immunogen or fusion protein as defined above, the method comprising harvesting cells and making hybridomas; extracting immunoglobulin variable region sequences from the hybridomas and constructing a humanized or murine-human chimeric antibody; and expressing the antibody in a host cell.

In some embodiments, the present invention provides an immunotherapeutic made by the foregoing method. In some embodiments, the recombinant antibody is operatively linked to a microbiocide. In some embodiments, the microbiocide is virucidal. In some embodiments, the microbiocide is selected from the group consisting of defensins, cathelicidins, and phospholipases. In some embodiments, the immunotherapeutic neutralizes Ebola virus. In some embodiments, the present invention provides a method of treating a subject suspected of being infected with Ebolavirus by administering the immunotherapeutic.

In some embodiments, the present invention provides a synthetic polypeptide which is an isoform of a protein of interest in which one or more immunosuppressive epitopes have been identified and functionally eliminated by the method described above. In some embodiments, the potential immunosuppressive epitopes have been modified to preclude their immunosuppressive function by a modification selected from the group consisting of changing one or more amino acids in the groove exposed motif to reduce the binding affinity; changing one or more T cell exposed motif amino acids to prevent binding to T cells; and changing one or more amino acids flanking within 4 amino acids of either side of the peptide to reduce the probability of excision. In some embodiments, an immunosuppressive epitope peptide identified in the natural isoform has been identified and excluded from the sequence of the synthetic isoform. In some embodiments, the present invention provides a vaccine comprising the synthetic polypeptide as described above.

In some embodiments, the present invention provides a polypeptide composition comprising a first synthetic peptide comprising a T-cell exposed motif that occurs with a desired frequency of representation compared to a reference database of reference proteins and a second peptide or polypeptide comprising a MHC molecule subunit in operable association with a third peptide or polypeptide which does not naturally occur with the second peptide and wherein the first synthetic peptide comprises a groove exposed motif that binds to the MHC molecule of the second peptide or polypeptide with high affinity . In some embodiments, the present invention provides a polypeptide composition comprising a first synthetic peptide comprising a MHC molecule subunit and a second peptide or polypeptide comprising a T-cell exposed motif that occurs with a desired frequency of representation compared to a reference database of reference proteins in operable association with a third molecule which does not naturally occur with the second peptide and wherein the second synthetic peptide comprises a groove exposed motif that binds to the MHC molecule of the second peptide or polypeptide with high affinity.

In some embodiments, the predetermined frequency of T-cell exposed motif occurrence in the reference database is greater than 1 in 64 T-cell exposed motifs and the database comprises at least 5000 proteins. In some embodiments, the predetermined frequency of T-cell exposed motif occurrence in the reference database is greater than or equal to 1 in 1024 T-cell exposed motifs and the database comprises at least 5000 proteins. In some embodiments, the predetermined frequency of T-cell exposed motif occurrence in the reference database is from 1 in 1024 to 1 in 16000 T-cell exposed motifs and the database comprises at least 5000 proteins. In some embodiments, the predetermined frequency of T-cell exposed motif occurrence in the reference database is less than 1 in 16000 T-cell exposed motifs and the database comprises at least 5000 proteins. In some embodiments, the T-cell exposed motif is selected from a reference database selected from the group consisting of T-cell exposed motifs found reference database of reference proteins is selected from the group consisting of immunoglobulin variable regions, immunoglobulin constant regions, T cell receptor molecules, proteins of the human proteome other than immunoglobulins, allergens and microorganism proteins. In some embodiments, the third molecule comprises a cytotoxin. In some embodiments, the third molecule comprises a radionuclide molecule. In some embodiments, the radionuclide is an alpha emitter. In some embodiments, the radionuclide is an Auger electron emitter. In some embodiments, the third molecule comprises a positron emitter.

In some embodiments, the second peptide or polypeptide comprising a T cell exposed motif is derived from an oncoprotein or a tumor associated protein. In some embodiments, the oncoprotein is a viral protein from the group comprising papillomavirus, polyomaviruses, adenoviruses, herpesviruses, and retroviruses. In some embodiments, the second peptide or polypeptide comprising a T cell exposed motif is derived from the synthetic polypeptides or fusion polypeptides described above.

In some embodiments, the third peptide or polypeptide is a non-radioactive cytotoxin or cytocide. In some embodiments, the third peptide or polypeptide is a label peptide or an anchor peptide.

In some embodiments, the MHC molecule is selected based on the alleles of a patient. In some embodiments, the MHC molecule subunit is an alpha chain subunit. In some embodiments, the MHC molecule subunit is a beta chain subunit. In some embodiments, the MHC molecule subunit is from an MHC I. In some embodiments, the MHC molecule subunit is from an MHC II.

In some embodiments, the present invention provides a method of suppressing an immune response in a patient by administration to a subject of a polypeptide composition as described above.

In some embodiments, the present invention provides a method of enumerating the T cells in a patient which bind to a specific T cell exposed motif comprising contacting patient T cells with a composition as described above and enumerating the percentage that bind to the composition. In some embodiments, the patient is a cancer patient.

In some embodiments, the present invention provides a synthetic polypeptide comprising a multiplicity of T cell exposed motifs in operable association, wherein the T-cell exposed motifs are selected from a reference database of T cell exposed motifs assembled from a group selected from the group consisting of T-cell exposed motifs found in germline immunoglobulin variable region sequences, T-cell exposed motifs found in somatically mutated immunoglobulin variable region sequences, T-cell exposed motifs found in immunoglobulin constant chains and T-cell exposed motifs found in T-cell receptor molecules, and wherein each of the T-cell exposed motifs in the multiplicity of T-cell exposed motifs have a desired frequency of representation in the reference database.

In some embodiments, the desired frequency of T-cell exposed motif representation in the reference database is greater than 1 in 64 T-cell exposed motifs and the database comprises at least 5000 proteins. In some embodiments, the desired frequency of T-cell exposed motif representation in the reference database is greater than 1 in 512 T-cell exposed motifs and the database comprises at least 5000 proteins. In some embodiments, the desired frequency of T-cell exposed motif representation in the reference database is from 1 in 512 to 1 in 16000 T-cell exposed motifs and the database comprises at least 5000 proteins. In some embodiments, the desired frequency of T-cell exposed motif representation in the reference database is less than 1 in 16000 T-cell exposed motifs and the database comprises at least 5000 proteins.

In some embodiments, the synthetic polypeptide further comprises a peptide linker sequence between each T-cell exposed motif. In some embodiments, the linker sequences each comprise 2-15 amino acids. In some embodiments, the linker sequences comprise endosomal peptidase cleavage sites with a probability of cleavage of >0.5. In some embodiments, the T-cell exposed motif is also categorized in a database comprising proteins from the groups consisting of the human proteome other than immunoglobulins, allergens, and microorganisms. In some embodiments, the multiplicity of T-cell exposed motifS comprise 2-10 different T-cell exposed motifs. In some embodiments, the multiplicity of T-cell exposed motifs comprise repetition of the same T-cell exposed motif. In some embodiments, the polypeptide is from 30 to 150 amino acids in length. In some embodiments, the polypeptide comprises less than 5 different amino acids. In some embodiments, the polypeptide comprises less than 10 different amino acids.

In some embodiments, the present invention provides a method of generating an immunosuppressive response by administration of the synthetic polypeptide as described above to a subject. In some embodiments, the subject has an autoimmune disease. In some embodiments, the autoimmune disease is from the group comprising multiple sclerosis, systemic lupus erythematosis, myasthenia gravis, rheumatoid arthritis, Alzheimers disease, diabetes, and Parkinsons disease.

In some embodiments, the present invention provides a polypeptide composition comprising a first synthetic polypeptide comprising a MHC beta chain and a second synthetic polypeptide comprising a MHC subunit chain operably linked to a third synthetic polypeptide which does not normally occur in association with the MHC alpha chain. In some embodiments, the third synthetic polypeptide is an immunoglobulin sequence. In some embodiments, the immunoglobulin sequence is a constant region sequence. In some embodiments, the cysteine residue forming the hinge cross link is substituted. In some embodiments, the third synthetic polypeptide is operably linked to a cytotoxin. In some embodiments, the third synthetic polypeptide incorporates a radionuclide. In some embodiments, the MHC subunit chain is an alpha chain. In some embodiments, the MHC subunit chain is a beta chain. In some embodiments, the MHC is a Type II MHC from the group comprising DR, DP and DQ. In some embodiments, the MHC is a Type I MHC from the group comprising MHC IA and MHC IB.

In some embodiments, the present invention provides a host cell expressing the polypeptide composition described above.

### DESCRIPTION OF THE FIGURES

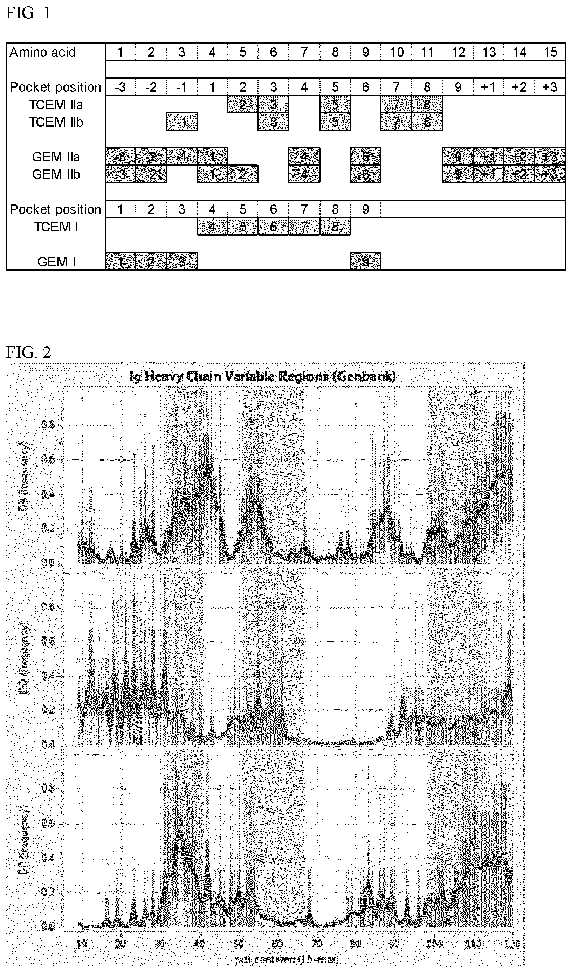

FIG. 1: Discontinuous sets of amino acids in peptides were placed into categorical groups based on the structural analysis of amino acid contact points in pMHC:T-cell receptor complexes (Rudolph *et al* [2]). The positions of the amino acids used in the classification scheme are shown. For MHC-II, T-cell exposed motifs are related to pocket positions in a 15-mer, with a conventional 9-mer core flanked by 3 amino acids on each end. A 9-mer is shown for MHC-I. Two types of class II T-cell exposed motifs, TCEM IIa and TCEM IIb are considered. They are discontinuous and occupy the register positions shown. TCEM IIa comprises amino acids 2,3,5,7,8 and TCEM IIb comprises -1,3,5,7,8. The obverse face of the 15-mer comprises the corresponding groove exposed motifs, GEM IIA and GEM IIb, which are primarily involved in peptide binding to the MHC. The class I motif is a continuous series of amino acids occupying amino acid positions 4,5,6,7,8 in a conventional MHC-I pocket. The GEM I, amino acids 1,2,3,9 are those amino acids primarily responsible for binding the peptide in the MHC-I.
FIG. 2: Predicted MHC binding affinity of all sequential peptides derived from a subset of ten thousand IGHV3-origin variable regions. A: DR, B: DP, C: DQ alleles MHC II (15-mers). The binding affinity is expressed as the fraction of alleles binding the peptide centered at the indicated position, where the binding affinity of the peptide to an allele is predicted more than 1 standard deviation below the mean for that allele within the particular protein. The solid line represents the average and the extensions show the 10% and 90% points. Thus, a predicted affinity as seen for DR centered at aa 38 of 0.62 indicates that on average 10 of the 16 DR alleles for which predictions are made are predicted to bind this peptide with an affinity in excess of 1 standard deviation below the mean. As shown by the extensions some peptides in are bound by essentially all MHC II alleles evaluated. The grey shaded background indicates the approximate location of the three CDRs.

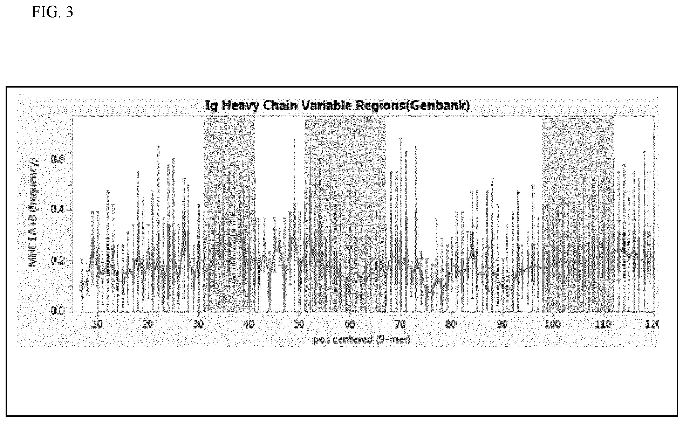

FIG. 3: Predicted binding affinity of MHC I A and B alleles (9-mers). In contrast to MHC-II the distribution of MHC-I binding is fairly uniform throughout the IGHV region. As for Figure 1, peptides are centered at the amino acid position shown and the Y axis shows the fraction of alleles which bind with an affinity of more than one standard deviation below the mean of all alleles at that position.

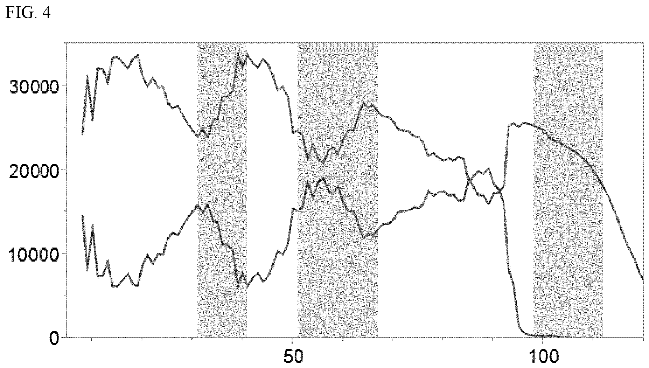

FIG. 4: Frequency of unique motifs as a function of amino acid centered location for the entire 40K dataset. The red line plots the number of unique germline-origin motifs and the blue line the unique motifs generated by SHM.

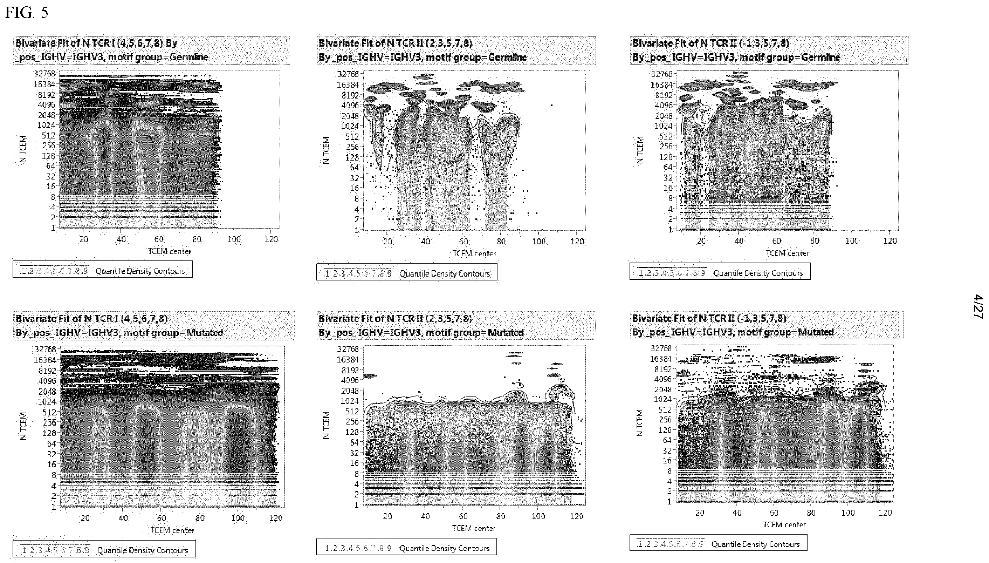

FIG. 5: Positional frequencies of unique T-cell exposed motifs are shown for TCEM IIa, TCEM IIb, and TCEM I for IGHV3. The center of each of the TCEM are plotted against the log₂ of the number of different motifs at the particular location in the IGHV. The upper panel shows IGHV3 germline sequences and the lower panel is from the entire SHM 40K data set that has 22,458 different IGHV3 proteins. A quantile density contouring algorithm was used to generate the color gradation contours. The highlighted colors show that the majority of the unique germline motifs are found in CDR 1 and CDR 2 whereas the majority of mutated motifs are found in CDR 3 (which is not found in the germline. Note that FW3 also has a substantial number of unique motifs. Figure shows output for IGHV3; other germline families are very similar.

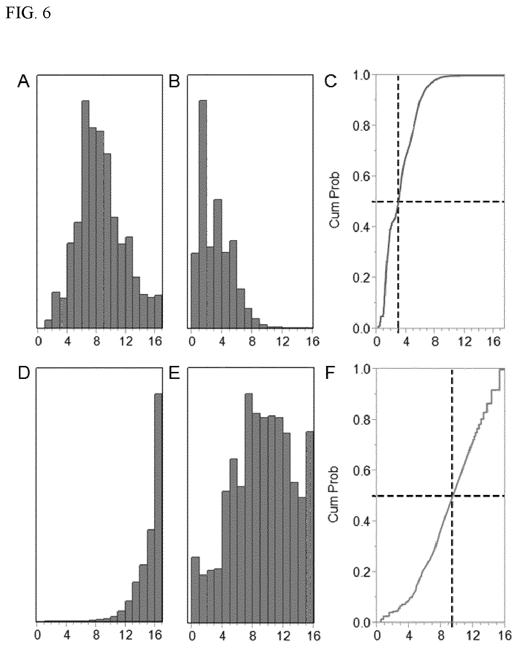

FIG. 6: Frequency distributions of unique T-cell exposed motifs in IGHV3. Frequency distributions of unique TCEM IIa motifs found in 22,458 IGHV3-origin sequences, comprising a total of 2.2x10⁶ possible motifs. Panels A-C show germline-origin motifs and Panels D-F show SHM-origin motifs. Panels A and D: histogram of frequency of occurrence (histogram bins as -log₂). Panels Band E: frequency weighted histogram. Panels C and F: cumulative distribution frequency of B and E. The dashed lines depict the frequency distribution midpoint.

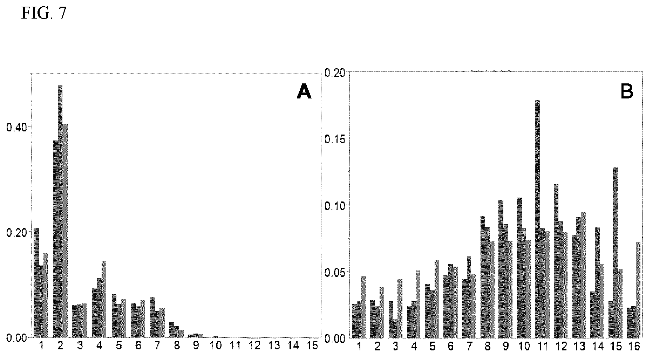

FIG. 7A-B: Distributions of T-cell exposed motif by frequency class for immunoglobulin class-defined IGHV. Histograms of distributions of TCEM Ila by frequency class for immunoglobulin class-defined IGHV. Panel A: germline-origin sequences and Panel B: SHM-origin motifs. IgG: red, IgM: green, and IgE: blue. A frequency classification for each motif was created by binning motifs based on the frequency in the 40K database on a -log₂ scale. For the Ig class-defined datasets shown here the TCEMs in each successive peptide indexed by a single amino acid in the IGHV were then assigned to a frequency class from the main database.

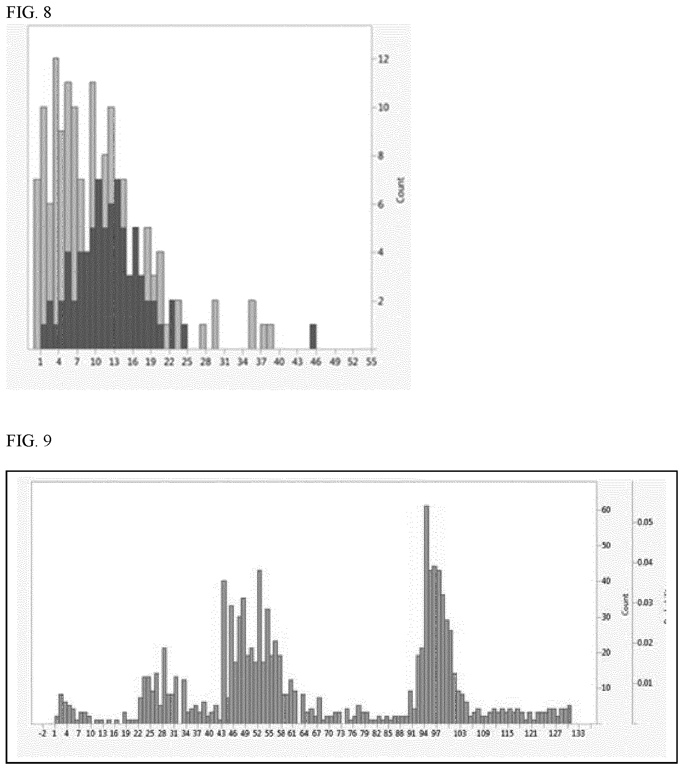

FIG. 8: Count shown in pale green of rare motifs in 163 biotherapeutic antibody products. These are motifs that are not found in the FC1 - FC16 group. The majority thus have 7-10 rare motifs, 46 have one rare motif. When the humanized subset is selected (dark green) it is seen that this group of products have a greater variety of very rare motifs (mean =12).
FIG. 9: Shows the distribution of rare motifs found in commercial antibodies across the variable region. The counts are aligned with the index amino acid of the TCEM. It is seen that more rare motifs occur in the CD3 region. Motifs found in the FC1-16 are excluded from this plot.

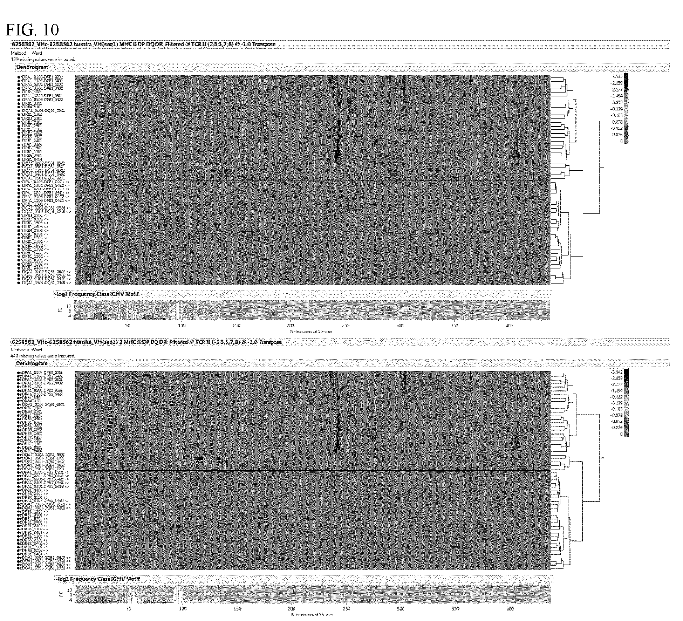

FIG. 10: Shows the differential usage of motifs between heavy chain variable and constant region. Example shown is a full length heavy chain (from Patent # US 6,258,562). Top Panel shows TCEM IIA, Bottom panel shows TCEM IIB motifs. In the bottom bar on each panel, motifs derived from germline HVIG are shown in green, motifs found in mutated HVIG database are shown in cream; in both cases the height of the bar shows the frequency group as indicated on the Y axis of the bottom bar. Motifs shown in red motif are not found in HVIG database. The initial 134 amino acid index positions correspond to the variable region. It is noted that the constant region comprises almost totally motifs not present in the 40K HVIG motif database.

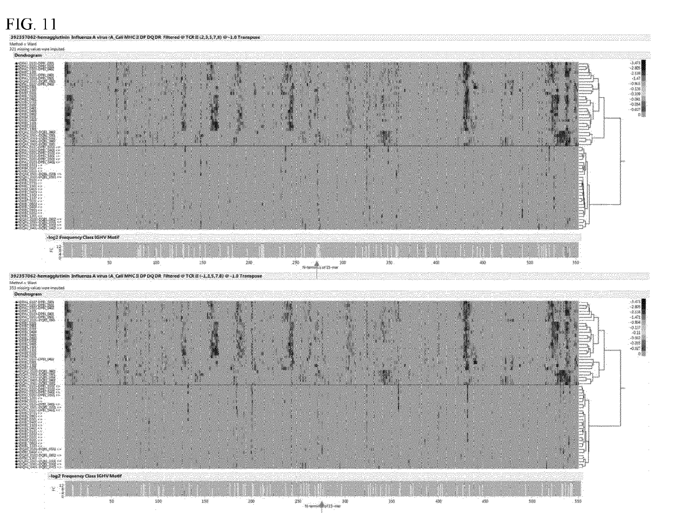

FIG. 11: Shows TCEM identified in hemagglutinin of Influenza A 2009 California. Arrows in the lower tier of each diagram indicate that in both TCEM IIa and TCEM IIb several alleles have peptides with motifs found in IGHV with a binding affinity exceeds -1 standard deviation at position 273-276 ; the top tier of each diagram shows the peptides which have binding affinity in excess of -1 standard deviation to the indicated alleles. Simultaneous peptide affinity threshold mapping of TCEM containing peptides in a target protein and in IGHV database. Horizontal axis is the amino acid position of the N-terminal amino acid of the peptide (9-mer for MHC I and 15-mer for MHC II) containing the matching TCEM. Allele identification is shown on the left portion of the panel. Allele designations without a suffix are the TCEM in the target protein and those with a "<>" suffix indicate the TCEM in the IGHV database. For both the target protein and the IGHV peptides that exceed the standardized affinity threshold are color coded as indicated by the thermometer. For the TCEM in the IGHV the threshold is the overall mean standardized affinity of binding for all the peptides containing the particular TCEM (ranges from singletons to thousands). Any TCEM can be found in association with a wide range of groove exposed motifs which confer a potential wide range of affinities for the peptides. The boxed-X peptides in the panel represent peptides in the target protein for which the threshold was exceeded in the IGHV. It should be noted that the peptides that exceed the threshold in the IGHV might or might not exceed that threshold in the target protein. For consistency they are designated with a boxed-X in either case. The number of peptides that contain a particular TCEM are shown by their frequency classification FC (FC = -log2 frequency) in the lower graphic pane. Thus a FC 12 implies the TCEM is found in 1/2¹² = 1/4096 and a FC 8 = 1/2⁸ = 1/256 frequency in IGHV clones.

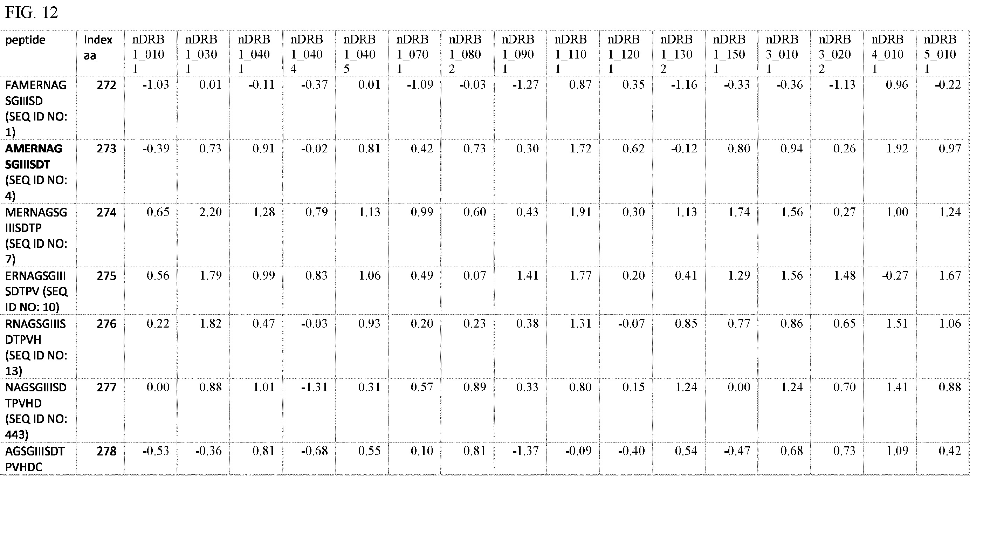

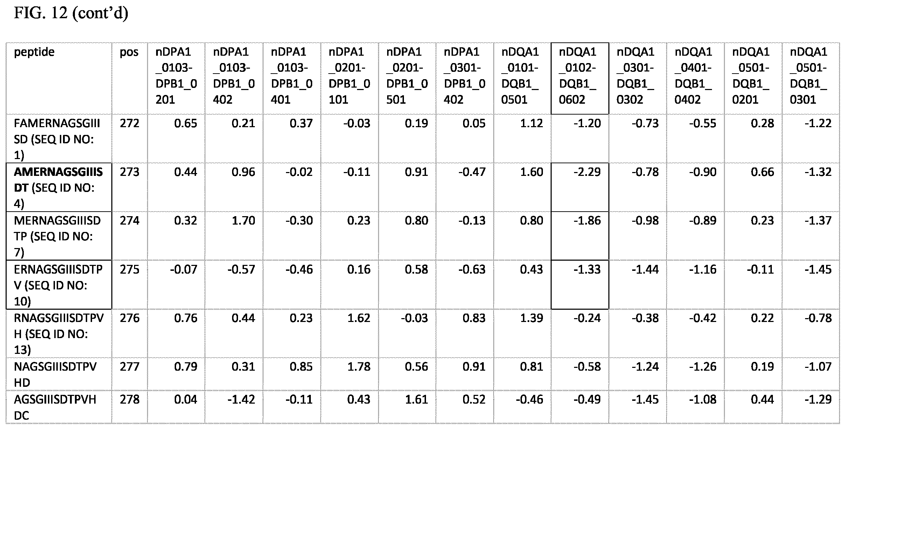

FIG. 12: Shows the predicted binding affinity of nDQA1_0102-DQB1_0602 in hemagglutinin of Influenza A 2009 California far exceeds that of other alleles at position 273-276.

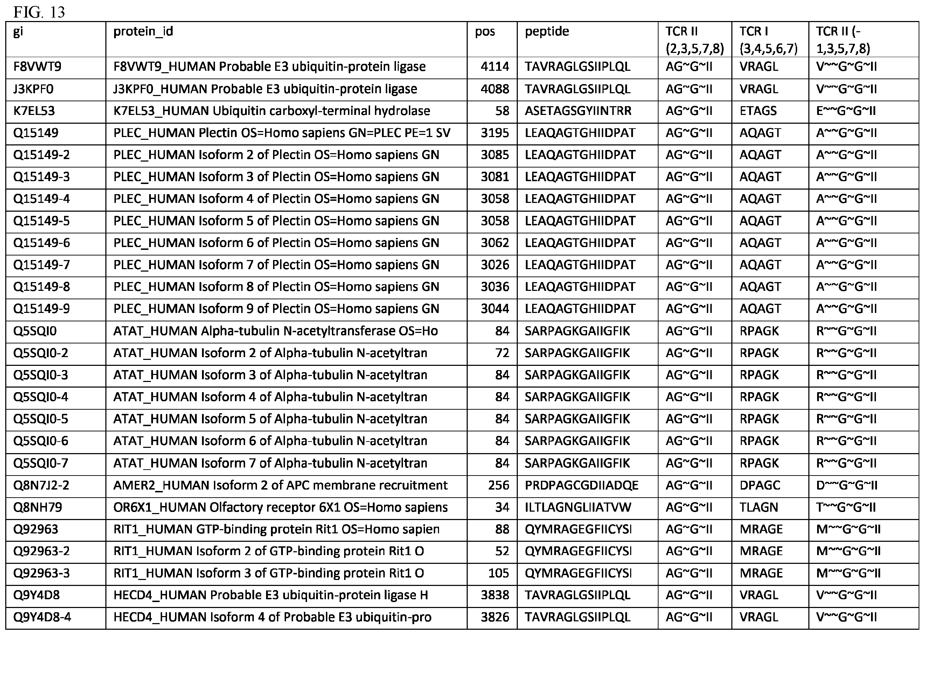

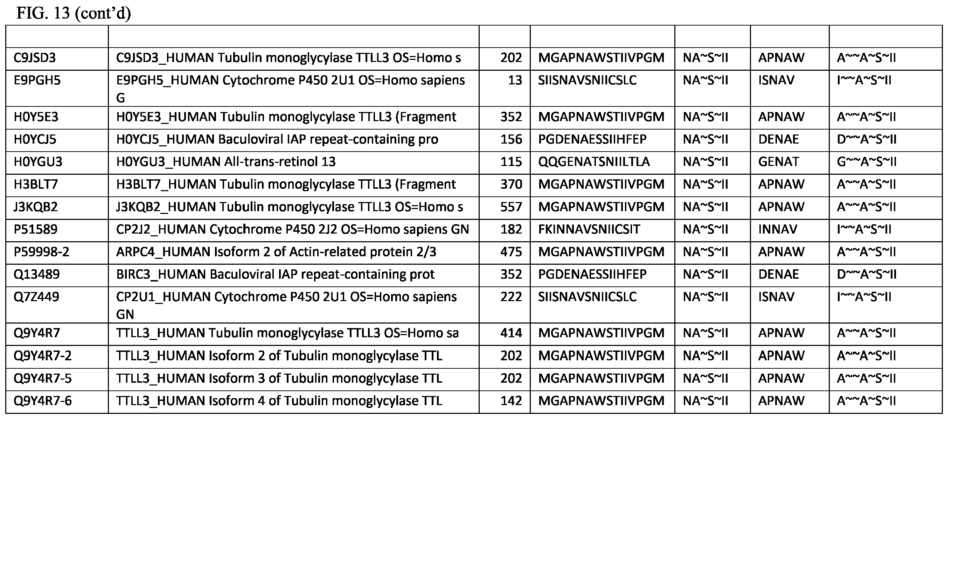

FIG. 13: Shows the proteins in the human proteome containing the two motifs of interest, AG^{~}G^{~}II and NA^{~}S^{~}II which are predicted to bind to DQA1*01:02/DQB1*06:02 DQ.

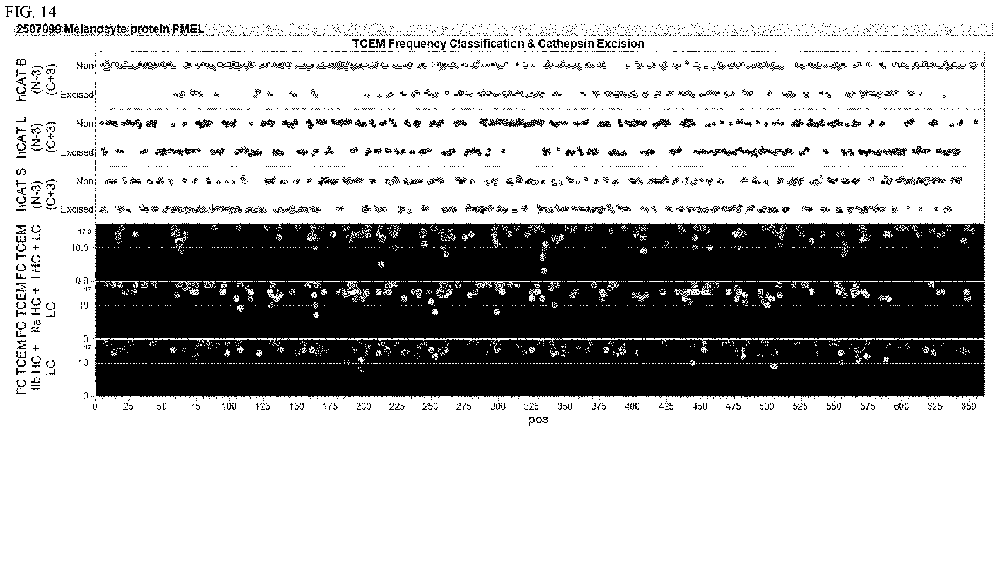

FIG. 14: PMEL showing the distribution of TCEM found in the IgV database. The upper tier indicates motifs in peptides with high probability of being excised by cathepsin. The lower tier indicates the location of IgV TCEM. In each of the three TCEM registers. In each row the dotted white line indicates FC 10 and any point lying below the dotted line is a high frequency motif capable of acting as a Tregulatory motif.

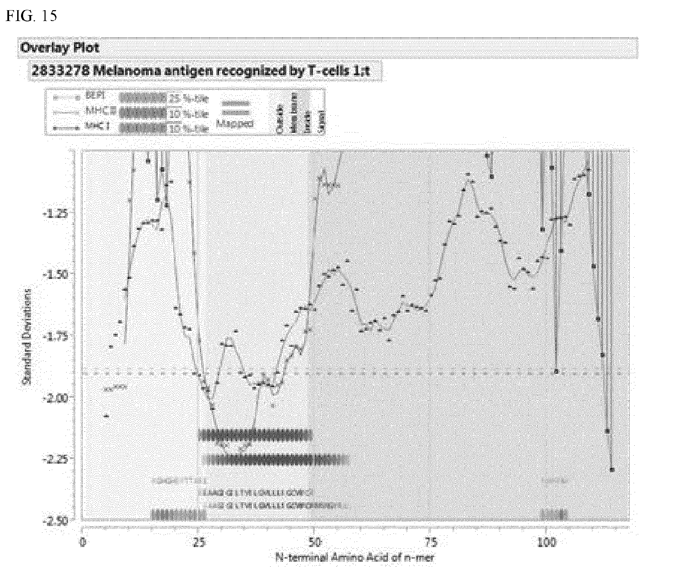

FIG. 15: MART-1 population permuted plot shows a single epitope dense region in this relatively small protein, lying between amino acids 25 -50, as indicated by the blue and red lines. The permuted average of predicted MHC binding affinity is calculated for each sequential peptide. For MHC-I 9-mers are used, for MHC-II 15-mers, each with a single amino acid displacement. At each sequential peptide position we examine every possible heterozygous pair of HLA and mouse MHC alleles and determine which allele of the pair has a higher predicted binding affinity. For each of the higher binding alleles of each pair, the mean and standard deviation of predicted binding affinities is calculated. The predicted binding affinity is represented in standard deviation units relative to the mean for all the peptides in the protein as a whole. As the lines on the graphic show the permuted average, any individual allele affinity may differ from the average. X axis: Sequential peptides are arrayed and numbered N-C. Positions are for the index amino acid of each sequential peptide, with a single amino acid displacement. The Red line shows the permuted average predicted MHC-IA and B (37 alleles) binding affinity by index position of sequential 9-mer peptides. The Blue line shows the permuted average predicted MHC-II DRB allele (16 alleles) binding affinity by index position of sequential 15-mer peptides. Both are plotted in standard deviation units, shown on the Y axis. Orange lines show the predicted probability of B-cell binding ("BEPI") for an amino acid centered in each sequential 9-mer peptide. Note that the standardized B-cell metric has been inverted (multiplied by -1) in order to overlay the standardized probability onto the same scale as the MHC data. Low numbers for MHC data represent high binding affinity, whereas low numbers equate to high BEPI contact probability. Ribbons (Red: MHC-I, Blue: MHC-II) indicate the 10% highest predicted affinity binding. Orange ribbons indicate the top 25% predicted probability B-cell binding. Background shading shows membrane (green) extramembrane (yellow), intramembrane (pink) location. Signal peptides have white background.

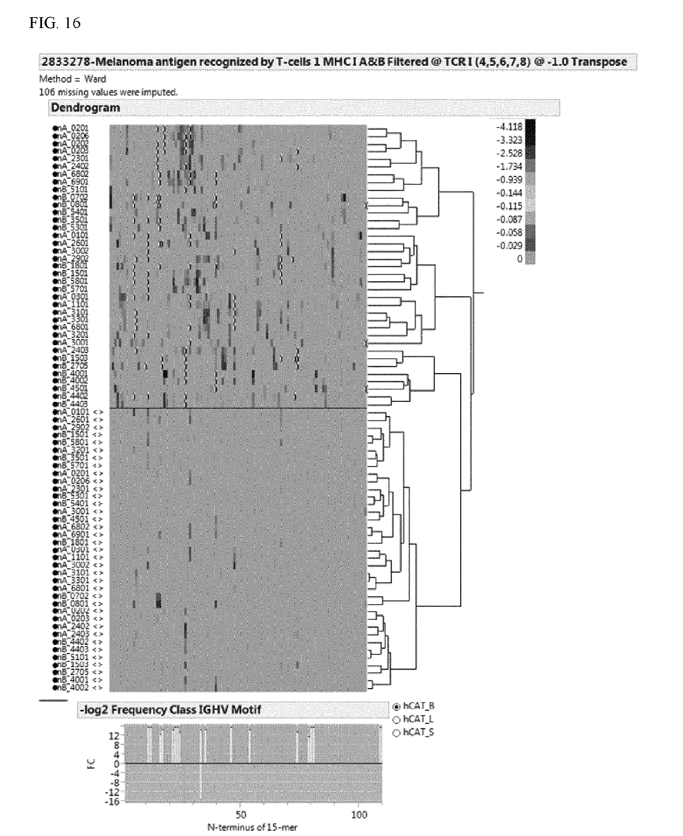

FIG. 16: Identification of MHC-I T-cell exposed motifs of IGHV origin in MART 1 that are found in the database of IGHV Affinities used in the comparisons were based on zero mean unit variance, within-protein transformed data. A database of approximately 40,000 IGHV was used as the reference comparison as described in Example 1. As any particular T-cell exposed motif in the IGHV is found up to thousands of times with different groove exposed motifs (GEMS) each motif has a different characteristic mean affinities (zero mean, unit variance) that was used in the comparison. For this visualization of locations of IGHV motifs in the MART 1 target protein a statistical threshold was selected of -1 standard deviation below the mean affinity for BOTH the target protein AND the IGHV comparative motifs. The locations of the matching TCEMs were then identified using the threshold mask. As a visual aid the locations of the TCEM from the IGHV that match those in the target protein are shown by a missing rectangle and X. Thus a missing rectangle corresponds to the location of a TCEM with a high affinity in the IGHV that is also found in the target protein with a high affinity. The lower panel shows a hierarchical cluster of MHC I alleles showing peptides which contain TCEM motifs which are present in the IGHV database in FC class 16 or more common, and which have a GEM binding affinity denoted by the pixel color of -1σ or more. The top panel shows the peptides in the protein of interest MART-1 which have a binding affinity greater than -1σ, with those peptides which are present in the lower panel denoted on the top panel by X. Darker blue pixels indicate positions with higher binding affinity (as shown in the thermometer). In the bottom panel it is noted that a TCEM I at index position 33 is excised by cathepsin B and has high binding affinity for a majority of alleles.

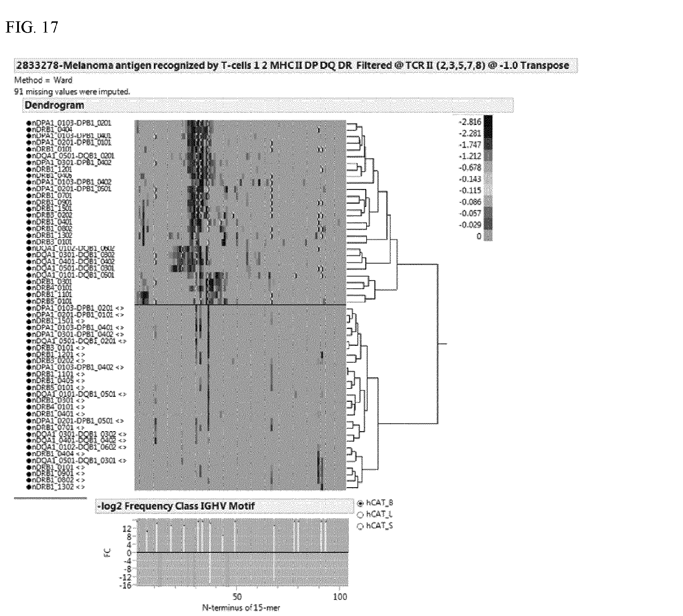

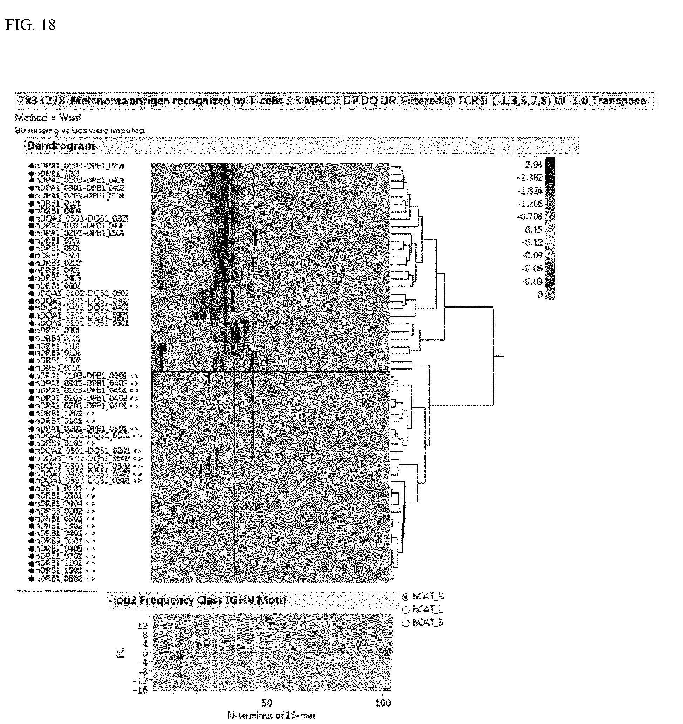

FIGs. 17 and 18: The same process is followed for MHC-II as was done for MHC-I in the prior figure. TCEM IIa and TCEM IIb motifs found in both MART I and the IGHV database are shown in the lower panel with the pixel color indicating affinity of GEM binding. The upper panel shows the high affinity binding peptides in MART I with the TCEM motifs from the lower panel denoted by X. The bottom ribbon indicates the frequency of the TCEM in the IGHV dataset and also whether the peptide is excised by any of the 3 cathepsins B, L or S. In both cases a high affinity TCEM is seen with index position 37, binding to nearly alleles and excised by cathepsin B.

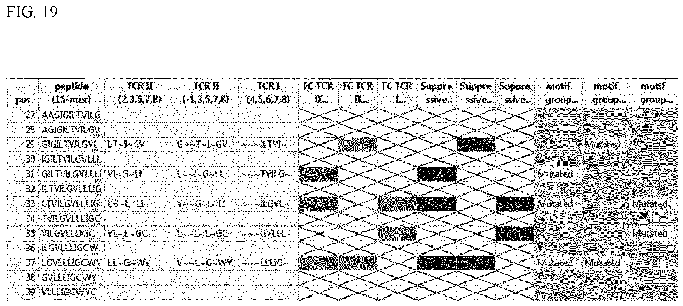

FIG. 19: Shows the peptides from MART-1 for which there are corresponding motifs in the IGHV database. The frequency TCEM motifs are shown for TCEMI and the two TCEM II and frequency classes are shown for each. In the right hand side of the panel the TCEMs are categorized as matching germline or mutated IGHV sequences. The high binding affinity of the peptide indexed at amino acid position 37 makes it a predicted immunosuppressive Treg for all alleles and at position 33 for TCEM I.

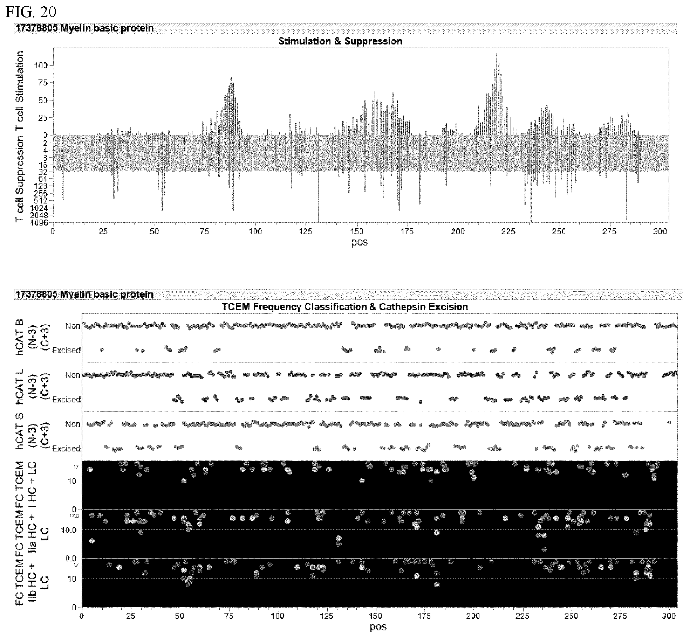

FIG. 20: Myelin basic protein showing the distribution of TCEM found in the IgV database. The upper tier indicates motifs in peptides with high probability of being excised by cathepsin. The lower tier indicates the location of IgV TCEM. In each of the three TCEM registers. In each row the dotted white line indicates FC 10 and any point lying below the dotted line is a high frequency motif capable of acting as a Tregulatory motif.

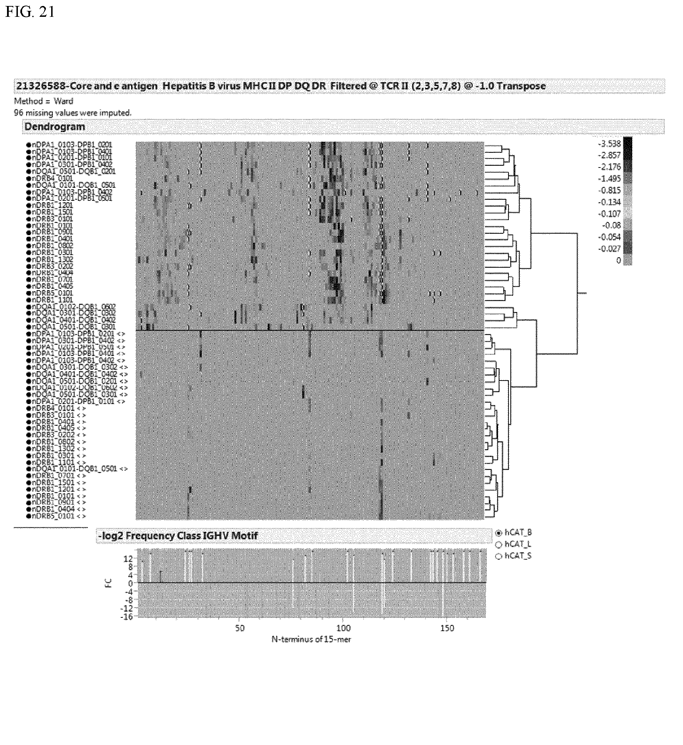

FIG. 21: TCEM IIA Motifs in Hepatitis B virus core protein. TCEM IIA motifs found in both hepatitis B core protein and the IGHV database are shown in the lower panel with the pixel color indicating affinity of GEM binding. The upper panel shows the high affinity binding peptides in hepatitis B core protein with the TCEM motifs from the lower panel denoted by X.. Note peptide with index at position 120 this has a FC = 12 equivalent to 1 in 4096 variable regions and is excised by cathepsin B.

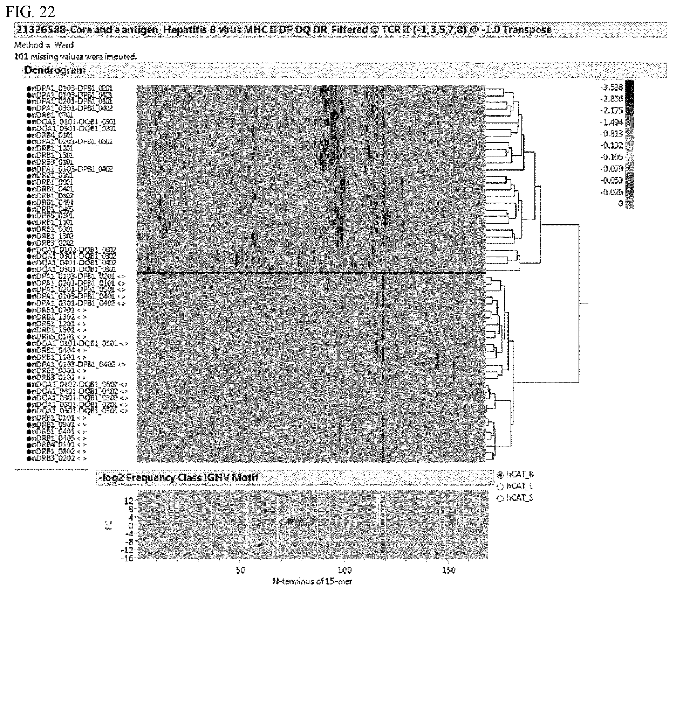

FIG. 22: TCEM IIB Motifs in Hepatitis B virus core protein. In this register the peptide with index position 120 is of FC 8 (1 in 256 variable regions) and is excised by cathepsin B.

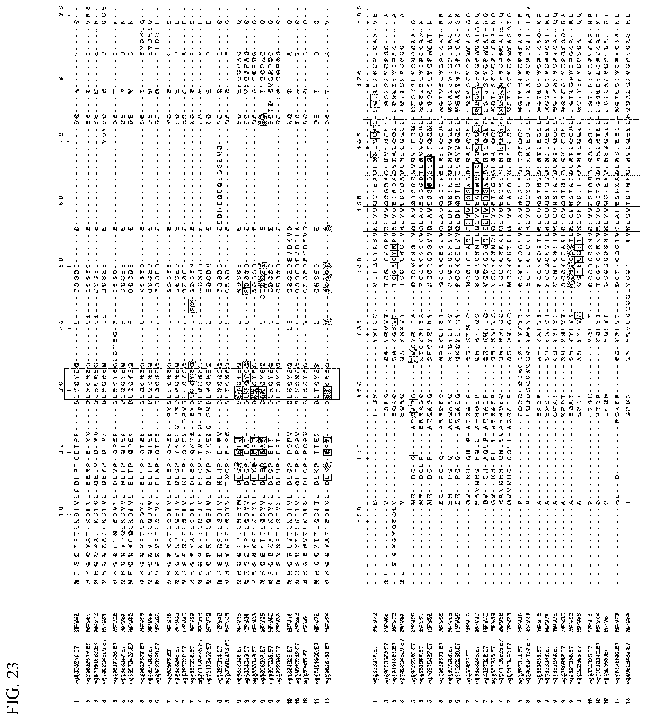

FIG. 23: Location of high frequency TCEM motifs in reference sequences of each E7 protein. Type IIa motifs are shaded grey, type IIb motifs are individually boxed, type I motifs are boldface and framed. The pRB binding site is framed, as are two secondary sites needed for pRB binding.

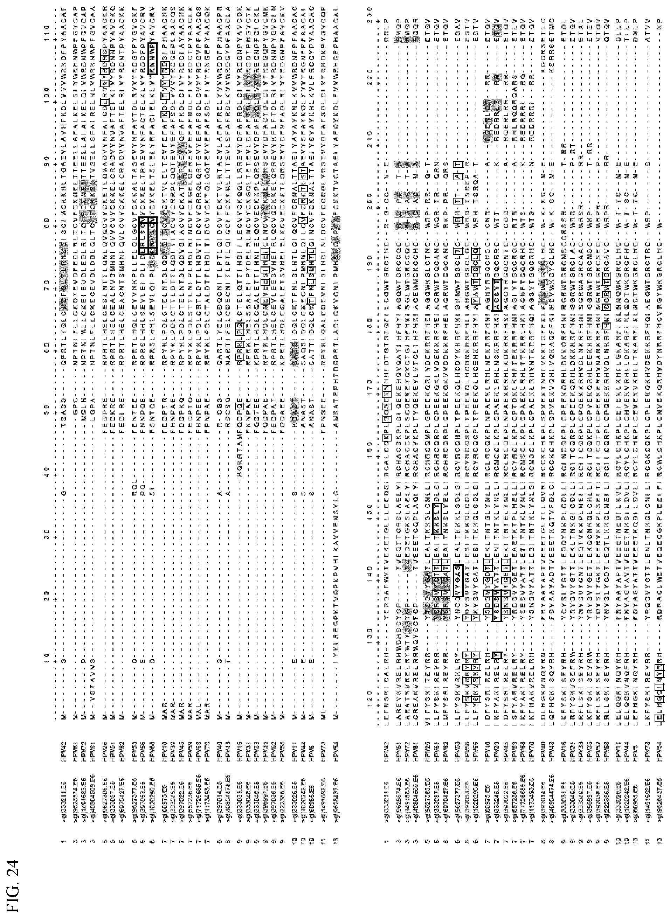

FIG. 24: Location of high frequency TCEM motifs in reference sequences of each E6 protein. Type IIa motifs are shaded grey, type IIb motifs are individually boxed, type I motifs are boldface and framed.

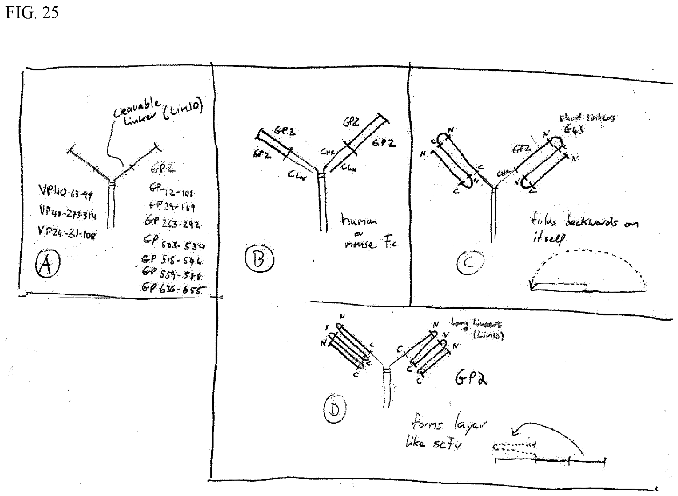

FIG. 25: Shows configuration of constructs for an Ebola vaccine as descried. A . Peptides attached to N terminal of heavy chain of an immunoglobulin with a cleavable linker B. peptides attached to both light and heavy chain N terminal C. Triple peptides with linkers assembled on a immunoglobulin heavy chain D. Triple peptides assembled with extended linkers allowing assembly as in natural spike protein.

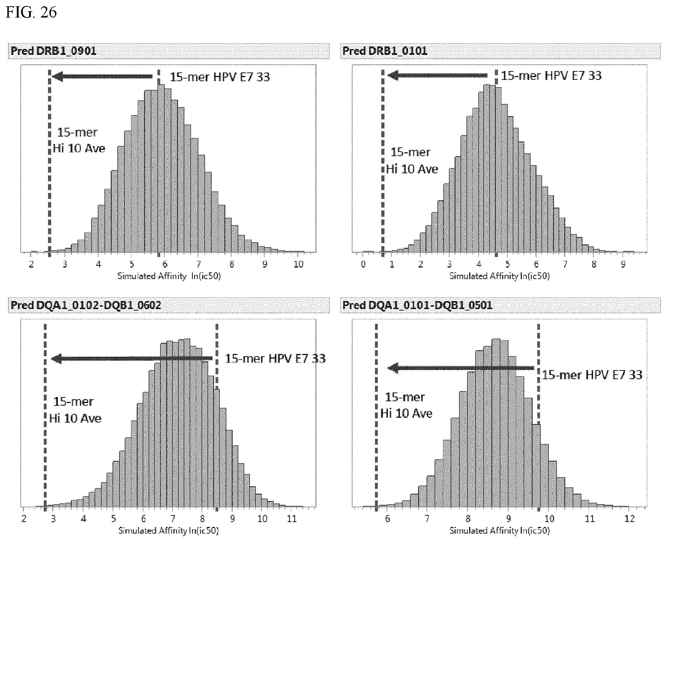

FIG. 26: Affinity distribution of 50,000 peptides generated by Monte Carlo simulation, which maintain the amino acid principal components of two overlapping TCEM frames comprising positions -1,2,3,5,7,8 constant for four selected alleles and varied all the remaining amino acid positions. Arrows show the mean and top 10 peptides.

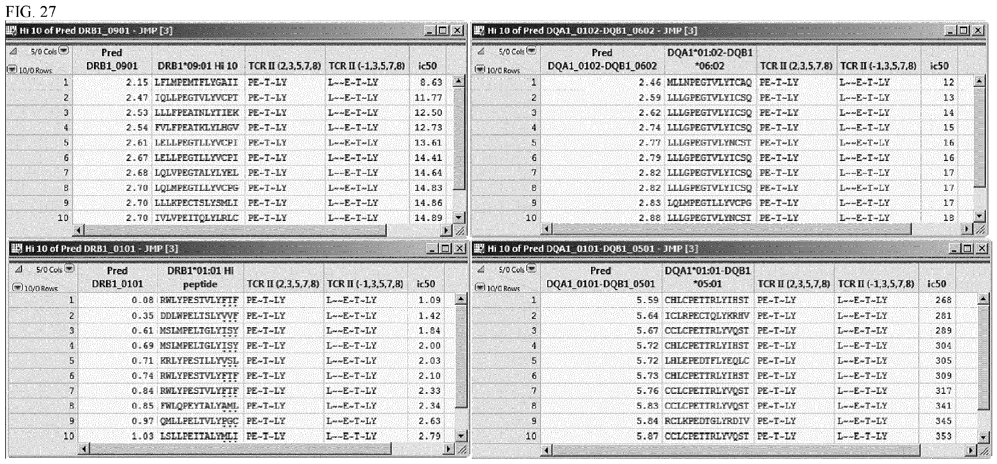

FIG. 27: Tabulation of the affinities (In(ic50)) of the up to ten highest affinity peptides generated for each allele of interest

### DEFINITIONS

As used herein, the term "genome" refers to the genetic material (*e.g*., chromosomes) of an organism or a host cell.

As used herein, the term "proteome" refers to the entire set of proteins expressed by a genome, cell, tissue or organism. A "partial proteome" refers to a subset the entire set of proteins expressed by a genome, cell, tissue or organism. Examples of "partial proteomes" include, but are not limited to, transmembrane proteins, secreted proteins, and proteins with a membrane motif. Human proteome refers to all the proteins comprised in a human being. Multiple such sets of proteins have been sequenced and are accessible at the InterPro international repository (www.ebi.ac.uk/interpro). Human proteome is also understood to include those proteins and antigens thereof which may be over-expressed in certain pathologies, or expressed in a different isoforms in certain pathologies. Hence, as used herein, tumor associated antigens are considered part of the human proteome.

As used herein, the terms "protein," "polypeptide," and "peptide" refer to a molecule comprising amino acids joined via peptide bonds. In general "peptide" is used to refer to a sequence of 20 or less amino acids and "polypeptide" is used to refer to a sequence of greater than 20 amino acids.

As used herein, the term, "synthetic polypeptide," "synthetic peptide" and "synthetic protein" refer to peptides, polypeptides, and proteins that are produced by a recombinant process (i.e., expression of exogenous nucleic acid encoding the peptide, polypeptide or protein in an organism, host cell, or cell-free system) or by chemical synthesis.

As used herein, the term "protein of interest" refers to a protein encoded by a nucleic acid of interest. It may be applied to any protein to which further analysis is applied or the properties of which are tested or examined. Similarly, as used herein, "target protein" may be used to describe a protein of interest that is subject to further analysis.

As used herein "peptidase" refers to an enzyme which cleaves a protein or peptide. The term peptidase may be used interchangeably with protease, proteinases, oligopeptidases, and proteolytic enzymes. Peptidases may be endopeptidases (endoproteases), or exopeptidases (exoproteases). Similarly the term peptidase inhibitor may be used interchangeably with protease inhibitor or inhibitor of any of the other alternate terms for peptidase.

As used herein, the term "exopeptidase" refers to a peptidase that requires a free N-terminal amino group, C-terminal carboxyl group or both, and hydrolyses a bond not more than three residues from the terminus. The exopeptidases are further divided into aminopeptidases, carboxypeptidases, dipeptidyl-peptidases, peptidyl-dipeptidases, tripeptidyl-peptidases and dipeptidases.

As used herein, the term "endopeptidase" refers to a peptidase that hydrolyses internal, alpha-peptide bonds in a polypeptide chain, tending to act away from the N-terminus or C-terminus. Examples of endopeptidases are chymotrypsin, pepsin, papain and cathepsins. A very few endopeptidases act a fixed distance from one terminus of the substrate, an example being mitochondrial intermediate peptidase. Some endopeptidases act only on substrates smaller than proteins, and these are termed oligopeptidases. An example of an oligopeptidase is thimet oligopeptidase. Endopeptidases initiate the digestion of food proteins, generating new N- and C-termini that are substrates for the exopeptidases that complete the process. Endopeptidases also process proteins by limited proteolysis. Examples are the removal of signal peptides from secreted proteins (e.g. signal peptidase I,) and the maturation of precursor proteins (e.g. enteropeptidase, furin,). In the nomenclature of the Nomenclature Committee of the International Union of Biochemistry and Molecular Biology (NC-IUBMB) endopeptidases are allocated to sub-subclasses EC 3.4.21, EC 3.4.22, EC 3.4.23, EC 3.4.24 and EC 3.4.25 for serine-, cysteine-, aspartic-, metallo- and threonine-type endopeptidases, respectively. Endopeptidases of particular interest are the cathepsins, and especially cathepsin B, L and S known to be active in antigen presenting cells.

As used herein, the term "immunogen" refers to a molecule which stimulates a response from the adaptive immune system, which may include responses drawn from the group comprising an antibody response, a cytotoxic T cell response, a T helper response, and a T cell memory. An immunogen may stimulate an upregulation of the immune response with a resultant inflammatory response, or may result in down regulation or immunosuppression. Thus the T-cell response may be a T regulatory response. An immunogen also may stimulate a B-cell response and lead to an increase in antibody titer.

As used herein, the term "native" (or wild type) when used in reference to a protein refers to proteins encoded by the genome of a cell, tissue, or organism, other than one manipulated to produce synthetic proteins.

As used herein the term "epitope" refers to a peptide sequence which elicits an immune response, from either T cells or B cells or antibody

As used herein, the term "B-cell epitope" refers to a polypeptide sequence that is recognized and bound by a B-cell receptor. A B-cell epitope may be a linear peptide or may comprise several discontinuous sequences which together are folded to form a structural epitope. Such component sequences which together make up a B-cell epitope are referred to herein as B-cell epitope sequences. Hence, a B-cell epitope may comprise one or more B-cell epitope sequences. Hence, a B cell epitope may comprise one or more B-cell epitope sequences. A linear B-cell epitope may comprise as few as 2-4 amino acids or more amino acids.

As used herein, the term "predicted B-cell epitope" refers to a polypeptide sequence that is predicted to bind to a B-cell receptor by a computer program, for example, as described in PCT US2011/029192, PCT US2012/055038, and US2014/014523, and in addition by Bepipred (Larsen, et al., Immunome Research 2:2, 2006.) and others as referenced by Larsen et al (ibid) (Hopp T et al PNAS 78:3824-3828, 1981; Parker J et al, Biochem. 25:5425-5432, 1986). A predicted B-cell epitope may refer to the identification of B-cell epitope sequences forming part of a structural B-cell epitope or to a complete B-cell epitope.

As used herein, the term "T-cell epitope" refers to a polypeptide sequence which when bound to a major histocompatibility protein molecule provides a configuration recognized by a T-cell receptor. Typically, T-cell epitopes are presented bound to a MHC molecule on the surface of an antigen-presenting cell.

As used herein, the term "predicted T-cell epitope" refers to a polypeptide sequence that is predicted to bind to a major histocompatibility protein molecule by the neural network algorithms described herein, by other computerized methods, or as determined experimentally.

As used herein, the term "major histocompatibility complex (MHC)" refers to the MHC Class I and MHC Class II genes and the proteins encoded thereby. Molecules of the MHC bind small peptides and present them on the surface of cells for recognition by T-cell receptor-bearing T-cells. The MHC-Is both polygenic (there are several MHC class I and MHC class II genes) and polyallelic or polymorphic (there are multiple alleles of each gene). The terms MHC-I, MHC-II, MHC-1 and MHC-2 are variously used herein to indicate these classes of molecules. Included are both classical and nonclassical MHC molecules. An MHC molecule is made up of multiple chains (alpha and beta chains) which associate to form a molecule. The MHC molecule contains a cleft or groove which forms a binding site for peptides. Peptides bound in the cleft or groove may then be presented to T-cell receptors. The term "MHC binding region" refers to the groove region of the MHC molecule where peptide binding occurs.

As used herein, a "MHC II binding groove" refers to the structure of an MHC molecule that binds to a peptide. The peptide that binds to the MHC II binding groove may be from about 11 amino acids to about 23 amino acids in length, but typically comprises a 15-mer. The amino acid positions in the peptide that binds to the groove are numbered based on a central core of 9 amino acids numbered 1-9, and positions outside the 9 amino acid core numbered as negative (N terminal) or positive (C terminal). Hence, in a 15mer the amino acid binding positions are numbered from -3 to +3 or as follows: -3, -2, -1, 1, 2, 3, 4, 5, 6, 7, 8, 9, +1, +2, +3.

As used herein, the term "haplotype" refers to the HLA alleles found on one chromosome and the proteins encoded thereby. Haplotype may also refer to the allele present at any one locus within the MHC. Each class of MHC-Is represented by several loci: e.g., HLA-A (Human Leukocyte Antigen-A), HLA-B, HLA-C, HLA-E, HLA-F, HLA-G, HLA-H, HLA-J, HLA-K, HLA-L, HLA-P and HLA-V for class I and HLA-DRA, HLA-DRB1-9, HLA-, HLA-DQA1, HLA-DQB1, HLA-DPA1, HLA-DPB1, HLA-DMA, HLA-DMB, HLA-DOA, and HLA-DOB for class II. The terms "HLA allele" and "MHC allele" are used interchangeably herein. HLA alleles are listed at hla.alleles.org/nomenclature/naming.html.

The MHCs exhibit extreme polymorphism: within the human population there are, at each genetic locus, a great number of haplotypes comprising distinct alleles-the IMGT/HLA database release (February 2010) lists 948 class I and 633 class II molecules, many of which are represented at high frequency (>1%). MHC alleles may differ by as many as 30-aa substitutions. Different polymorphic MHC alleles, of both class I and class II, have different peptide specificities: each allele encodes proteins that bind peptides exhibiting particular sequence patterns.

The naming of new HLA genes and allele sequences and their quality control is the responsibility of the WHO Nomenclature Committee for Factors of the HLA System, which first met in 1968, and laid down the criteria for successive meetings. This committee meets regularly to discuss issues of nomenclature and has published 19 major reports documenting firstly the HLA antigens and more recently the genes and alleles. The standardization of HLA antigenic specifications has been controlled by the exchange of typing reagents and cells in the International Histocompatibility Workshops. The IMGT/HLA Database collects both new and confirmatory sequences, which are then expertly analyzed and curated before been named by the Nomenclature Committee. The resulting sequences are then included in the tools and files made available from both the IMGT/HLA Database and at hla.alleles.org.

Each HLA allele name has a unique number corresponding to up to four sets of digits separated by colons. See e.g., hla.alleles.org/nomenclature/naming.html which provides a description of standard HLA nomenclature and Marsh et al., Nomenclature for Factors of the HLA System, 2010 Tissue Antigens 2010 75:291-455. HLA-DRB1*13:01 and HLA-DRB1*13:01:01:02 are examples of standard HLA nomenclature. The length of the allele designation is dependent on the sequence of the allele and that of its nearest relative. All alleles receive at least a four digit name, which corresponds to the first two sets of digits, longer names are only assigned when necessary.

The digits before the first colon describe the type, which often corresponds to the serological antigen carried by an allotype, The next set of digits are used to list the subtypes, numbers being assigned in the order in which DNA sequences have been determined. Alleles whose numbers differ in the two sets of digits must differ in one or more nucleotide substitutions that change the amino acid sequence of the encoded protein. Alleles that differ only by synonymous nucleotide substitutions (also called silent or non-coding substitutions) within the coding sequence are distinguished by the use of the third set of digits. Alleles that only differ by sequence polymorphisms in the introns or in the 5' or 3' untranslated regions that flank the exons and introns are distinguished by the use of the fourth set of digits. In addition to the unique allele number there are additional optional suffixes that may be added to an allele to indicate its expression status. Alleles that have been shown not to be expressed, 'Null' alleles have been given the suffix 'N'. Those alleles which have been shown to be alternatively expressed may have the suffix 'L', 'S', 'C', 'A' or 'Q'. The suffix 'L' is used to indicate an allele which has been shown to have 'Low' cell surface expression when compared to normal levels. The 'S' suffix is used to denote an allele specifying a protein which is expressed as a soluble 'Secreted' molecule but is not present on the cell surface. A 'C' suffix to indicate an allele product which is present in the 'Cytoplasm' but not on the cell surface. An 'A' suffix to indicate 'Aberrant' expression where there is some doubt as to whether a protein is expressed. A 'Q' suffix when the expression of an allele is 'Questionable' given that the mutation seen in the allele has previously been shown to affect normal expression levels.

In some instances, the HLA designations used herein may differ from the standard HLA nomenclature just described due to limitations in entering characters in the databases described herein. As an example, DRB1_0104, DRB1*0104, and DRB1-0104 are equivalent to the standard nomenclature of DRB1*01:04. In most instances, the asterisk is replaced with an underscore or dash and the semicolon between the two digit sets is omitted.

As used herein, the term "polypeptide sequence that binds to at least one major histocompatibility complex (MHC) binding region" refers to a polypeptide sequence that is recognized and bound by one or more particular MHC binding regions as predicted by the neural network algorithms described herein or as determined experimentally.

As used herein the terms "canonical" and "non-canonical" are used to refer to the orientation of an amino acid sequence. Canonical refers to an amino acid sequence presented or read in the N terminal to C terminal order; non-canonical is used to describe an amino acid sequence presented in the inverted or C terminal to N terminal order.

As used herein, the term "allergen" refers to an antigenic substance capable of producing immediate hypersensitivity and includes both synthetic as well as natural immunostimulant peptides and proteins. Allergen includes but is not limited to any protein or peptide catalogued in the Structural Database of Allergenic Proteins database http://fermi.utmb.edu/SDAP/index.html

As used herein, the term "transmembrane protein" refers to proteins that span a biological membrane. There are two basic types of transmembrane proteins. Alpha-helical proteins are present in the inner membranes of bacterial cells or the plasma membrane of eukaryotes, and sometimes in the outer membranes. Beta-barrel proteins are found only in outer membranes of Gram-negative bacteria, cell wall of Gram-positive bacteria, and outer membranes of mitochondria and chloroplasts.

As used herein, the term "consensus protease cleavage site" refers to an amino acid sequence that is recognized by a protease such as trypsin or pepsin.

As used herein, the term "affinity" refers to a measure of the strength of binding between two members of a binding pair, for example, an antibody and an epitope and an epitope and a MHC-I or II haplotype. K_{d} is the dissociation constant and has units of molarity. The affinity constant is the inverse of the dissociation constant. An affinity constant is sometimes used as a generic term to describe this chemical entity. It is a direct measure of the energy of binding. The natural logarithm of K is linearly related to the Gibbs free energy of binding through the equation ΔG₀ = -RT LN(K) where R= gas constant and temperature is in degrees Kelvin. Affinity may be determined experimentally, for example by surface plasmon resonance (SPR) using commercially available Biacore SPR units (GE Healthcare) or *in silico* by methods such as those described herein in detail. Affinity may also be expressed as the ic50 or inhibitory concentration 50, that concentration at which 50% of the peptide is displaced. Likewise In(ic50) refers to the natural log of the ic50.

The term "K_{off}", as used herein, is intended to refer to the off rate constant, for example, for dissociation of an antibody from the antibody/antigen complex, or for dissociation of an epitope from an MHC haplotype.

The term "K_{d}", as used herein, is intended to refer to the dissociation constant (the reciprocal of the affinity constant "Ka"), for example, for a particular antibody-antigen interaction or interaction between an epitope and an MHC haplotype.

As used herein, the terms "strong binder" and "strong binding" and "High binder" and "high binding" or "high affinity" refer to a binding pair or describe a binding pair that have an affinity of greater than 2 x10⁷M⁻¹ (equivalent to a dissociation constant of 50nM Kd)

As used herein, the term "moderate binder" and "moderate binding" and "moderate affinity" refer to a binding pair or describe a binding pair that have an affinity of from 2 x10⁷M⁻¹ to 2 x10⁶M⁻¹.

As used herein, the terms "weak binder" and "weak binding" and "low affinity" refer to a binding pair or describe a binding pair that have an affinity of less than 2 x10⁶M⁻¹ (equivalent to a dissociation constant of 500nM Kd)

Binding affinity may also be expressed by the standard deviation from the mean binding found in the peptides making up a protein. Hence a binding affinity may be expressed as "-1σ" or <-1σ, where this refers to a binding affinity of 1 or more standard deviations below the mean. A common mathematical transformation used in statistical analysis is a process called standardization wherein the distribution is transformed from its standard units to standard deviation units where the distribution has a mean of zero and a variance (and standard deviation) of 1. Because each protein comprises unique distributions for the different MHC alleles standardization of the affinity data to zero mean and unit variance provides a numerical scale where different alleles and different proteins can be compared. Analysis of a wide range of experimental results suggest that a criterion of standard deviation units can be used to discriminate between potential immunological responses and non-responses. An affinity of 1 standard deviation below the mean was found to be a useful threshold in this regard and thus approximately 15% (16.2% to be exact) of the peptides found in any protein will fall into this category.

The terms "specific binding" or "specifically binding" when used in reference to the interaction of an antibody and a protein or peptide or an epitope and an MHC haplotype means that the interaction is dependent upon the presence of a particular structure (i.e., the antigenic determinant or epitope) on the protein; in other words the antibody is recognizing and binding to a specific protein structure rather than to proteins in general. For example, if an antibody is specific for epitope "A," the presence of a protein containing epitope A (or free, unlabeled A) in a reaction containing labeled "A" and the antibody will reduce the amount of labeled A bound to the antibody.

As used herein, the term "antigen binding protein" refers to proteins that bind to a specific antigen. "Antigen binding proteins" include, but are not limited to, immunoglobulins, including polyclonal, monoclonal, chimeric, single chain, and humanized antibodies, Fab fragments, F(ab')2 fragments, and Fab expression libraries. Various procedures known in the art are used for the production of polyclonal antibodies. For the production of antibody, various host animals can be immunized by injection with the peptide corresponding to the desired epitope including but not limited to rabbits, mice, rats, sheep, goats, *etc.* Various adjuvants are used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and *Corynebacterium parvum.*

For preparation of monoclonal antibodies, any technique that provides for the production of antibody molecules by continuous cell lines in culture may be used (*See e.g.,* Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature, 256:495-497 [1975]), as well as the trioma technique, the human B-cell hybridoma technique (*See e.g.,* Kozbor et al., Immunol. Today, 4:72 [1983]), and the EBV-hybridoma technique to produce human monoclonal antibodies (Cole et al., in Monoclonal Antibodies and Cancer Therapy, Alan R. Liss, Inc., pp. 77-96 [1985]). In other embodiments, suitable monoclonal antibodies, including recombinant chimeric monoclonal antibodies and chimeric monoclonal antibody fusion proteins are prepared as described herein.

According to the invention, techniques described for the production of single chain antibodies (US 4,946,778) can be adapted to produce specific single chain antibodies as desired. An additional embodiment of the invention utilizes the techniques known in the art for the construction of Fab expression libraries (Huse et al., Science, 246:1275-1281 [1989]) to allow rapid and easy identification of monoclonal Fab fragments with the desired specificity.

Antibody fragments that contain the idiotype (antigen binding region) of the antibody molecule can be generated by known techniques. For example, such fragments include but are not limited to: the F(ab')2 fragment that can be produced by pepsin digestion of an antibody molecule; the Fab' fragments that can be generated by reducing the disulfide bridges of an F(ab')2 fragment, and the Fab fragments that can be generated by treating an antibody molecule with papain and a reducing agent.

Genes encoding antigen-binding proteins can be isolated by methods known in the art. In the production of antibodies, screening for the desired antibody can be accomplished by techniques known in the art (*e.g*., radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g.,* gel agglutination assays, hemagglutination assays, *etc*.), complement fixation assays, immunofluorescence assays, protein A assays, and immunoelectrophoresis assays, *etc*.) *etc.*

As used herein "immunoglobulin" means the distinct antibody molecule secreted by a clonal line of B cells; hence when the term "100 immunoglobulins" is used it conveys the distinct products of 100 different B-cell clones and their lineages.

As used herein, the terms "computer memory" and "computer memory device" refer to any storage media readable by a computer processor. Examples of computer memory include, but are not limited to, RAM, ROM, computer chips, digital video disc (DVDs), compact discs (CDs), hard disk drives (HDD), and magnetic tape.

As used herein, the term "computer readable medium" refers to any device or system for storing and providing information (e.g., data and instructions) to a computer processor. Examples of computer readable media include, but are not limited to, DVDs, CDs, hard disk drives, magnetic tape and servers for streaming media over networks.

As used herein, the terms "processor" and "central processing unit" or "CPU" are used interchangeably and refer to a device that is able to read a program from a computer memory (e.g., ROM or other computer memory) and perform a set of steps according to the program.

As used herein, the term "support vector machine" refers to a set of related supervised learning methods used for classification and regression. Given a set of training examples, each marked as belonging to one of two categories, an SVM training algorithm builds a model that predicts whether a new example falls into one category or the other.

As used herein, the term "classifier" when used in relation to statistical processes refers to processes such as neural nets and support vector machines.

As used herein "neural net", which is used interchangeably with "neural network" and sometimes abbreviated as NN, refers to various configurations of classifiers used in machine learning, including multilayered perceptrons with one or more hidden layer, support vector machines and dynamic Bayesian networks. These methods share in common the ability to be trained, the quality of their training evaluated, and their ability to make either categorical classifications of non numeric data or to generate equations for predictions of continuous numbers in a regression mode. Perceptron as used herein is a classifier which maps its input x to an output value which is a function of x, or a graphical representation thereof.

As used herein, the term "principal component analysis", or as abbreviated PCA, refers to a mathematical process which reduces the dimensionality of a set of data (Wold, S., Sjorstrom,M., and Eriksson,L., Chemometrics and Intelligent Laboratory Systems 2001. 58: 109-130.; Multivariate and Megavariate Data Analysis Basic Principles and Applications (Parts I&II) by L. Eriksson, E. Johansson, N. Kettaneh-Wold, and J. Trygg , 2006 2nd Edit. Umetrics Academy ). Derivation of principal components is a linear transformation that locates directions of maximum variance in the original input data, and rotates the data along these axes. For n original variables, n principal components are formed as follows: The first principal component is the linear combination of the standardized original variables that has the greatest possible variance. Each subsequent principal component is the linear combination of the standardized original variables that has the greatest possible variance and is uncorrelated with all previously defined components. Further, the principal components are scale-independent in that they can be developed from different types of measurements. The application of PCA generates numerical coefficients (descriptors). The coefficients are effectively proxy variables whose numerical values are seen to be related to underlying physical properties of the molecules. A description of the application of PCA to generate descriptors of amino acids and by combination thereof peptides is provided in PCT US2011/029192, Unlike neural nets PCA do not have any predictive capability. PCA is deductive not inductive.

As used herein, the term "vector" when used in relation to a computer algorithm or the present invention, refers to the mathematical properties of the amino acid sequence.

As used herein, the term "vector," when used in relation to recombinant DNA technology, refers to any genetic element, such as a plasmid, phage, transposon, cosmid, chromosome, retrovirus, virion, *etc.,* which is capable of replication when associated with the proper control elements and which can transfer gene sequences between cells. Thus, the term includes cloning and expression vehicles, as well as viral vectors.

As used herein, the terms "biocide" or "biocides" or "microbiocides" refer to at least a portion of a naturally occurring or synthetic molecule (*e.g.,* peptides or enzymes) that directly kills or promotes the death and/or attenuation of (*e.g.,* prevents growth and/or replication) of biological targets (*e.g.,* bacteria, parasites, yeast, viruses, fungi, protozoans and the like). Examples of biocides include, but are not limited to, bactericides, viricides, fungicides, parasiticides, and the like.

As used herein, the terms "protein biocide" and "protein biocides" or "protein microbiocides" refer to at least a portion of a naturally occurring or synthetic peptide molecule or enzyme that directly kills or promotes the death and/or attenuation of (*e.g.,* prevents growth and/or replication) of biological targets (*e.g.,* bacteria, parasites, yeast, viruses, fungi, protozoans and the like). Examples of biocides include, but are not limited to, bactericides, viricides, fungicides, parasiticides, and the like.

As used herein, the term "neutralization," "pathogen neutralization," refer to destruction or inactivation (*e.g.,* loss of virulence) of a "pathogen" (*e.g.,* bacterium, parasite, virus, fungus, mold, prion, and the like) thus preventing the pathogen's ability to initiate a disease state in a subject.

As used herein, the term "microorganism targeting molecule" refers to any molecule (*e.g.,* protein) that interacts with a microorganism. In preferred embodiments, the microorganism targeting molecule specifically interacts with microorganisms at the exclusion of non-microorganism host cells. Preferred microorganism targeting molecules interact with broad classes of microorganism (*e.g.,* all bacteria or all gram positive or negative bacteria). However, the present invention also contemplates microorganism targeting molecules that interact with a specific species or sub-species of microorganism. In some preferred embodiments, microorganism targeting molecules interact with "Pathogen Associated Molecular Patterns (PAMPS)". In some embodiments, microorganism targeting molecules are recognition molecules that are known to interact with or bind to PAMPS (*e.g.,* including, but not limited to, as CD14, lipopolysaccharide binding protein (LBP), surfactant protein D (SP-D), and Mannan binding lectin (MBL)). In other embodiments, microorganism targeting molecules are antibodies (*e.g.,* monoclonal antibodies directed towards PAMPS or monoclonal antibodies directed to specific organisms or serotype specific epitopes).

As used herein the term "biofilm" refers to an aggregation of microorganisms (e.g., bacteria) surrounded by an extracellular matrix or slime adherent on a surface *in vivo* or *ex vivo,* wherein the microorganisms adopt altered metabolic states.

As used herein, the term "host cell" refers to any eukaryotic cell (*e.g.,* mammalian cells, avian cells, amphibian cells, plant cells, fish cells, insect cells, yeast cells), and bacteria cells, and the like, whether located *in vitro* or *in vivo* (*e.g.,* in a transgenic organism).

As used herein, the term "cell culture" refers to any *in vitro* culture of cells. Included within this term are continuous cell lines (*e.g.,* with an immortal phenotype), primary cell cultures, finite cell lines (*e.g.,* non-transformed cells), and any other cell population maintained *in vitro,* including oocytes and embryos.

The term "isolated" when used in relation to a nucleic acid, as in "an isolated oligonucleotide" refers to a nucleic acid sequence that is identified and separated from at least one contaminant nucleic acid with which it is ordinarily associated in its natural source. Isolated nucleic acids are nucleic acids present in a form or setting that is different from that in which they are found in nature. In contrast, non-isolated nucleic acids are nucleic acids such as DNA and RNA that are found in the state in which they exist in nature.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

A "subject" is an animal such as vertebrate, preferably a mammal such as a human, a bird, or a fish. Mammals are understood to include, but are not limited to, murines, simians, humans, bovines, ovines, cervids, equines, porcines, canines, felines *etc*.).

An "effective amount" is an amount sufficient to effect beneficial or desired results. An effective amount can be administered in one or more administrations,

As used herein, the term "purified" or "to purify" refers to the removal of undesired components from a sample. As used herein, the term "substantially purified" refers to molecules, either nucleic or amino acid sequences, that are removed from their natural environment, isolated or separated, and are at least 60% free, preferably 75% free, and most preferably 90% free from other components with which they are naturally associated. An "isolated polynucleotide" is therefore a substantially purified polynucleotide.

The terms "bacteria" and "bacterium" refer to prokaryotic organisms, including those within all of the phyla in the Kingdom Procaryotae. It is intended that the term encompass all microorganisms considered to be bacteria including *Mycoplasma, Chlamydia, Actinomyces, Streptomyces,* and *Rickettsia.* All forms of bacteria are included within this definition including cocci, bacilli, spirochetes, spheroplasts, protoplasts, *etc.* Also included within this term are prokaryotic organisms that are gram negative or gram positive. "Gram negative" and "gram positive" refer to staining patterns with the Gram-staining process that is well known in the art. (*See e.g.,* Finegold and Martin, Diagnostic Microbiology, 6th Ed., CV Mosby St. Louis, pp. 13-15 [1982]). "Gram positive bacteria" are bacteria that retain the primary dye used in the Gram stain, causing the stained cells to appear dark blue to purple under the microscope. "Gram negative bacteria" do not retain the primary dye used in the Gram stain, but are stained by the counterstain. Thus, gram negative bacteria appear red. In some embodiments, the bacteria are those capable of causing disease (pathogens) and those that cause product degradation or spoilage.

"Strain" as used herein in reference to a microorganism describes an isolate of a microorganism (e.g., bacteria, virus, fungus, parasite) considered to be of the same species but with a unique genome and, if nucleotide changes are non-synonymous, a unique proteome differing from other strains of the same organism. Typically strains may be the result of isolation from a different host or at a different location and time but multiple strains of the same organism may be isolated from the same host.

As used herein "Complementarity Determining Regions" (CDRs) are those parts of the immunoglobulin variable chains which determine how these molecules bind to their specific antigen. Each immunoglobulin variable region typically comprises three CDRs and these are the most highly variable regions of the molecule.

As used herein, the term "motif" refers to a characteristic sequence of amino acids forming a distinctive pattern.

The term "Groove Exposed Motif" (GEM) as used herein refers to a subset of amino acids within a peptide that binds to an MHC molecule; the GEM comprises those amino acids which are turned inward towards the groove formed by the MHC molecule and which play a significant role in determining the binding affinity. In the case of human MHC-I the GEM amino acids are typically (1,2,3,9). In the case of MHC-II molecules two formats of GEM are most common comprising amino acids (-3,2,-1,1,4,6,9,+1,+2,+3) and (-3,2,1,2,4,6,9,+1,+2,+3) based on a 15 -mer peptide with a central core of 9 amino acids numbered 1-9 and positions outside the core numbered as negative (N terminal) or positive (C terminal).

"Immunoglobulin germline" is used herein to refer to the variable region sequences encoded in the inherited germline genes and which have not yet undergone any somatic hypermutation. Each individual carries and expresses multiple copies of germline genes for the variable regions of heavy and light chains. These undergo somatic hypermutation during affinity maturation. Information on the germline sequences of immunoglobulins is collated and referenced by www. imgt.org [3]. "Germline family" as used herein refers to the 7 main gene groups, catalogued at IMGT, which share similarity in their sequences and which are further subdivided into subfamilies.

"Affinity maturation" is the molecular evolution that occurs during somatic hypermutation during which unique variable region sequences generated that are the best at targeting and neutralizing and antigen become clonally expanded and dominate the responding cell populations.

"Germline motif" as used herein describes the amino acid subsets that are found in germline immunoglobulins. Germline motifs comprise both GEM and TCEM motifs found in the variable regions of immunoglobulins which have not yet undergone somatic hypermutation. "Immunopathology" when used herein describes an abnormality of the immune system. An immunopathology may affect B-cells and their lineage causing qualitative or quantitative changes in the production of immunoglobulins. Immunopathologies may alternatively affect T-cells and result in abnormal T-cell responses. Immunopathologies may also affect the antigen presenting cells. Immunopathologies may be the result of neoplasias of the cells of the immune system. Immunopathology is also used to describe diseases mediated by the immune system such as autoimmune diseases. Illustrative examples of immunopathologies include, but are not limited to, B-cell lymphoma, T-cell lymphomas, Systemic Lupus Erythematosus (SLE), allergies, hypersensitivities, immunodeficiency syndromes, radiation exposure or chronic fatigue syndrome.

"Obverse" as used herein describes the outward directed face or the side facing outwards. Hence, in the context of a pMHC complex, the obverse side is that face presented to the T-cell receptor and comprises the space-shape made up of the TCEM and the contiguous and surrounding outward facing components of the MHC molecule that will be different for each different MHC allele.

"pMHC" Is used to describe a complex of a peptide bound to an MHC molecule. In many instances a peptide bound to an MHC-I will be a 9-mer or 10-mer however other sizes of 7-11 amino acids may be thus bound. Similarly MHC-II molecules may form pMHC complexes with peptides of 15 amino acids or with peptides of other sizes from 11-23 amino acids. The term pMHC is thus understood to include any short peptide bound to a corresponding MHC. "Somatic hypermutation" (SHM), as used herein refers to the process by which variability in the immunoglobulin variable region is generated during the proliferation of individual B-cells responding to an immune stimulus. SHM occurs in the complementarity determining regions.

"T-cell exposed motif" (TCEM), as used herein, refers to the sub set of amino acids in a peptide bound in a MHC molecule which are directed outwards and exposed to a T-cell binding to the pMHC complex. A T-cell binds to a complex molecular space-shape made up of the outer surface MHC of the particular HLA allele and the exposed amino acids of the peptide bound within the MHC. Hence any T-cell recognizes a space shape or receptor which is specific to the combination of HLA and peptide. The amino acids which comprise the TCEM in an MHC-I binding peptide typically comprise positions 4, 5, 6, 7, 8 of a 9-mer. The amino acids which comprise the TCEM in an MHC-II binding peptide typically comprise 2, 3, 5, 7, 8 or -1, 3, 5, 7, 8 based on a 15-mer peptide with a central core of 9 amino acids numbered 1-9 and positions outside the core numbered as negative (N terminal) or positive (C terminal). As indicated under pMHC, the peptide bound to a MHC may be of other lengths and thus the numbering system here is considered a non-exclusive example of the instances of 9-mer and 15 mer peptides.

"Regulatory T-cell" or "Treg" as used herein, refers to a T-cell which has an immunosuppressive or down-regulatory function. Regulatory T-cells were formerly known as suppressor T-cells. Regulatory T-cells come in many forms but typically are characterized by expression CD4+, CD25, and Foxp3. Tregs are involved in shutting down immune responses after they have successfully eliminated invading organisms, and also in preventing immune responses to self-antigens or autoimmunity.

"Tregitope" as used herein describes an epitope to which a Treg or regulatory T-cell binds. "uTOPE^{™} analysis" as used herein refers to the computer assisted processes for predicting binding of peptides to MHC and predicting cathepsin cleavage, described in PCT US2011/029192, PCT US2012/055038, and US2014/01452.

"Framework region" as used herein refers to the amino acid sequences within an immunoglobulin variable region which do not undergo somatic hypermutation.

"Isotype" as used herein refers to the related proteins of particular gene family. Immunoglobulin isotype refers to the distinct forms of heavy and light chains in the immunoglobulins. In heavy chains there are five heavy chain isotypes (alpha, delta, gamma, epsilon, and mu, leading to the formation of IgA, IgD, IgG, IgE and IgM respectively) and light chains have two isotypes (kappa and lambda). Isotype when applied to immunoglobulins herein is used interchangeably with immunoglobulin "class".

"Isoform" as used herein refers to different forms of a protein which differ in a small number of amino acids. The isoform may be a full length protein (i.e., by reference to a reference wild-type protein or isoform) or a modified form of a partial protein, i.e., be shorter in length than a reference wild-type protein or isoform.

"Class switch recombination" (CSR) as used herein refers to the change from one isotype of immunoglobulin to another in an activated B cell, wherein the constant region associated with a specific variable region is changed, typically from IgM to IgG or other isotypes.

"Immunostimulation" as used herein refers to the signaling that leads to activation of an immune response, whether said immune response is characterized by a recruitment of cells or the release of cytokines which lead to suppression of the immune response. Thus immunostimulation refers to both upregulation or down regulation.

"Up-regulation" as used herein refers to an immunostimulation which leads to cytokine release and cell recruitment tending to eliminate a non self or exogenous epitope. Such responses include recruitment of T cells, including effectors such as cytotoxic T cells, and inflammation. In an adverse reaction upregulation may be directed to a self-epitope.

"Down regulation" as used herein refers to an immunostimulation which leads to cytokine release that tends to dampen or eliminate a cell response. In some instances such elimination may include apoptosis of the responding T cells.

"Frequency class" or "frequency classification" as used herein is used to describe the counts of TCEM motifs found in a given dataset of peptides. A logarithmic (log base 2) frequency categorization scheme was developed to describe the distribution of motifs in a dataset. As the cellular interactions between T-cells and antigen presenting cells displaying the motifs in MHC molecules on their surfaces are the ultimate result of the molecular interactions, using a log base 2 system implies that each adjacent frequency class would double or halve the cellular interactions with that motif. Thus using such a frequency categorization scheme makes it possible to characterize subtle differences in motif usage as well as providing a comprehensible way of visualizing the cellular interaction dynamics with the different motifs. Hence a Frequency Class 2, or FC 2 means 1 in 4, a Frequency class 10 or FC 10 means 1 in 2¹⁰ or 1 in 1024.

"40K set" as used herein refers to the database of 40,000 IGHV assembled from Genbank as described in Example 1

"IGHV" as used herein is an abbreviation for immunoglobulin heavy chain variable regions "IGLV" as used herein is an abbreviation for immunoglobulin light chain variable regions "Adverse immune response" as used herein may refer to (a) the induction of immunosuppression when the appropriate response is an active immune response to eliminate a pathogen or tumor or (b) the induction of an upregulated active immune response to a self-antigen or (c) an excessive up-regulation unbalanced by any suppression, as may occur for instance in an allergic response.

As used herein "epitope mimic" or "TCEM mimic" is used to describe a peptide which has an identical or overlapping TCEM, but may have a different GEM. Such a mimic occurring in one protein may induce an immune response directed towards another protein which carries the same TCEM motif. This may give rise to autoimmunity or inappropriate responses to the second protein.

As used herein "MART" refers to melanoma associated antigen recognized by T cells

As used herein "PMEL" refers to Melanocyte protein

As used herein "MAGE" refers to the protein product of any member of the melanoma-associated antigen gene family

"Anchor peptide", as used herein, refers to peptides or polypeptides which allow binding to a substrate to facilitate purification or which facilitate attachment to a solid medium such as a bead or plastic dish or are capable of insertion into a membrane of a cell or liposome or virus like particle. Among the examples of anchor peptides are the following, which are considered non limiting, his tags, immunoglobulins, Fc region of immunoglobulin, G coupled protein, receptor ligand, biotin, and FLAG tags

"Cytotoxin" or "cytocide" as used herein refers to a peptide or polypeptide which is toxic to cells and which causes cell death. Among the non-limiting examples of such polypeptides are RNAses, phospholipase, membrane active peptides such as cercropin, and diphtheria toxin. Cytotoxin also includes radionuclides which are cytotoxic.

"Cytokine" as used herein refers to a protein which is active in cell signaling and may include, among other examples, chemokines, interferons, interleukins, lymphokines, granulocyte colony-stimulating factor tumor necrosis factor and programmed death proteins.

As used herein the term "Alpha emitter" refers to a radioisotope which emits alpha radiation. Examples of alpha emitters which may be suitable for clinical use include Astatine-211, Bismuth-212, Bismuth-213, Actinium-225 Radium-223, Terbium-149, Fermium-255

As used herein "Auger particles" refers to the low energy electrons emitted by radionuclides such as but not limited to, Gadolinium-67, Technicium-99, Indium-111, lodine-123, lodine-125, Tellurium-201. Auger electrons are advantageous as they have a short path of transit through tissue.

As used herein "oncoprotein" means a protein encoded by an oncogene which can cause the transformation of a cell into a tumor cell if introduced into it. Examples of oncoproteins include but are not limited to the early proteins of papillomaviruses, polyomaviruses, adenoviruses and herpesviruses, however oncoproteins are not necessarily of viral origin.

"Label peptide" as used herein refers to a peptide or polypeptide which provides, either directly or by a ligated residue, a colorimetric , fluorescent, radiation emitting, light emitting, metallic or radiopaque signal which can be used to identify the location of said peptide. Among the non-limiting examples of such label peptides are streptavidin, fluorescein, luciferase, gold, ferritin, tritium,

"MHC subunit chain" as used herein refers to the alpha and beta subunits of MHC molecules. A MHC II molecule is made up of an alpha chain which is constant among each of the DR, DP, and DQ variants and a beta chain which varies by allele. The MHC I molecule is made up of a constant beta macroglobulin and a variable MHC A, B or C chain.

### DESCRIPTION OF THE INVENTION

The present invention provides methods and systems for identifying and classifying epitopes and use of that information to analyze proteins and peptides within proteins, especially potential epitopes, and to use the information to design synthetic peptides and proteins, analyze biopharmaceutical and other proteins, and diagnose autoimmune conditions and immunopathologies.

Any protein taken up by an antigen presenting cell (APC) can be processed to lead to stimulation of an immune response. Proteins may be derived from endogenous sources, such as cellular proteins and antibodies or from exogenous sources, including but not limited to pathogens, environmental and microbiome microorganisms, allergens and other environmental proteins. Antigen presenting cells include, but are not limited to, dendritic cells, B-cells and macrophages. Peptides may be presented on the surface of any cell bound to MHC molecules. Each cell carries MHC molecules encoded in various gene loci and heterozygous copies thereof. Each allele of each MHC locus has a unique binding groove which engages peptides released by enzyme action from proteins. The endopeptidase cleavage to release peptides and the binding reaction between short peptides and the MHC molecules has been well studied and modelled (see, e.g., PCT US2011/029192, PCT US2012/055038, US2014/014523, and PCT US2014/041525. The present invention addresses characterization of the interaction between a T-cell receptor and the complex of a peptide bound in a MHC molecular groove. The peptides which binding MHC grooves are typically a 9-mer binding an MHC-I and a 15-mer binding in a MHC-II groove. In a preferred embodiment therefore these peptide sizes are used throughout the analyses presented herein. However these peptide lengths should not be considered limiting and the same processes can be implemented and used as the basis for analysis of peptides of 7-11 amino acids in the case of MHC -I molecules and peptides of 11-23 amino acids for MHC-II alleles.

Peptides which are bound in MHC grooves comprise two sets of amino acids: those that face inwards into the groove and determine the binding affinity to the MHC molecule (the groove exposed motifs or GEM) and those which do not interact with the groove, but rather are on the obverse side exposed outwardly to the T-cells (the T-cell exposed motifs or TCEM). In the case of MHC-I, molecules the central amino acids 4, 5, 6, 7, 8 of the typical nonamer peptide bound form the T-cell exposed motif or TCEM, while the binding affinity to the groove is determined by amino acids 1, 2, 3, 9, the groove exposed motif or GEM. It has been recognized that two sets of amino acid positions may form these configurations for MHC-II presentation (Rudolph et al How TCRs bind MHCs, peptides, and coreceptors. Ann Rev Immunol (2006) 24:419-466 [2]). In the context of a MHC-II binding groove defined as a 15 amino acid chain:
- TCEM IIa exposes amino acids in pocket positions (2,3,5,7,8) with the corresponding GEM Ila amino acids as (-3,2,-1,1,4,6,9,+1,+2,+3)
- TCEM IIb exposes (-1,3,5,7,8) with the corresponding GEM IIb (-3,2,1,2,4,6,9,+1,+2,+3)

Figure 1 illustrates the amino acid and MHC pocket position numbering scheme used herein. It should be understood that any given peptide may comprise both TCEM which are formed by binding in MHC-I grooves and TCEM which are formed by binding in MHC-II molecule grooves; and indeed that a single peptide may comprise not only MHC-I and MHC-II TCEMs, but also can fulfill the criteria of both TCEM IIa and TCEM IIb as described above. Furthermore any peptide may be arranged in an MHC groove in a canonical or non-canonical position and hence a TCEM may be exposed to a T cell in either orientation.

Any given TCEM may be combined with many different GEM to make up the entire MHC binding peptide. Which amino acids are found in the GEM positions is a function of the protein of origin, whether self or non-self. It follows that a GEM may be designed or engineered to provide a desired binding affinity. The GEM amino acids will determine binding affinity on an MHC allele and locus specific basis. Thus binding to MHC-IA and MHC-IB, and to MHC-II DR, DQ and DP alleles of MHCII all can result in higher or lower binding affinity GEMS. Such binding is also competitive relative to other excised peptides from the same protein or otherwise found in the same cellular location.

Based on these structural considerations of which amino acids determine pMHC binding affinity and which amino acids are exposed to T-cells as the obverse face of a pMHC complex, it is possible to categorize any set of peptides, irrespective of whether their source is from antibody molecules, molecules from pathogens, or from biotherapeutic molecules or any other protein. Whether a pMHC will be an upregulating or downregulating epitope, is a product of both its binding affinity (and hence dwell time) in the context of the host MHC alleles and the frequency with which T-cells have been previously exposed to that motif.

By combining predictions of pMHC binding affinities and endosomal processing with databases of TCEM motif frequencies, it is possible to create databases to cross reference molecules from different origins in order to make predictions as to their immunogenic potential as components of vaccines, and their role as possible sources of auto-immune responses or allergic responses, or as regulatory T-cell responses. One embodiment of the present invention is therefore to provide methods to quantify the frequency of TCEM motifs and to categorize them as frequent or rare with respect to a particular reference database of reference proteins. By so doing it becomes possible to predict whether a given TCEM, when combined with a GEM which provides more or less binding affinity in a host of a specific immunogenetic composition will contribute to up or down regulation of the adaptive immune response. Further, in one preferred embodiment, it enables the design of proteins which embody immunogens with desired properties.

The primary function of the adaptive immune system is to differentiate self from not-self and to allow the body to mount an appropriate response to molecules, once identified as self or as not-self. This may require that there is no response to a self-antigen (tolerization) and at the other extreme a very vigorous and rapid cellular, antibody and cytokine response to an invading pathogen. Calibration of the response according to the recognition of the antigen is the product of several layers of recognition and the balance of qualitative, quantitative, and temporal stimuli.

Qualitative stimuli include the characteristics of the TCEM motifs as presented in the context of the binding MHC molecule. Quantitative stimuli are determined by the GEM which determines the MHC binding affinity d, and hence dwell time of the peptide in the MHC, the number of T cells responding, as a function of the frequency of the TCEM motif, and the binding affinity between the T cell receptor and the pMHC complex.

One source of variation and diversity in peptide sequences that are exposed to the immune system is the generation of unique immunoglobulin variable region sequences as the result of somatic hypermutation. Each of the many germline immunoglobulin sequences an individual host carries may give rise to many different mutated sequences. The light chain and heavy chain variable regions, denoted IGHV and IGLV, are the parts of the molecule that undergo this molecular evolution to enable them to specifically recognize antigens. The specific, mutated sequences are found in both the B-cell receptors (BCR) and secreted immunoglobulin (Ig) molecules. After molecular evolution of the variable region sequences of the BCR the Ig molecules undergo class-switch recombination (CSR) and are secreted as immunoglobulin molecules, one unique sequence per cell clonal line. Thus, as the result of the somatic hypermutation, the immunoglobulin variable regions are a vast source of peptide variation. This is in addition to the background repertoire of peptides found within the un-mutated germline sequences.

Not only do the mutated immunoglobulin sequences become presented on B-cell surfaces as B-cell receptors and secreted as antibodies, but the same sequences, and those of germline sequences, are also processed by the endosomal enzymes of B-cells so that fragments of the sequences become presented on the surface of B-cells bound in the grooves of the MHC molecules [4,5]. B-cells therefore display peptide fragments of immunoglobulin variable regions as both MHC-I and MHC-II complexes with bound peptides (pMHC). The pMHC are recognized by contacting T-cells and based on such recognition may give rise to secondary cellular responses. Hence, immunoglobulin variable regions form an important source of education of the T-cell repertoire in the genetically defined context of the individual's MHC molecules.

T-cells are exposed in the thymus to self-antigens and certain clones of T-cell precursors are deleted. Further education of T-cells occurs through on going exposure to self-antigens. An additional tier of T-cell stimulation is then provided on a continuous basis by exposure to the diversity of motifs generated through antibody hypermutation. This allows T-cells to be exposed to motifs which are common and those which are rare. Those which are common motifs, if they are also associated with a long dwell time in the MHC due to a high biding affinity, tend to down regulate T-cells.

In one embodiment, the present invention identifies a set of TCEM sequences which are common and which are likely to result in down-regulation. While occasional reports of such Tregs have been recorded [6], the present invention provides a comprehensive and systematic way to identify high frequency down regulatory motifs on an allele specific basis. It further provides sequence identification of such high frequency motifs.

A further source of TCEMs exposed to the immune response is the immunoglobulin constant region. Each isotype or class of immunoglobulin (IgM, IgA, IgD, IgE, IgG and the subclasses thereof) comprises a different sequence and hence a different set of TCEM motifs. Within each isotype the sequences and hence motifs are highly conserved. As we show, the repertoire of motifs found in the constant regions are different from those found in the variable region. It appears therefore that motifs in the constant region are excluded from the 'vocabulary" of motifs used in the variable region in naturally derived immunoglobulins. Constant region motifs -as they are indeed constant - are the most frequent class of TCEM motifs derived from immunoglobulins. It follows therefore that when these motifs are also high binding affinity they may stimulate Tregulatory or suppressor responses. In one embodiment therefore the present invention identifies constant regions TCEM motifs which have high predicted binding affinity to various MHC alleles and are predicted to have a T-regulatory function when bound by these MHC alleles.

A further source of TCEMs which lead to modulation of the T cell repertoire is the proteins of the microbiome, whether this be the skin, mucosal, or gastrointestinal microbiome. In addition, the pathogens, environmental proteins and allergens to which an individual is exposed during their lifetime will further mold their T cell repertoire. In another set of embodiments therefore databases comprising the proteomes of microorganisms, or allergens are assembled and the TCEMs therein examined and categorized according to the frequency distribution of the TCEMs in constituent peptides and the predicted binding affinity of the intercalated GEM motifs. This enables not only the better design of vaccines but understanding of mechanisms of immune evasion. This may arise when microorganisms evolve to utilize immunoglobulin or human proteome like motifs. Differing patterns of motif usage may be detected in bacterial pathogens with chronic intracellular habitats and viruses characterized by latent infection as compared to those which have a rapid infectious course. By understanding the patterns of evasion based on TCEM usage more effective methods of intervention in infectious disease may be developed,

In further embodiments, this invention provides a method to evaluate biopharmaceutical proteins to determine the probability that they will cause an adverse immune response. In another embodiment the method can be applied to evaluate a vaccine candidate both for the presence of unique TCEMs likely to lead to a protective or therapeutic immune response and to determine the presence of down regulating TCEMs which will tend to negate a protective response.

As high speed and deep sequencing capabilities increase the number of antibody variable regions available for analysis will increase exponentially, hence the capability described herein will find increasing importance in analysis of immunoglobulin hypervariable regions as a database of TCEMs. In parallel, as sequencing of other proteomes and, in particular, as increased accessibility of individual human proteomes is available, the methods described herein provide a systematic approach to matching TCEMs identified in pathogens, antibodies and other environmental immune stimuli to the TCEMs found in the human proteome, and hence enabling recognition of potential mimics which can give rise to autoimmune responses. Further, as protein therapeutics are the fastest growing class of drugs, it becomes increasingly important to understand whether these are likely to provoke adverse immunogenicity or autoimmune responses.

Autoimmune diseases, in which the immune response is directed against self-epitopes constitute a very large and serious group of diseases. Autoimmune diseases include an array of dysregulations or imbalances of self-recognition. Adverse immune responses may arise through up-regulation (for instance when an infectious agent provides an epitope mimic) or conversely by loss of self-tolerance. By being able to identify epitope mimics which can give rise to autoimmune responses, the present invention also offers a means to design interventions to mitigate autoimmune responses. This may be approached by administration of motifs to upregulate or down regulate the response the mimic and either to induce immunity or to repair tolerance. Such intervention would require the approach provided by the present invention to identify the epitope motif and categorize its ability to upregulate or downregulate in the context of the specific host HLA.

Among the diseases recognized to be the result of autoimmunity, or to have an autoimmune component, are celiac disease, narcolepsy, rheumatoid arthritis and multiple sclerosis (Jones, E. Y. et al , 2006. Nat.Rev.Immunol. 6:271-282.). Other autoimmune diseases include but are not limited to Ankylosing Spondylitis, Atopic allergy, Atopic Dermatitis, Autoimmune cardiomyopathy, Autoimmune enteropathy, Autoimmune hemolytic anemia, Autoimmune hepatitis, Autoimmune inner ear disease, Autoimmune lymphoproliferative syndrome, Autoimmune peripheral neuropathy, Autoimmune pancreatitis, Autoimmune polyendocrine syndrome, Autoimmune progesterone dermatitis, Autoimmune thrombocytopenic purpura, Autoimmune uveitis, Bullous Pemphigoid, Castleman's disease, Celiac disease, Cogan syndrome, Cold agglutinin disease, Crohn's Disease, Dermatomyositis, Diabetes mellitus type 1, Eosinophilic fasciitis, Gastrointestinal pemphigoid, Goodpasture's syndrome, Graves' disease, Guillain-Barré syndrome, Anti-ganglioside Hashimoto's encephalitis, Hashimoto's thyroiditis, Systemic Lupus erythematosus, Miller-Fisher syndrome, Mixed Connective Tissue Disease, Myasthenia gravis, Pemphigus vulgaris, Polymyositis, Primary biliary cirrhosis, Psoriasis, Psoriatic Arthritis, Relapsing polychondritis, Rheumatoid arthritis, Sjögren's syndrome, Temporal arteritis, Ulcerative Colitis, Vasculitis, and Wegener's granulomatosis.

In a number of other instances microbial infections are known to lead to a subsequent autoimmune reaction, including, for example but not limited to, in Lyme Disease, Streptococcal infections, and chronic respiratory infections (Hildenbrand, P. et al, 2009. Am.J.Neuroradiol. 30:1079-1087; Lee, J. L. et al, Autoimmun.Rev. 10.1016 .2009; Leidinger, P. et al Respir.Res. 10:20., 2009), Guillan Barre (Yuki N (2001) Lancet Infect Dis 1 (1): 29-37, Yuki N (2005) Curr Opin Immunol 17 (6): 577-582,; Kieseier BC et al, (2004) Muscle Nerve 30 (2): 131-156), rheumatoid arthritis (Rashid T et al (2007) Clin Exp Rheumatol 25 (2): 259-267), rheumatic fever (Guilherme L, Kalil J (2009) J Clin Immunol),. In one embodiment the computer based analysis system described herein allows characterization of TCEMs which are epitope mimics and can be applied to a variety of potential mimic substrates, including but not limited to vaccines, biotherapeutic drugs, food ingredients and the like, to enable prediction of whether an adverse reaction could arise through exposure of an individual to a molecular mimic and which individuals (i.e. comprising which HLA haplotypes) may be most at risk.

The development of vaccines can result in a large population of subjects being exposed to a novel array of epitope motifs. A number of situations have arisen in which adverse immune responses to vaccines have resulted through autoimmune reactions. As one non-limiting example, Influenza vaccination has been associated with Guillan Barre disease (Vellozzi Clin Infect Dis 2014) and with narcolepsy ( (Ahmed et al 2015 [7]). The present invention therefore provides methods to evaluate vaccines and identify potential mimics and the individuals in which such autoimmune responses may occur. It further provides a means of pharmacovigilance for reacting to reports of adverse reactions as vaccines are applied to a wider population.

Certain pathologies of B-cells result in the production of abnormal antibodies and the disruption of normal patterns of SHM. Examples of such diseases include lymphomas, leukemias, lupus erythematosus; such examples should not be considered limiting. B-cell lymphomas may arise as the result of infection as in Burkett's lymphoma or be the product of other causes of mutagenesis. Among the B-cell lymphomas are diffuse large B-cell lymphoma, Follicular lymphoma Mucosa-Associated Lymphatic Tissue lymphoma, Small cell lymphocytic lymphoma, Mantle cell lymphoma, Burkett's lymphoma, Mediastinal large B-cell lymphoma, Waldenström macroglobulinemia, Nodal marginal zone B-cell lymphoma, Splenic marginal zone lymphoma), Intravascular large B-cell lymphoma, Primary effusion lymphoma, and Lymphomatoid granulomatosis. B-cell leukemias include B-cell chronic lymphocytic leukemia/small lymphocytic lymphoma, Acute lymphoblastic leukemia, B-cell prolymphocytic leukemia, Precursor B lymphoblastic leukemia and Hairy cell leukemia. In each of these immunopathologies the characteristics of the epitope motif frequencies, including the TCEM, found in the immunoglobulins produced, is changed as immunoglobulin synthesis is disrupted or maturation is changed. A further embodiment of the present invention is to characterize the patterns of TCEM found in such abnormal antibodies. In one application of this capability the invention enables diagnosis of abnormalities of immunoglobulins and the underlying immunopathologies. In a further embodiment, the invention offers a means of motif characterization needed to design interventions for some B-cell pathologies, whether by introduction of up regulatory or down regulatory stimuli.

Personalized medicine approaches are of increasing interest to the health sector. FDA published a Guidance (2013 Paving the Way for Personalized Medicine FDA's Role in a New Era of Medical Product Development U.S. Department Of Health And Human Services U.S. Food And Drug Administration) in which it notes that paradigmatic developments in science and technology offer new promise for developing targeted therapeutics and tools for predicting who will respond to a medical therapy or who will suffer ill effects. The present invention offers just such a development in that it allows better understanding of the interaction between individual immunogenetics and protein immunogens and the development of biotherapeutics and vaccines specifically targeted to an individual patient or immunogenetic subgroup.

The design of clinical trials to evaluate new protein drug products and vaccines can be enhanced by a better understanding of the role of immunogenetics. More efficient use of enrollees can be made if the trial is designed with reference to the immunogenetics of the patients. An embodiment of the present invention is therefore to identify similarities in the response of patients to protein drugs and vaccines based on the patient HLA and to stratify the trial accordingly. This is because the individual HLA will affect both binding affinity of GEM and the context of presentation of TCEM. A particular embodiment of the invention is to anticipate potential effects of TCEM that are found in the drug product tested based on potential autoimmune mimics and the stratification of the MHC binding affinity of the corresponding GEMs.

The methods provided herein also allow analysis of the epitopes associated with cancer. Tumor associated proteins may be analyzed to identify regions of predicted high affinity MHC binding and antibody targets. Among the epitopes are those which have TCEMs that are also found at high frequency and in germline and mutated immunoglobulin sequences. This tends to give rise to immunosuppression. Furthermore, tolerance to tumor surface antigens tends to inhibit an aggressive immune response from attacking and destroying cancer cells. Tolerance may arise as the result of excessive stimulation by a particular TCEM over time resulting in down regulation. By defining the TCEMs found in tumor associated antigen proteins it becomes possible to design interventions to upregulate or downregulate the anti-tumor responses by introduction of peptides with matching TCEMs but different levels of MHC binding affinity reflected in their GEM sequences. The ability to identify TCEM and to use them in conjunction with modified GEMs to alter the dwell time in contact with a MHC, and hence whether they lead to up or down regulation, is one approach to modulation of a T-cell response.

In some embodiments the discovery of immunosuppressive epitopes can be a step in the process of designing immunomodulatory interventions. A protein comprises some epitopes which up-regulate the CD4+ and CD8+ immune responses and some epitope peptides which result in a down-regulation or T-regulatory response to the tumor associated antigen. The balance of upregulation and down regulation is essential to the outcome of the overall immune response. As previously described, the function of a TCEM as an upregulator or down regulator will depend not only on the frequency of prior exposure to the TCEM motif but also to the affinity of binding and hence the MHC dwell time of the peptide of interest. In one embodiment of the present invention tumor associated antigens are examined to determine the position of both upregulating and down regulating epitopes. The design of vaccinal peptides will be guided by those peptides which are upregulating, but which also are not compromised by downregulating peptides, either in the immediate proximity or in the same protein.

The present invention also offers a means of designing patient-specific cancer interventions. In some embodiments, analysis of the balance between upregulating and downregulating epitopes can assist in understanding the progression of cancer. Tumor associated antigen proteins comprise both up and down regulating epitopes based on the combination of exposed TCEM motifs and the affinity of GEM binding. In some embodiments the present invention enables analysis that demonstrates that different isoforms or splice variants of tumor associated antigens differ in the presence and location of T regulatory down regulating epitopes which may depend on the immunogenetic makeup of the host. In yet other embodiment examination of a tumor associated antigen using the methods described herein identifies a T regulatory epitope which may impede the development of a protective response to a vaccine. The tumor associated antigens which may be thus analyzed include, but are not limited to those which arise from mutation, tumor antigens that are shared between many tumors, differentiation antigens and antigens that are over expressed in tumors. Such tumor antigens are compiled in several databases including cancerimmunity.org. In some particular embodiments analysis is applied to tumor associated antigens including: abnormal spindle-like microcephaly associated splice variant 1, abnormal spindle-like microcephaly-associated protein isoform 2, ATPase family AAA domain-containing protein 2, Carbonic anhydrase 9, Carcinoembryonic antigen-related cell adhesion molecule 5, cyclin-dependent kinase 1 isoform 1, cytochrome b-c1 complex subunit 7 isoform 1, Endoplasmin, IQGAP3, kinesin-like protein KIF2C , Kinetochore protein Nuf2, Late secretory pathway protein AVL9 homolog, lipid phosphate phosphohydrolase 2 isoform 1, macrophage-stimulating protein receptor isoform 1 preproprotein, Matrix metalloproteinase 3, Melanocyte protein PMEL, MART Melanoma antigen recognized by T-cells 1, melanoma associated antigens (MAGE), Mucin-1, Mucin-6, Nuclear transcription factor Y subunit PROM1, protein prostate-specific antigen, Prostatic acid phosphatase, Receptor tyrosine-protein kinase erbB-2, receptor tyrosine-protein kinase erbB-3 isoform 1, serine threonine-protein kinase PLK4 isoform 1, Siah2 protein, soluble MET variant 6, Stromelysin-3 structural maintenance of chromosomes protein, tyrosinase, Tyrosine-protein kinase ABL1, ubiquitin carboxyl-terminal hydrolase L5. In one preferred embodiment the tumor associated antigen analyzed is expressed in melanoma and includes but is not limited, to MART, PMEL, MAGE antigens. In yet further embodiments, analysis is applied to characterize the neoantigens which arise spontaneously through mutation and which may give rise to cancer. Such progression is critically linked to the individual's immunogenetics and this may be the determining factor in whether progression to neoplasia results from any given mutation.

Having identified a peptide which carries a Treg motif opens the way to design interventions in which the down regulation of the immune response can be manipulated, to reduce or eliminate the down regulation. Hence limitations on an otherwise protective or upregulated immune response to a cancer associated protein or tumor associated antigen can be removed.

Another approach, enabled by the methods presented herein, is to use TCEM to target additional molecules to T-cells. Among these additional molecules, but not limited to them, are agents which are toxic to T-cells. Hence a cytotoxin or cytocide could be directed specifically to a T-cell by fusing it to, or expressing as a fusion with, a MHC molecule which binds to a peptide which has been selected based on its TCEM and with a GEM selected to give a high binding affinity to the MHC. Thus when a T-cell specifically binds to said TCEM it will be exposed to the cytotoxin or cytocide. In some embodiments, where a peptide motif that induces a T regulatory response is identified, that motif can be used to bind to an MHC and serve as a trap or binder for the specific corresponding T-cell. When said peptide motif is bound to an MHC molecule further linked to a cytotoxin, the TCEM motif can serve as a "poison pill" to very selectively trap and kill T-cells that bind to the particular peptide MHC complex. In some embodiments this can be used as a means of enhancing the response to a therapeutic vaccine by eliminating downregulating T cell responses. The present invention thus offers a means of identifying the peptide motifs which can be used to selectively eliminate certain adverse T-cell populations. Such adverse T-cells may play a role in the immune response to tumors, to infectious agents or in autoimmunity.

In an alternate example the MHC molecule is expressed in association with or fused to a cytokine so that when bound to a selected TCEM the cytokine cell signaling activates the T cell response, inflammatory response or alternatively downregulates it. In this case the GEM counterpart to the selected TCEM can be adjusted by changing amino acids to ensure a tight binding to the MHC molecule. A broad array of cytokines may be utilized and are well known to those skilled in the art but include among many other examples, interferons, interleukins, granulocyte stimulating factors and programmed death proteins.

A further application of a TCEM bound by a selected GEM to a tagged MHC molecule is a configuration which can assist in capturing the T cells of interest in vitro. In this application a MHC molecule is fused to, or expressed with, a second peptide of choice such as a His tag, Fc component of immunoglobulin, Flag tag or other tag which enables binding to a substrate such as a tissue culture dish, bead, or purification matrix. Similarly a suitable tag can be added to enable insertion into a cell membrane or a virus like particle or liposome to "decorate" such carriers with the pMHC with a desired TCEM, necessary to attract and bind certain T cells of interest *in vitro* or *in vivo.*

Underlying the ability to categorize TCEM as common or rare is the development of reference databases of TCEM and the peptides of which they form a part. The present invention in one embodiment provides methods to generate such reference databases and to enable the extraction and ranking of TCEM and GEM motifs. This method of extraction may be applied to sequences assembled from public or private repositories of immunoglobulin sequences, including but not limited to Genbank and IMGT, but also to human proteome sequence databases. In some embodiments the relevant databases are of T cell receptor proteins. In yet further embodiments the source database repository is of microbes or allergens, such as PATRIC, VIPR (www. patric.org, http://www.viprbrc.org), allergen (Structural Database of Allergenic Proteins database http://fermi.utmb.edu/SDAP/index.html), microbiome (www.hmpdacc.org/HMRGD) and biopharmaceuticals (IMGT.org). The extraction of the discontinuous motifs are an essential step to allowing identification of matching motifs in proteins of different sources and in the reference databases. The present invention teaches the importance of such motif extraction and provides methods for performing extraction. This capability is expected to have great utility as the accessibility of high throughput deep sequencing increases.

The administration of peptide mixtures which are shown to have an immunosuppressive effect is known to the art [8]. One specific example of this is the administration of an empirically derived mixture polymers of four amino acids to make Glatirimer acetate. The present invention offers a means to proactively design an immunosuppressive peptide mixture rather than depending on empirical experimentation.

In some particular embodiments the present invention allows the identification of TCEM motifs that are particularly likely to be immunosuppressive. A TCEM motif that occurs in IGHV more frequently than 1 in 4000, corresponding to occurrence in up to and including frequency class 12, and especially those occurring more often than 1 in 1000 corresponding to frequency class 10, whether of germline or somatically mutated origin would be likely to be recognized as self. This is also the case for TCEM motifs occurring in immunoglobulin constant regions of any isotype. If TCEMS of either source (constant or variable chain) also comprise a high affinity GEM, then they would be likely to generate a T regulatory response. In some particular embodiments therefore, the present invention enables the design of synthetic peptides and synthetic polypeptide compositions which would bind to MHC II and invoke an immunosuppressive response. Such peptides may include a single TCEM motif. In yet other embodiments a synthetic polypeptide may be generated which includes multiple TCEM motifs each derived from FC 1-12 of immunoglobulin variable region origin or constant region and each capable of contributing a suppressive effect. In some embodiments it may be desired to modulate the immunosuppressive effect by inclusion only of TCEM motifs from FC 6-12, to reduce the overall weight of immunosuppressive stimuli. In yet other embodiments an immunosuppressive motif or motifs may be selected with a particular specific antigen target of immunosuppression in mind; in others the intent may be to generate a non-specific immunosuppression.

In other embodiments identifying patterns of TCEM in microorganisms can aid understanding of strategies of immune evasion adopted by various organisms. In these circumstances understanding whether a TCEM is used commonly or rarely within the proteome of a specific organism may be important to assess immune response.

In some embodiments particular configurations of the immunosuppressive TCEMs may be selected. As examples, which are non-limiting, a polypeptide comprising the TCEM may be comprised of a limited number of amino acids. In some preferred embodiments this may be only 3 or 4 or 5 amino acids, or some other subset, while in other embodiments it may be the full repertoire of 20 amino acids. In yet other embodiments the TCEM with immunosuppressive properties may be bound to an MHC molecule which is linked to a cytokine in order to generate an immunosuppressive cytokine response in the proximity of T cells that bind to the protein of interest in a specific manner.

In some embodiments such immunosuppressive peptides and polypeptides may be administered to subjects as an intervention for the management of autoimmune diseases. Such autoimmune diseases are many, but include multiple sclerosis, lupus erythematosus, rheumatoid arthritis and others as are listed elsewhere in this specification.

It will be further understood that in the development of a vaccine to protect against an infectious microorganism (virus, bacteria, mycoplasma, fungus, parasite, etc.) the key challenges are to identify immunogens which will confer protective immunity, whether by generation of neutralizing antibody or by a cytotoxic cellular response. In this process it is important to understand which potential epitopes for vaccine inclusion will provide an upregulation of T-helper cells and T-effecter cells and stimulate antibody production. It is equally important to understand which epitopes may lead to immunosuppression or to the generation of an antibody response which does not generate any immune memory enabling recall. In some situations this evaluation must be constantly updated in light of the obtaining of new field isolates of an organism so as to determine if a proposed or approved vaccine is still relevant to current field isolates.

In the face of the recent epidemic of Ebolavirus (EBOV) disease this situation arises, where a number of reference strains of the virus, some dating from as long as 40 years ago, must be compared to current day isolates. In one particular embodiment therefore the present invention enables the conduct of immunovigilance to compare the presence of immunostimulatory epitopes and immunosuppressive epitopes in multiple strains of a virus. The examination of current strains also enables the design of a vaccine in which specific upregulatory epitope motifs are identified and then further selected based on the absence of probable immunosuppressive motifs. Such probable T-suppressive motifs are identified by reference to a reference database of T-cell exposed motifs. While EBOV is cited as a topical example, this example should not be considered limiting and the same strategy may be applied to diseases of other infectious microbial etiology. In a further non-limiting example the same strategy is applied to influenza. The characteristics which enable design of a good vaccine also can be applied to develop a good immunogen for the generation of antibodies in an animal model, thereby facilitating the preparation of hybridomas which serve as the basis for developing antibody based immunotherapies, providing a yet further embodiment of the invention.

Although these examples pertain to two viral diseases the same approach is equally applicable to development of vaccines and evaluation of epitope changes in bacterial, fungal and parasite microorganisms.

### EXAMPLES:

### Example 1: Assembly of motifs in immunoglobulin variable regions as a reference database

As of December 2013, Genbank contained approximately 45,000 sequences identified as immunoglobulin heavy variable regions (IGHV) and for which the repository metadata records did not contain indications that they were derived from individuals with immunopathology. Some sequences were submitted in conjunction with specific publications; others were direct submissions. The majority do not indicate a source molecule isotype. Genbank does not provide a means to identify and link sequence submissions in the database for heavy and light chain immunoglobulins from the same molecule. In some cases these can be deduced from accession numbers.

Approximately 45,000 heavy chain variable regions were retrieved from NCBI Protein resource with a search argument "(immunoglobulin heavy chain variable region) AND (homo sapiens)". The numbers of IGHV greatly outnumber the number of light chain sequences. In addition, because of the way proteins are deposited and annotated the heavy chain and light chain variable region pairs are not explicitly connected. Therefore only IGHV sequences were used in this analysis. Restrictive combinations of search arguments were used to create non-redundant subsets of this larger set that were either immunoglobulin class-defined or for which the metadata attached to the accession indicated that they were associated with an immunopathology. Additionally, manual curation was used to remove sequences that were obviously not immunoglobulins. The final dataset thus included approximately 40,000 (n=39,957) non-class-defined immunoglobulins. As the resulting dataset comprises many different accession groups from studies carried out over a considerable period of time it can be considered a representative sample of the gamut of "natural" human immunoglobulins. Accessions with signal peptides were identified and removed from the input sequences using the combined signal peptide and transmembrane predictor Phobius (phobius.sbc.su.se). IGHV were included in the final set if they contained at least 80 amino acids, a value approximating the shortest germline equivalent sequence. All sequences longer than 130 amino acids were truncated at that point.

Class-defined IGHV sets of IgG (n=1630), IgE (n=667), and IgM (n=537) were derived similarly by adding additional key words to the search arguments. There are inevitable biases in the class-defined datasets. For example the sources of nearly all of the IgE sequences were from cohorts of asthmatics [9-11] and either did not include or identify the sequences of non-asthmatics in the cohorts. Likewise the IgG sequences were derived from an HIV study [12]. Germline IGHV (n=161) were obtained from the IMGT repository (www.imgt.org), and immunoglobulin heavy chain constant regions (IGHC) class reference sequences from Genbank. Additional database collections were assembled including 163 commercial biotherapeutic sequences (IMGT.org repository). Ig sequences where the secondary annotations specified derivation from an immunopathology were assembled by a Genbank query. These numbered approximately 4000.

The human proteome, exclusive of immunoglobulins, was downloaded from www.uniprot.org comprising approximately 81,000 proteins which includes multiple isoforms of some proteins.

These datasets, together with additional datasets described in subsequent Examples, were used as reference series for comparison of the binding patterns, determination of motif frequencies, and comparison of proteins of interest.

### Example 2: Extraction and scoring of motifs

For each of the analyses described below each sequence in the derived databases was broken into 15-mers and 9-mers, each offset by a single amino acid. Thus, the combined set of 40,000 IGHV sequences resulted in approximately 4.2 x 10⁶ peptides.

The same manipulations were subsequently carried out with the IGHV germline sequences, immunoglobulin constant regions, immunoglobulin light chain variable regions, and the human proteome and T cell receptor sequences.

For each derived 9-mer and 15-mer peptide, the predicted binding affinity to 37 MHC I and 28 MHC II alleles was determined. This was done using methods previously described [13]. Briefly, the principal components of physical properties of each amino acid were derived from a large set of published data. The first three principal components account for approximately 90% of the variability. For a peptide a matrix is constructed consisting of the descriptors for each of the constituent amino acids. Binding affinity datasets for the MHC I and MHC II alleles were obtained from IEDB (www.iedb.org) and used as training sets for neural network development (NN). Amino acid sequences of the peptides were converted to principal component matrices and a bootstrap aggregation "bagging" process was used with the training sets to produce ensembles of NN equations for each of the alleles [14,15]. The ensemble approach enables the computation of a predicted mean and variance of affinity for each component peptide. Predictions for each MHC for each protein will exhibit a unique distribution binding affinities. Thus for computations consisting of either binding affinities for a number of alleles or a number of different proteins the predicted mean affinities for each allele are standardized to zero mean and unit variance. This standardization is done within protein using a Johnson Sb algorithm (JMP platform) and the predictions for the protein reported in standard deviation units.

The probability of cleavage of each protein by human cathepsin B, L, or S was determined using methods previously described. Briefly, matrices of the amino acid principal components were derived as outlined above for a cleavage site octomer (CSO). By convention the scissile bond is between amino acids 4 and 5 of the CSO and is described as P1P1'. Large proteomic data sets of cleavage by the three cathepsins were used to produce the input cleavage training sets for development of a NN binary classifiers [16-18]. Bagging was used to create the ensemble predictors and the median of the probabilities of the predictive equation ensembles is used as the probability of scissile bond cleavage of a CSO. The accuracy of the predictors varies for the different cathepsins and for different P1P1' dipeptides. The overall median AROC for the classifiers is 0.87. Using this process a probability of cleavage by the each of cathepsins was computed for all possible octomers indexed by single amino acids from each immunoglobulin.

It should be noted that there is a substantial cellular tropism to cathepsin expression and not all APC have the same profile of cathepsins; B cells do not express cathepsin L. We have examined and have found a good overall concordance across a variety of published endosomal cleavage datasets such as CLIP processing [19] and display of self-peptides [20].

We extracted datasets of T-cell exposed motifs (TCEM) and groove exposed motifs (GEM) motifs from all 40,000 curated IGHV proteins. This was done by creating sets of 15-mer motifs, and then the corresponding sets of TCEM for MHC-I TCEM and MHC -II TCEMa and TCEMb, in which the different relevant TCEM positions are given their amino acid, while non-TCEM positions were replaced by a standard non-amino acid code ("^{~}") or an X. TCEM positions are shown in Figure 1. In this way it is possible using standard relational algebra of sets to extract replicated TCEMs as well as determining their frequencies of occurrence and their affiliated GEM binding affinity characteristics. Hence 15-mers with their associated TCEM and GEM were generated from all downloaded sequences.

We initially applied uTOPE^{™} MHC Binding affinity prediction analysis (see, e.g., PCT US2011/029192, PCT US2012/055038, US2014/01452, and US2014/041525 ) to a subset of the IGHV sequences. Because of computational resource limitations we initially did a full MHC binding prediction predictions on approximately a third of the 40K set (14 K sequences).Some figures provided herein are based on this initial subset. Having now completed the processing of the complete dataset no significant differences are seen in the figures, or in the conclusions drawn from the subset vs the whole dataset.

### Motif Extraction

Each of the mature somatic hypermutated (SHM) IGHV sequences comprise approximately 120 amino acids (without signal peptides) and thus produce approximately 110 motifs. Thus, the 40,000 IGHV proteins produced about 4.4 x 10⁶ peptides each having 3 different potential TCEM configurations. As any one discontinuous pentamer motif can have 20⁵ different configurations, or 3.2 million, 9.6 million total potential motifs exist in the following three possible configurations (as seen in Figure 1):
1. MCH IIA (2,3,5,7,8) as 15-mer
2. MHC IIB (-1,3,5,7,8) as 15-mer
3. MHC I (4,5,6,7,8) as 9-mer

We observed that there is a high level of motif re-use within the IGHV. Each of the 3 sets consisted of only approximately 275,000 unique motif sequences and thus there was significant motif re-use in different molecules. The motif usage frequencies were found to follow a power law (Pareto) distribution characteristic of network ensembles, as shown in Table 1.

**Table 1.**

| FC TCR II | N Rows | Mean(N TCR II | Mean(N TCR II |
|---|---|---|---|
| (-1,3,5,7,8) | | (-1,3,5,7,8), Germline) | (-1,3,5,7,8), Mutated) |
| 1 | 8 | 25,710 | |
| 2 | 56 | 15,205 | 13,919 |
| 3 | 43 | 6,453 | 6,022 |
| 4 | 140 | 3,683 | 3,417 |
| 5 | 215 | 1,715 | 1,639 |
| 6 | 500 | 908 | 878 |
| 7 | 571 | 451 | 430 |
| 8 | 1,136 | 225 | 215 |
| 9 | 1,853 | 115 | 110 |
| 10 | 3,146 | 61 | 54 |
| 11 | 6,372 | 29 | 27 |
| 12 | 12,592 | 14 | 13 |
| 13 | 24,279 | 7 | 6 |
| 14 | 34,571 | 4 | 3 |
| 15 | 55,298 | 2 | 2 |
| 16 | 135,960 | 1 | 1 |

Motifs of germline origin were processed in the same manner as the non-germline somatically hypermutated set above. The pattern of usage of germline-origin motif sequences were found to follow a similar distribution pattern. Pareto distributions are found in a wide variety of physical and biological systems and tend to exhibit linear behavior over many orders of magnitude. As is common when dealing with systems displaying this distribution pattern a logarithmic (log base 2) frequency categorization scheme was developed. As the cellular interactions between T-cells and antigen presenting cells displaying the motifs in MHC molecules on their surfaces are the ultimate result of the molecular interactions, using a log base 2 system implies that each adjacent frequency class would double or halve the cellular interactions with that motif. Thus using such a frequency categorization scheme makes it possible to characterize subtle differences in motif usage as well as providing a comprehensible way of visualizing the cellular interaction dynamics with the different motifs. Overall, the variable regions were found to comprise approximately equal numbers of germline-origin and somatic hypermutated sequences.

When both the SHM and Germline motif sets were extracted, each of the TCEMs were assigned unique identifier keys which indicated their genetic origin and frequency classification. The identifier keys are essential for carrying out the set algebra manipulations to identify and characterize the TCEMs in different protein molecules.

Thus, relational set algebra manipulation of the combined SHM and germline TCEMs using the keys assigned in combination with a protein sequence of any origin can be used to characterize all of the motifs in a particular protein into three groups as being of SHM-origin, of germline-origin, or of neither. In addition, the frequency classification scheme provides insights into how T-cells will react to sequences in the proteins of interest.

The 40K database of immunoglobulin heavy chain variable region is taken to represent the "normal" situation, but in this database the depositors of the sequences did not identify (or may not have known) the isotype origin of the antibody molecule. However in some cases the depositors included additional metadata with the Genbank sequence accession. Thus, a further set of databases were created using metadata in the accession records of the Genbank sequences. In particular, two broad categories of sequences were identified: one set comprised antibody molecules that were isotype identified (IgG, IgE, IgM) and a second set comprised antibody molecules associated with several different immunopathologies. Immunopathologies such as rheumatoid arthritis, lupus erythematosus, leukemia, lymphoma, multiple sclerosis and the like are generally characterized by abnormal T-cell and B-cell interactions. The metadata was further curated to create unique, non-redundant sets of molecules. Subsets were created for IgG, IgE, IgM, lupus erythematosus (SLE), rheumatoid arthritis, chronic lymphocytic leukemia, lymphoma, and multiple sclerosis. Further curation was necessary because the original Genbank metadata sometimes associated more than one immunopathology with a particular antibody molecule. In total, these further curated subsets comprised approximately 8,000 molecules with individual subsets ranging from 200 molecules to 3000 IGHV sequences. These sequences were categorized for motifs as described above.

The human proteome database comprised approximately 81,000 proteins. This includes multiple isoforms of many proteins, hence the 81,000 exceeds the total proteome of any one human being by approximately 4 fold. The proteome consists of about twice as many proteins as the IGHV database, but their average size is considerably larger than that of the IGHV. When decomposed into their composite 15-mer peptides each indexed by one amino acid this resulted a total of 33 million peptides. When processed similarly to described above this produced about 2.42 million unique motifs, comprising about 7.5 million unique motif sequences in aggregate for the three motif configurations. As expected, IGHV-origin motifs were found in the proteome. The 275,000 motifs of IGHV-origin were found to match approximately 10% of the human proteome motifs. Thus, a database of IGHV sequences ten times the size of the current should provide complete coverage for all motifs in the human proteome. The total B-cell clones in a human exceed 10⁷ and thus the full B-cell population provides full coverage of the human proteome. In addition, it was found that about 15% of the IGHV-origin motifs had no matches in the human proteome thus giving credence to the concept that the IGHV provides a broad training of the immune system even for proteins foreign to the body.

### Database Assembly

The datasets resulting from the above processes were designed to be analyzed and manipulated within JMP^{®} (SAS Inc., Raleigh NC) using tools that combine relational set algebra with statistical analysis. In particular, the sets were created so that a set JOIN operation with any protein or group of proteins (for example a virus, a bacteria, a biotherapeutic) would enable the identification of matching motifs between the sets.

For Example:
JOIN-ing the motif sequences from influenza H1N1 with the 40K IGHV motif sequence dataset identified a set of motif sequences present in both the virus and the antibody molecules and which had a characteristic binding pattern with a clinically relevant MHC HLA.
A further JOIN with the human proteome identified a specific subset of proteins that also contained the same motif and further identifies motifs from other human proteins of non-immunoglobulin origin that likewise share motifs with the virus.

Although JMP^{®} is used in this Example, this is not considered limiting and alternative computer programs may be used to derive and process the datasets.

### Categorization based on GEM motifs

From the same database of downloaded sequences as in Example 1, we then identified the GEM motifs based on the following peptide positions and as shown in Figure 1:
GEM MHC-IIA as (-3,2,-1,1,4,6,9,+1,+2,+3) as 15-mer
GEM MHC-IIB ((-3,2,1,2,4,6,9,+1,+2,+3) as 15-mer
GEM MHC-I (1,2,3,9) as 9-mer

These motifs were categorized based on the frequency of their association with any given TCEM. MHC groove binding affinities were calculated as described in PCT US2011/029192,

The GEM motifs were then categorized into 3 groups based on the average binding affinities found in association with given TCEMS. This allows us to identify combinations of TCEM frequency and GEM affinity found in the IGHV databases.

When any other protein is examined, for example a pathogen protein, TCEMs may be identified which are also found in high or low frequency in IGHV, however in the new protein of interest these will be associated with a variety of different GEMs which provide for lower or higher binding affinity on an allele specific basis. The joint classification of TCEM frequency/GEM affinity allows a comparative index of the likelihood of interface with a T-cell that recognizes the same motif in the context of the same MHC allele

### Example 3: Characteristics of Motifs observed in IGHV

Each of the seven IGHV germline families designated IGHV1 - IGHV7 exhibit distinct patterns. Sequences from all seven families were processed and graphics generated. To simplify the description of the multi-dimensional patterns, we will refer primarily to sequences of IGHV3 germline origin subsets which is the largest family, derived from the larger datasets [3]. The pattern of MHC-II binding in approximately ten thousand IGHV3 molecules (isotype undefined) is shown in **Figure 2****.** The shaded areas in Figure 2 show the approximate the locations of the CDRs. There are several regions where peptides generated by SHM result in GEM with predicted high affinities for a range of MHC-II alleles, for example ranging widely from 0-16 different DRB alleles. In other regions the peptides have uniformly low predicted binding affinities. The predicted binding affinities of DP and DR alleles have similar patterns, but differ from the DQ alleles. In particular, the DQ alleles have a preference for peptides in Framework region 1. Interestingly, the patterns of predicted pMHC binding affinity before (in the germlines), and after SHM are similar. In the regions with predicted high affinity pMHC binding, it would be expected that the roles of the different pMHC types would be different: germline-origin motifs would maintain their TCEM and provide a suppressive or down regulatory function, whereas SHM-motifs would stimulate CD4+ helper activity. **Figure 3** shows the corresponding MHC -I binding affinity pattern.

The patterns of repeated motifs are shown in **Figure 4** **and** **5****.** A total of 61 germline motifs are found un-mutated in approximately 25% of the molecules (i.e., 10,000 of 40,000 IGHV). While the germline-origin TCEM motifs are conserved in mature antibodies, such antibodies typically have a wide range of pMHC affinities due to somatic hypermutation affecting the GEM amino acids. There are also several regions which show a high degree of repetition in the SHM TCEM. The two highest repeated zones are at centered at approximately at aa 90 and at about aa 105, which correspond to the N- and C-terminal sides of the CDR3. Taken together with the data in Figure 2, it is clear that regions of high motif repeats are also regions where predicted high affinity pMHC binding is found. Thus, there are repeated, high affinity pMHC with the same TCEM, but with different GEM.

### Frequency scoring system

Examination of the patterns in **Figure 5** indicate a gradation of TCEM repeats. We therefore devised a numerical system to classify the motifs based on their characteristic frequency of occurrence. We use log base 2 categories as a convenient classification which also provides a system in which the two-fold difference in motif frequencies in adjacent categories indicates a two-fold difference in the frequency of T-cell: B-cell encounters. With a total of about 40,000 total IGHV under consideration, a log base 2 system with a range of 16 categories provides an appropriate range for categorizing from high frequency motifs to those that occur as singletons. (1/2¹ - 1/2¹⁶ corresponds to the occurrence in 1/2 to 1/65,536 cells ). This scaling system provides a system for handling increasing size of database as well. As more sequences accumulate it is unlikely that frequency of motifs will change their categories significantly. Thus, the frequency categories are expected to be relatively constant and the scoring system can accommodate databases of various sizes. For example, doubling the repository from 40K to 80K is likely to add more singletons but a singleton 1/40,000 is most likely to only another identical motif (2/80,000 is the same frequency). Only 40,000 unique IGHV sequences could be assembled from Genbank but the frequency distributions show that at this size the cumulative distribution it approaching the upper asymptote; increasing the database size would not alter the fact that > 90% occur in FC1-15. This is confirmed by analysis the non-redundant immunoglobulin class-defined subsets.

The result was therefore a 16 category classification in which a class FC1 (1/2), FC3 (1/8) etc. represent common motifs and FC11 -FC16 are very rare motifs (1/65,536). It should be noted that certain synthetic antibodies contain TCEMs which occur in yet rarer frequencies (>FC16), as discussed below.

**Figure 6** shows the frequency distributions of MHC-II TCEMs found in IGHV germline sequences. and in IGHV sequences which have undergone somatic hypermutation. Figure 6 shows plots for TCEM IIa; very similar patterns were observed for TCEM II and TCEM I.

Germline sequences show a large number of commonly repeated TCEMs whereas mutated TCEMs have a spike in rare TCEMs. Approximately 11% of the TCEMs in mutated IGHV are found only once in the database; conversely 89% of the motifs are used repeatedly. As each variable region has about 100 motifs, each molecule will have roughly 10 unique motifs.

### Patterns of TCEM in specific subsets of IGHV

### Germline:

Each IGHV germline origin family has a unique set of motifs and thus exhibits its own characteristic motif repeat patterns. The patterns in **Figure 6C** show that the 80% of the germline sequences are found in the commonest frequency categories (FC) 1, 2 and 3. In contrast in **Figure 6F** the cumulative frequency distribution of IGHV which have undergone SHM is sigmoid on a log axis reflective of a log-normal distribution and shows that 50% of the cumulative motifs found in SHM regions occur by FC 10 and below with most occurring between FC 5 and FC 10 corresponding to 1/2⁵ - 1/2¹⁰ or about 1/32 to 1/1024 clonal-origin cells. The spike in category FC3 in the SHM group is the result of motifs in the sequences found bordering CDR3 as seen in **Figures 4** **and** **5****.** These results imply that multiple, high affinity, repeated TCEM are found in a relatively high proportion of the clonal lines.

**Table 2: Rare and common TCEM motifs from germline and mutated sequences. Each motif is characterized by a specific frequency in the database. An inverse log base 2 classification scheme was developed to provide a convenient number of classification groups. A total of 16 frequency classes are used for the database of 40,000 unique motifs. A motif found exactly 10,000 times would be given a frequency class of 2 (1/2² = 1/4). However, motifs are not found at precise frequencies and thus the frequency class designation is rounded to the next highest integer. For example, a motif found 10,500 times would also be assigned to frequency class 3, whereas a motif found 9,500 times would also be classified as frequency class 4.**

| **Germline Antibody Variable Regions** | | Frequency class |
|---|---|---|
| Very Common | >1 in 4 | FC 1 and FC2 |
| Common | >1 in 16 | FC3 and FC4 |

| **Mature Mutated Antibody variable regions** | | Frequency class |
|---|---|---|
| Very Common | >1 in 64 | FC1 toFC6 |
| Common | >1 in 512 | FC 7 to FC9 |
| Very rare | <1 in 65,536 | FC16 |
| Rare | <1 in 32,768 | FC15 |

When the cumulative frequencies for each of the motif patterns (TCEM IIa and TCEM IIb) are combined the summary of the frequencies is shown in Table 1; hence a very common germline motif is found in ¼ of all germline sequences and a very rare motif is found only in 1/40,000 variable region sequences.

### Immunoglobulin class

**Figure 7** shows the Frequency Class (FC) patterns for three different groups of isotype-defined or class defined IGHV3, IgG, IgM and IgE. The patterns show that for IgG and IgE there is a shift towards more low frequency (FC >10) motifs; this is expected as SHM progresses from an IgM starting germline. The spike in FC 16 corresponds to singletons with a cellular clonal frequencies in the range of 1/2¹⁵ - 1/2¹⁶ (approximately 1/50,000). There is a clear up-tic in the FC patterns for the singletons. For the large dataset used to derive the FC distribution patterns, singletons comprised 9.7% of the total motifs. The pattern for IgE, which like IgG has undergone a class switch, has a peak at FC 11 & 12 and then tails off dramatically to be less than IgM for FC 14-16. While different from IgG, it is unclear whether this is a characteristic of IgE generally or might be due to bias arising from the inclusion in the database of a large number of samples are from a study of asthmatic children [9]. In either case the differences in T-help activity associated with TCEM could be of relevance.

### Biotherapeutics and synthetics:

Examination of 163 commercial biotherapeutic antibodies cataloged in IMGT.org shows that while many motifs follow the same general distribution within frequency categories found in germline and the Genbank origin naturally derived mutated sequence database, these antibodies also have a large number of completely unique motifs unrecognized in naturally occurring antibodies.

**Figure 8 and Figure 9** shows the pattern of such rare TCEM in the 163 commercial biotherapeutic antibodies. These motifs are not found at all in the 40 thousand "normal" antibodies in our Genbank derived database used as the basis for motif frequency categorization; i.e., they do not fall within our categories of FC 1-16. Nor are the motifs found in either the isotype-defined groups downloaded from Genbank. The highlighted group are humanized molecules that have an average of 12 rare motifs. The remainder of the molecules are fully human (derived from phage display or humanized mice) or chimerics in which the IGHV would be entirely of murine origin. There is broad overlap in the occurrence of rare motifs between all of these sources.

### Example 4: Immunoglobulin Constant Regions

Examination of the peptide motifs in the constant regions of each isotype of immunoglobulin revealed that the TCEM motifs used in constant regions are distinct from those used in immunoglobulin variable regions. **Figure 10** shows the clear distinction between motif use in constant and variable regions.

Motifs used in the immunoglobulin constant region are excluded from use in normal immunoglobulin variable regions. This may reflect the effect of central or early tolerance to these motifs. Essentially the TCEM motifs found in the constant region correspond to the commonest frequency class as they are present in all immunoglobulins of that isotype. It follows that when they also comprise GEM motifs which give rise to high binding affinity they are motifs with a high probability of serving as T regulatory stimulators for individuals carrying the corresponding MHC allele.

The constant region motifs which have binding affinity in excess of 2 standard deviations below the mean were determined; these are very strong binders likely to have along dwell time in the MHC groove and consequently are most likely to have an immune suppressive effect.

Example 5: Immunoglobulin Light Chain Variable region and T-cell receptor databases Human immunoglobulin light chain variable regions (IGLV) were downloaded from Genbank using the argument "(immunoglobulin light chain variable region) AND (homo sapiens)".

As of June 2014 approximately 16,000 sequences were available comprising 4,022 kappa and 4,921 lambda and 7419 variants. Various restrictive combinations of search arguments were used to create non-redundant subsets of this larger set that were either immunoglobulin class-defined or for which the metadata attached to the accession indicated that they were associated with an immunopathology. Additionally, manual curation was used to remove sequences that were obviously not immunoglobulins or not light chains. IGLV were included in the final set if they contained at least 80 amino acids, a value approximating the shortest germline equivalent sequence. All sequences longer than 130 amino acids were truncated at that point. For Germline IGLV 108 sequences comprising 72 kappa germlines + 36 lambda germlines were obtained from the IMGT repository (www.imgt.org), and immunoglobulin light chain constant regions (IGLC) class reference sequences from Genbank.

TCEM and GEM motifs were extracted, scored, and categorized as described in Example 2 and 3. A total of 2106 Germline and 106,325 mutated unique TCEM IIA motifs, 2125 Germline and 106,845 mutated TCEM IIb motifs, and 2040 Germline and 104,879 mutated TCEM I motifs were found in IGLV. TCEM motifs extracted from IGLV were compared with those extracted previously from IGHV. Overall there is an overlap of approximately 60 IGLV unique : 45 IGHV+IGLV shared for all TCEM frames. Where a motif occurred in both datasets it was reclassified to the dataset in which it has the highest frequency of occurrence. Hence a motif previously identified as an IGHV FC8 which appears with a higher frequency in the IGLV dataset in 1 in 64 light chains was reclassified as a FC6 etc.

New algorithms were written to incorporate the IHLV dataset into the reference datasets and to reference motifs found in other proteins of interest according to whether they correspond to an IGHV or IGLV frequency category.

### T-cell receptor motifs

T-cell receptor germline and naturally occurring mutated sequences were downloaded from IMGT and from Genbank using (T-cell receptor) AND (Homo sapiens) as a search argument. Sequences that were found to be from unrelated sources or were derived from pathological conditions were removed. The resulting data set comprised 121 germline and 7338 human TCRs. Alpha, beta, gamma, and delta sequences were deduced by the Germline motif content, where these categories were not indicated in the Genbank annotations. The 121 germline sequences comprised 47 alpha, 60 beta, 11 gamma, 3 delta sequences.

Having curated the Genbank set as described for light chains, the remaining dataset comprised 7,338 molecules which in turn comprises 472,274 total TCEM motifs. The Genbank set includes some each of the alpha, beta, delta and gamma derivatives but also many unspecified sequences, in which the origin was deduced retrospectively by the motif distribution.

We evaluated the overlap of motif distribution with that in the IGHV and IGLV complex. Among the 472,274 total TCEM motifs in the TCR set, 5,009/6,863 (72.98%) germline and 33,300/48,090 (69.24%) mutated sequence origin motifs were unique to the TCR set.

Only 8,292 of 280,000 were shared with the IGHV motifs ( ~ 3%) and approximately the same with the light chain motifs. Overall 3,243 found in the TCR set were found in both light and heavy chain variable region motif sets, ~ 1%. These numbers are consistent with the level of sharing previously observed between proteome light chain and heavy chain.

Tables 3a-c show the degree of overlap among the TCR and immunoglobulin variable region motifs.

**Table 3a. Overlaps between TCR and between immunoglobulin motifs for Type 1 motifs**

| **motif group (4,5,6,7,8) TCR** | **motif group (4,5,6,7,8) LC** | **motif group (4,5,6,7,8) HC** | **N Rows** |
|---|---|---|---|
| Germline TCR | | | 4,627 |
| Germline TCR | | Germline_HC | 2 |
| Germline TCR | | Mutated_HC | 944 |
| Germline TCR | Germline_LC | | 16 |
| Germline TCR | Germline_LC | Mutated_HC | 60 |
| Germline TCR | Mutated_LC | | 533 |
| Germline TCR | Mutated_LC | Germline_HC | 23 |
| Germline TCR | Mutated_LC | Mutated_HC | 524 |
| *Total* | | | 6,729 |
| Mutated TCR | | | 29,177 |
| Mutated TCR | | Germline_HC | 15 |
| Mutated TCR | | Mutated_HC | 8,325 |
| Mutated TCR | Germline_LC | | 29 |
| Mutated TCR | Germline_LC | Germline_HC | 7 |
| Mutated TCR | Germline_LC | Mutated_HC | 195 |
| Mutated TCR | Mutated_LC | | 2,281 |
| Mutated TCR | Mutated_LC | Germline_HC | 233 |
| Mutated TCR | Mutated_LC | Mutated_HC | 3,305 |
| *Total* | | | 43,567 |

**Table 3b. Overlaps between TCR and between immunoglobulin motifs for Type IIA motifs**

| **motif group (2,3,5,7,8) TCR** | **motif group (2,3,5,7,8) LC** | **motif group (2,3,5,7,8) HC** | **N Rows** |
|---|---|---|---|
| | | Germline_HC | 333 |
| | | Mutated_HC | 219,131 |
| | Germline_LC | | 727 |
| | Germline_LC | Germline_HC | 8 |
| | Germline_LC | Mutated_HC | 1,107 |
| | Mutated_LC | | 55,651 |
| | Mutated_LC | Germline_HC | 1,500 |
| | Mutated_LC | Mutated_HC | 42,031 |
| | | | |
| Germline TCR | | | 4,846 |
| Germline TCR | | Mutated_HC | 864 |
| Germline TCR | Germline_LC | | 11 |
| Germline TCR | Germline_LC | Germline_HC | 4 |
| Germline TCR | Germline_LC | Mutated_HC | 39 |
| Germline TCR | Mutated_LC | | 551 |
| Germline TCR | Mutated_LC | Germline_HC | 25 |
| Germline TCR | Mutated_LC | Mutated_HC | 482 |
| ***Total*** | | | 6,822 |
| Mutated TCR | | | 32,164 |
| Mutated TCR | | Germline_HC | 16 |
| Mutated TCR | | Mutated_HC | 7,880 |
| Mutated TCR | Germline_LC | | 29 |
| Mutated TCR | Germline_LC | Germline_HC | 5 |
| Mutated TCR | Germline_LC | Mutated_HC | 176 |
| Mutated TCR | Mutated_LC | | 2,622 |
| Mutated TCR | Mutated_LC | Germline | 238 |
| Mutated TCR | Mutated_LC | Mutated | 3,225 |
| ***Total*** | | | 46,355 |

**Table 3c. Overlaps between TCR and between immunoglobulin motifs for Type IIB motifs**

| **motif group (-1,3,5,7,8) TCR** | **motif group (-1,3,5,7,8) LC** | **motif group (-1,3,5,7,8) HC** | **N Rows** |
|---|---|---|---|
| | | Germline_HC | 338 |
| | | Mutated_HC | 218,888 |
| | Germline_LC | | 757 |
| | Germline_LC | Germline_HC | 18 |
| | Germline_LC | Mutated_HC | 1,089 |
| | Mutated_LC | | 56,418 |
| | Mutated_LC | Germline_HC | 1,508 |
| | Mutated_LC | Mutated_HC | 41,647 |
| | | | |
| **Germline TCR** | | | 5,009 |
| **Germline TCR** | | Germline_HC | 4 |
| **Germline TCR** | | Mutated_HC | 796 |
| **Germline TCR** | Germline_LC | | 9 |
| **Germline TCR** | Germline_LC | Germline_HC | 4 |
| **Germline TCR** | Germline_LC | Mutated_HC | 40 |
| **Germline TCR** | Mutated_LC | | 522 |
| **Germline TCR** | Mutated_LC | Germline_HC | 24 |
| **Germline TCR** | Mutated_LC | Mutated_HC | 455 |
| ***Total*** | | | 6,863 |
| **Mutated TCR** | | | 33,300 |
| **Mutated TCR** | | Germline_HC | 19 |
| **Mutated TCR** | | Mutated_HC | 8,292 |
| **Mutated TCR** | Germline_LC | | 44 |
| **Mutated TCR** | Germline_LC | Germline_HC | 10 |
| **Mutated TCR** | Germline_LC | Mutated_HC | 154 |
| **Mutated TCR** | Mutated_LC | | 2,817 |
| **Mutated TCR** | Mutated_LC | Germline_HC | 220 |
| **Mutated TCR** | Mutated_LC | Mutated_HC | 3,234 |
| ***Total*** | | | 48,090 |

### Example 6 Microbial database assembly

To establish a reference database enabling the determination of frequency of use of T cell exposed motifs in microorganisms two groups of bacteria were utilized. This database is an example of the approach in determining T cell exposed motif use in microbes and is not considered limiting with respect to the bacteria included nor to the class of microorganisms. The first group of bacteria comprises 38 species in 67 genera from the gastrointestinal microbiome. The second group comprises up to five proteomes each of 28 species in 12 genera of pathogenic bacteria. We characterized the patterns of TCEM usage within each proteome dataset, and compared them to each other, and to the repertoire of motifs in immunoglobulin variable regions.
a. Gastrointestinal microbiome bacteria (Microbiome) The proteomes were assembled from the NIH Human Microbiome Project Reference Genomes database (www.hmpdacc.org/HMRGD) and included 67 species in 35 genera (listed in Table 4). This set comprised, a total of 378,061 proteins with an average length of 290 amino acids. This is not a set of fully annotated proteomes and may include some partial proteomes. The final set comprised 1.09x10⁸ total TCEM in each of the three reading frames and is thus of a comparable total size to the bacterial pathogen set.
b. Exemplary bacterial pathogens (Pathogens) comprising 28 species each represented by up to 5 proteomes (132 in all) selected at random from the complete proteomes available at PATRIC (www.patricbrc.org)[21]. The genera included were: *Bordetella, Brucella, Burkholderia, Chlamydia, Clostridium, Coxiella, Francisella, Mycobacterium, Neisseria, Staphylococcus, Streptococcus,* and *Ureaplasma.* Species and strains are shown in Table 4. Proteomes ranged in size from approximately 6000 proteins (*Burkholderia*) to 689 (*Ureaplasma*). Overall 427,906 proteins were included in the analysis. The final pathogen dataset comprised 1.16 x 10⁸ TCEM in each of the three TCEM reading frames.

To construct the TCEM sets, concatenated amino acid FASTA files of each of the proteomes were assembled, wherein each protein in the proteome was then decomposed into sets of 9-mer and 15-mer peptides, each offset by a single amino acid and placed in successive rows of a JMP^{®} data table. Three different TCEM reading frames were constructed from each peptide in corresponding columns using column formulas of JMP^{®}. These were TCEM I comprising amino acids (4,5,6,7,8) of a core MHC-I 9-mer, and TCEM IIa and TCEM IIb comprising amino acids corresponding to amino acids (2,3,5,7,8) and (-1,3,5,7,8) of the central 9-mer core of a 15-mer [2,22]. The groove exposed motifs (GEM) responsible for the binding interactions were assembled as the intercalated set of non-TCEM amino acids in each peptide. The unique identifier of each protein, the peptide, and the N terminal positions of each peptide in the parent protein within the proteome were retained with each TCEM. In this way TCEM sets were hyperlinked to the parental proteins by JMP^{®} so that the parental proteins could be readily retrieved for other analyses. Three sets of tables, one for each TCEM reading frame, were constructed for each protein set.

**Table 4 - Pathogen and Gastrointestinal Microbiome proteomes included in analysis Compete proteomes of the following organisms were downloaded from PATRIC. Isolate identifications shown are as listed in PATRIC. Gastrointestinal microbiome bacterial proteomes were assembled from the NIH Human Microbiome Project Reference Genomes database (www.hmpdacc.org/HMRGD).**

| **Pathogens** | **GI microbiome** |
|---|---|
| ***Bordetella bronchiseptica 2371640*** | *Anaerobaculum hydrogeniformans ATCC BAA-1850* |
| ***Bordetella bronchiseptica 253*** | *Anaerostipes caccae DSM 14662* |
| ***Bordetella bronchiseptica D445*** | *Anaerostipes sp. 3 2 56FAA* |
| ***Bordetella bronchiseptica RB50*** | *Bacteroides cellulosilyticus DSM 14838* |
| ***Bordetella bronchiseptica SEAT0006*** | *Bacteroides clarus YIT 12056* |
| ***Bordetella pertussis B1920*** | *Bacteroides eggerthii DSM 20697* |
| ***Bordetella pertussis Bp H897*** | *Bacteroides sp. 3 119* |
| ***Bordetella pertussis Bp SEAT 0004*** | *Bacteroides sp. D22* |
| ***Bordetella pertussis CHLA15*** | *Bacteroides xylanisolvens SD CC 1b* |
| ***Bordetella pertussis Tohama I*** | *Bacteroides xylanisolvens SD CC 2a* |
| ***Brucella melitensis 043*** | *Bifidobacterium breve DSM 20213* = *JCM 1192* |
| ***Brucella melitensis ATCC 23457*** | *Bifidobacterium sp. 12 1 47BFAA* |
| ***Brucella melitensis B115*** | *Citrobacter youngae ATCC 29220* |
| ***Brucella melitensis M5*** | *Clostridiales butyrate-prod* |
| ***Brucella melitensis S66*** | *Clostridiales butyrate-producing bacter* |
| ***Burkholderia cenocepacia AU 1054*** | *Clostridiales butyrate-producing bacterium SS* |
| ***Burkholderia cenocepacia H111*** | *Clostridiales butyrate-producing bacterium SSC*/*2* |
| ***Burkholderia cenocepacia KC-01*** | *Clostridium sp. M62*/*1* |
| ***Burkholderia cepacia ATCC 25416*** | *Clostridium sp. SS2*/*1* |
| ***Burkholderia cepacia Bu72*** | *Coprobacillus sp. 8 2 54BFAA* |
| ***Burkholderia mallei ATCC 10399*** | *Coprococcus sp. HPP0074* |
| ***Burkholderia mallei ATCC 23344*** | *Corynebacterium sp. HFH0082* |
| ***Burkholderia mallei FMH*** | *Edwardsiella tarda ATCC 23685* |
| ***Burkholderia mallei JHU*** | *Enterobacter cancerogenus ATCC 35316* |
| ***Burkholderia mallei NCTC 10247*** | *Enterococcus faecalis TX2134* |
| ***Burkholderia pseudomallei 1026b*** | *Erysipelotrichaceae bacterium 21 3* |
| ***Burkholderia pseudomallei 1106a*** | *Escherichia coli 4 1 47FAA* |
| ***Burkholderia pseudomallei 1106b*** | *Escherichia coli MS 60-1* |
| ***Burkholderia pseudomallei K96243*** | *Escherichia coli MS 69-1* |
| ***Burkholderia pseudomallei MSHR305*** | *Escherichia coli MS 78-1* |
| ***Chlamydia trachomatis A363*** | *Escherichia coli MS 84-1* |
| ***Chlamydia trachomatis Ds2923*** | *Eubacterium rectale M104*/*1* |
| ***Chlamydia trachomatis FSWS*** | *Faecalibacterium prausnitzii M21*/*2* |
| ***Chlamydia trachomatis Jali20*** | *Fusobacterium mortiferumATCC 9817* |
| ***Chlamydia trachomatis Sweden2*** | *Fusobacterium varium ATCC 27725* |
| ***Clostridium difficile ATCC 43255*** | *Hafnia alvei ATCC 51873* |
| ***Clostridium difficile ATCC 9689*** | *Helicobacter pylori GAM101Biv* |
| ***Clostridium difficile CD165*** | *Helicobacter pylori GAM246Ai* |
| ***Clostridium difficile DA00212*** | *Helicobacter pylori GAM252T* |
| ***Clostridium difficile Y270*** | *Helicobacter pylori GAM83Bi* |
| ***Clostridium perfringens ATCC 13124*** | *Helicobacter pylori GAM93Bi* |
| ***Clostridium perfringens CPE str F4969*** | *Helicobacter pylori GAM96Ai* |
| ***Clostridium perfringens E str JGS1987*** | *Helicobacter pylori HP116Bi* |
| ***Clostridium perfringens JJC*** | *Helicobacter pylori HP250BFiii* |
| ***Clostridium perfringens str 13*** | *Helicobacter pylori HP250BSi* |
| ***Coxiella burnetii Cb185*** | *Klebsiella pneumoniae subsp. pneumoniae WGLW3* |
| ***Coxiella burnetii Dugway 51108-111*** | *Lachnospiraceae bacterium 2 1 58FAA* |
| ***Coxiella burnetii RSA 331*** | *Lachnospiraceae bacterium 3 1 57FAA CT1* |
| ***Coxiella burnetii RSA 493*** | *Lachnospiraceae bacterium 5 1 57FAA* |
| ***Coxiella burnetii Z3055*** | *Lachnospiraceae bacterium 5 1 63FAA* |
| ***Francisella novicida FTE*** | *Lachnospiraceae bacterium 7 1 58FAA* |
| ***Francisella novicida FTG*** | *Lactobacillus reuteri MM4-1A* |
| ***Francisella novicida GA99-3548*** | *Lactobacillus reuteri SD2112* |
| ***Francisella novicida GA99-3549*** | *Listeria innocua ATCC 33091* |
| ***Francisella novicida U112*** | *Megamonas hypermegale ART12*/*1* |
| ***Francisella tularensis subsp holarctica*** | *Methanobrevibacter smithii DSM 2375* |
| ***Francisella tularensis subsp holarctica 257*** | *Paraprevotella xylaniphila YIT 11841* |
| ***Francisella tularensis subsp holarctica F92*** | *Phascolarctobacterium succinatutens YIT 12067* |
| ***Francisella tularensis subsp holarctica FSC022*** | *Prevotella oralis HGA0225* |
| ***Francisella tularensis subsp holarctica FSC200*** | *Roseburia intestinalis M50*/*1* |
| ***Francisella tularensis subsp tularensis FSC033*** | *Roseburia intestinalis XB6B4* |
| ***Francisella tularensis subsp tularensis FSC198*** | *Ruminococcus obeum A2-162* |
| ***Francisella tularensis subsp tularensis MAOO-2987*** | *Ruminococcus torques L2-14* |
| ***Francisella tularensis subsp tularensis NE061598*** | *Succinatimonas hippei YIT 12066* |
| ***Francisella tularensis subsp tularensis SCHU S4*** | *Sutterella wadsworthensis 2 1 59BFAA* |
| ***Mycobacterium abscessus 45-0303*** | *Veillonella sp. 6 127* |
| ***Mycobacterium abscessus 55-1212*** | *Weissella paramesenteroides ATCC 33313* |
| ***Mycobacterium abscessus 6G-0212*** | |
| ***Mycobacterium abscessus M156*** | |
| ***Mycobacterium abscessus V06705*** | |
| ***Mycobacterium bovis BCG str ATCC 35733*** | |
| ***Mycobacterium bovis BCG str ATCC 35740*** | |
| ***Mycobacterium bovis BCG str ATCC 35743*** | |
| ***Mycobacterium bovis BCG str Glaxo*** | |
| ***Mycobacterium bovis BCG str Pasteur 1173P2*** | |
| ***Mycobacterium leprae Br4923*** | |
| ***Mycobacterium leprae TN*** | |
| ***Mycobacterium tuberculosis H37Ra*** | |
| ***Mycobacterium tuberculosis H37RvAE*** | |
| ***Mycobacterium tuberculosis H37RvCO*** | |
| ***Mycobacterium tuberculosis H37RvHA*** | |
| ***Mycobacterium tuberculosis H37RvJO*** | |
| ***Neisseria gonorrhoeae 1291*** | |
| ***Neisseria gonorrhoeae DGI2*** | |
| ***Neisseria gonorrhoeae FA 1090*** | |
| ***Neisseria gonorrhoeae PID18*** | |
| ***Neisseria gonorrhoeae SK-93-1035*** | |
| ***Neisseria meningitidis 2003022*** | |
| ***Neisseria meningitidis 2004032*** | |
| ***Neisseria meningitidis 64182*** | |
| ***Neisseria meningitidis 93003*** | |
| ***Neisseria meningitidis NM3222*** | |
| ***Staphylococcus aureus subsp aureus ATCC 51811*** | |
| ***Staphylococcus aureus subsp aureus ATCC BAA-*39** | |
| ***Staphylococcus aureus subsp aureus COL*** | |
| ***Staphylococcus aureus subsp aureus MRSA131*** | |
| ***Staphylococcus aureus subsp aureus USA300 TCH1516*** | |
| ***Staphylococcus epidermidis NIH051668*** | |
| ***Staphylococcus epidermidis VCU120*** | |
| ***Staphylococcus epidermidis VCU139*** | |
| ***Staphylococcus epidermidis W23144*** | |
| ***Staphylococcus epidermidis WI05*** | |
| ***Streptococcus agalactiae A909*** | |
| ***Streptococcus agalactiae CCUG 24810*** | |
| ***Streptococcus agalactiae GB00247*** | |
| ***Streptococcus agalactiae GB00951*** | |
| ***Streptococcus agalactiae ILRI005*** | |
| ***Streptococcus dysgalactiae subsp dysgalactiae ATCC 27957*** | |
| ***Streptococcus dysgalactiae subsp equisimilis 167*** | |
| ***Streptococcus dysgalactiae subsp equisimilis ATCC 12394*** | |
| ***Streptococcus dysgalactiae subsp equisimilis GGS 124*** | |
| ***Streptococcus dysgalactiae subsp equisimilis RE378*** | |
| ***Streptococcus mutans TCI-110*** | |
| ***Streptococcus mutans TCI-149*** | |
| ***Streptococcus mutans TCI-223*** | |
| ***Streptococcus mutans TCI-400*** | |
| ***Streptococcus mutans TCI-92*** | |
| ***Streptococcus pneumoniae ATCC 700669*** | |
| ***Streptococcus pneumoniae Hungary19A-6*** | |
| ***Streptococcus pneumoniae SP195*** | |
| ***Streptococcus pneumoniae Taiwan19F-14*** | |
| ***Streptococcus pneumoniae TCH843119A*** | |
| ***Streptococcus pyogenes GA03805*** | |
| ***Streptococcus pyogenes GA16797*** | |
| ***Streptococcus pyogenes GA19681*** | |
| ***Streptococcus pyogenes MGAS2096*** | |
| ***Streptococcus pyogenes UTMEM-1*** | |
| ***Ureaplasma urealyticum 2608*** | |
| ***Ureaplasma urealyticum serovar 11 str ATCC 33695*** | |
| ***Ureaplasma urealyticum serovar 2 str ATCC 27814*** | |
| ***Ureaplasma urealyticum serovar 7 str ATCC 27819*** | |
| ***Ureaplasma urealyticum serovar 9 str ATCC 33175*** | |

### Example 7: Analysis of specific epitopes

### Influenza A 2009 H1N1 California

Type 1 narcolepsy has been described as an autoimmune disorder found almost exclusively in individuals carrying a DQA1*01:02/DQB1*06:02 allele [23,24]. In 2010 following the spread of H1N1 influenza a cluster of diagnoses narcolepsy cases following influenza infection was described [25] . Simultaneously cases of narcolepsy in Europe were linked to vaccination with the Pandermix^{®} influenza vaccine containing the California 2009 H1N1 hemagglutinin protein (Influenza A virus (A/California/07/2009(H1N1) gi 392357062) [26]. A recent publication identified a possible key CD4+ epitope at amino acid positions 275-287 in the California 2009 hemagglutinin . We examined the binding affinity of CD4+ alleles across the sequence of interest and identified DQA1*01:02/DQB1*06:02 allele showing a predicted binding affinity of -2.29 standard deviations below the mean for the 15 mer index position 273 an extraordinarily high binding affinity and -1.86 and -1.33 in positions 274 and 275 respectively, **(****Figure 11** **and** **12****)** . DQA1*01:02/DQB1*06:02 is the only MHC-II allele which has a binding affinity significantly below the mean, hence the only allele of 28 tested that is likely to bind tightly in this region. Furthermore when we examined the TCEM in this region we find several motifs with a high frequency in IGHV. These include the following shown in Table 5:

| peptide | 15mer index position | TCEMIIa | TCEMIIb | Mutated FC class |
|---|---|---|---|---|
| FAMERNAGSGIIISD (SEQ ID NO: 1) | 272 | RN~G~GI (SEQ ID NO: 2) | M~~N~G~GI (SEQ ID NO: 3) | 13 |
| AMERNAGSGIIISDT (SEQ ID NO: 4) | 273 | **NA~S~II** (SEQ ID NO: 5) | E~~A~S~II (SEQ ID NO: 6) | 16 |
| MERNAGSGIIISDTP (SEQ ID NO: 7) | 274 | **AG~G~II** (SEQ ID NO: 8) | R~~G~G~II (SEQ ID NO: 9) | 16 |
| ERNAGSGIIISDTPV (SEQ ID NO: 10) | 275 | GS~I~IS (SEQ ID NO: 11) | N~~S~I~IS (SEQ ID NO: 12) | 16 |
| RNAGSGIIISDTPVH (SEQ ID NO: 13) | 276 | SG~I~SD (SEQ ID NO: 14) | A~~G~I~SD(SEQ ID NO: 15) | 9 |

Examination of the occurrence of these by allele shows that only the two boldfaced in the above table are present for DQA1*01:02/DQB1*06:02, the others are found associated with other alleles. DQA1*01:02/DQB1*06:02 is the only MHC-II allele which has a binding affinity significantly below the mean, hence the only allele of 28 tested that is likely to bind tightly in this region. The exact peptide sequences binding to DQA1*01:02/DQB1*06:02 with high affinity are absent from the hemagglutinin protein of H1N1 influenza virus isolates made earlier in 2009 or in 2010 and is the product of a specific amino acid mutation.

Narcolepsy arises when cells in the hypothalamus with receptors for orexin (hypocretin) are destroyed or cease to function. No direct causal mechanism linking the influenza motif to narcolepsy has been established, although a partial motif match to orexin has been suspected; it is not clear that this provides a causal relationship as it would not explain the loss of hypothalamic cells. The identified motifs can be searched to find their occurrence in self-proteins to determine if any are potentially causally associated with narcolepsy. Example 5 shows how such a search was conducted for one of the motifs. We continue to examine for sources of possible mimics in the hypocretin pathway. Alternative explanations have recently been proposed [7].

### Influenza hemagglutinin "universal" B-cell epitope:

The presence of a highly conserved B-cell epitope in the hemagglutinin of influenza H1, H2 and H5 at position 375-380 is well documented [27,28]. The exact position in this range varies a little depending on the hemagglutinin and isolate. Our own observations have shown that this B-cell epitope, while highly conserved, is in a region devoid of high affinity MHC-II or MHC-I binding and thus unlikely to benefit from T-cell help essential to memory. Only one allele, DQA1*05:01-DQB1*03:01 shows any likelihood of binding to a series of peptides in this stretch of the protein.

Interestingly, the TCEM centered at amino acid position 378 in H1N1 California 2009 (gi 392357062) is found to be a germline motif GS~Y~AD (SEQ ID NO:469) which occurs in germline FC4. In other words it is present in 1 in 16 germline IGHV. Among all microbial proteins we have examined this is an exceptionally high level of germline TCEM frequency. The implication of this is that not only does the TCEM associated with the highly conserved motif have a GEM of low binding affinity but the TCEM would be highly likely to be an immunosuppressive motif. This may well be a contributing factor in the extreme conservation of the B-cell epitope at this point.

### Example 8 Evaluation of potential immunosuppressive motifs in influenza hemagglutinin

The surface proteins of influenza and in particular the hemagglutinin are the target of neutralizing and protective antibodies and as such are the focus of vaccine design. Influenza viruses are highly variable both between different classes of hemagglutinin (H1, H3, H5, H7 etc.) and within any one hemagglutinin class; they undergo constant antigenic drift. Thus there is considerable focus on designing vaccines which can provide high antibody titers responsive to each newly emerging strain of the virus. By applying the classification of TCEM motifs to a multiplicity of the sequences of influenza HA isolated over the years we determined that each HA (from any different isolate) may carry from about 3 up to about 20 TCEM motifs which are found with high frequency in immunoglobulins (FC10 or lower as previously defined in this invention) and are thus likely to elicit an immunosuppressive T cell response (Treg) whenever they occur in peptides which are also bound by MHC molecules. It would be desirable to eliminate these potentially suppressive motifs to favor a better antibody response. The positions at which such high frequency motifs occur varies but shown some consistency within HA class (H7 vs H1 etc.). We describe here the analysis of HA1 sequences from 447 H3N2 isolates made between 1968 and 2003 and demonstrate the distribution of potentially immunosuppressive TCEM motifs. We then propose substitutions of 1-3 amino acids which can change the frequency category of a TCEM motif from likely immunosuppressive (<FC10) to likely immunostimulatory (FC14-16 or >16). While this example addresses a panel of H3 isolates, the same approach is equally applicable to isolates of other HA classes and thus the example is not considered limiting. The following steps were employed:
a. Array sequences of HA by position and FC motif and class, for each of TCEM IIA, IIB and TCEM I
b. Determine positions which have motifs of TCEM <FC10.
c. Evaluate peptides of other isolates in the panel of >400 hemagglutinins at the same or adjacent amino acid position to identify motifs functional in other isolates that have a higher frequency category.
d. Identify the substitution which will achieve a change of FC category to >FC14 and preferably to FC 16 or >16.
e. Evaluate where the motifs are found within the human proteome to determine if any potential adverse targeting may occur through the creation of a substitute motif.

Overall 50 TCEM IIA, 48 TCEM IIB and 51 TCEM I motifs of FC ≤ 10 were found the panel of H3N2 isolates. Some such motifs occurred in >400 isolates, some occurred in as few as one isolate. Substitution motifs were identified for each. In the interests of space we provide an illustrative subset of the results for all motif positions of all the viruses.

Any single virus isolate or vaccine candidate can be evaluated by the same means; evaluation of the larger database allows substitution of motifs known to be functional in comparable isolates of influenza. Within any given isolate the modifications of TCEM IIA, IIB and TCEM I have to be reconciled internally to make sure that improvements to one motif do not adversely affect an alternate TCEM register.

**Table 6. TCEM IIA**

| pos | TCR II (2,3,5,7,8) | FC TCR II (2,3,5,7,8) | Occurrences in 447 isolates N Rows | FC TCR II (2,3,5,7,8) | TCR II (2,3,5,7,8) |
|---|---|---|---|---|---|
| **Wild type** | | | **Substitution** | | |
| 32 | VP~G~LV (SEQ ID NO: 16) | 10 | 435 | VP~G~IV (SEQ ID NO: 17) | 16 |
| 33 | VP~G~LV (SEQ ID NO: 16) | 10 | 2 | VP~G~IV (SEQ ID NO: 17) | 16 |
| 37 | VP~G~LV (SEQ ID NO: 16) | 10 | 1 | VP~G~IV (SEQ ID NO: 17) | 16 |
| 45 | VP~G~LV (SEQ ID NO: 16) | 10 | 1 | VP~G~IV (SEQ ID NO: 17) | 16 |
| 60 | TG~I~GS (SEQ ID NO: 18) | 10 | 1 | TG~I~DS (SEQ ID NO: 19) | >16 |
| 119 | SL~S~VA (SEQ ID NO: 20) | 10 | 439 | SL~S~IA (SEQ ID NO: 21) | 13 |

**Table 7. TCEM IIB**

| pos | TCR II (-1,3,5,7,8) | FC TCR II (-1,3,5,7,8) | Occurrences in 447 isolates N Rows | Substitution | |
|---|---|---|---|---|---|
| 37 | G~~V~T~TN (SEQ ID NO: 22) | 0 | 366 | G~~V~T~TD (SEQ ID NO: 23) | 14 |
| 38 | G~~V~T~TN (SEQ ID NO:22 ) | 0 | 2 | G~~V~T~TD (SEQ ID NO: 23) | 14 |
| 42 | G~~V~T~TN (SEQ ID NO:22 ) | 0 | 1 | G~~V~T~TD (SEQ ID NO: 23) | 14 |
| 50 | G~~V~T~TN (SEQ ID NO:22 ) | 0 | 1 | G~~V~T~TD (SEQ ID NO: 23) | 14 |
| 54 | T~~V~S~ST (SEQ ID NO: | 7 | 421 | T~~V~S~TT (SEQ ID NO: | 16 |
| | 24) | | | 25) | |
| 55 | T~~V~S~ST (SEQ ID NO:24) | 7 | 2 | T~~V~S~TT (SEQ ID NO: 25) | 16 |

**Table 8. TCEM I**

| pos | TCR I (4,5,6,7,8) | FC TCR I (4,5,6,7,8) | Occurrences in 447 isolates N Rows | Substitution | |
|---|---|---|---|---|---|
| 4 | ~~~LSYIS~ (SEQ ID NO: 26) | 9 | 1 | ~~~LSYIF~ (SEQ ID NO: 27) | 14 |
| 22 | ~~~STATL~ (SEQ ID NO: 28) | 10 | 441 | ~~~RTATL~ (SEQ ID NO: 29) | 13 |
| 23 | ~~~STATL~ (SEQ ID NO: 28) | 10 | 2 | ~~~RTATL~ (SEQ ID NO:29 ) | 13 |
| 35 | ~~~STATL~ (SEQ ID NO: 28) | 10 | 1 | ~~~RTATL~ (SEQ ID NO: 29) | 13 |
| 58 | ~~~GSSTG~ (SEQ ID NO: 30) | 8 | 1 | ~~~SSSTG~ (SEQ ID NO: 31) | 12 |

When the proposed substitute motifs were compared to motifs occurring in the proteome the number of matches, again for the subset shown above, are those shown in Table 9.

**Table 9.**

| TCR II (2,3,5,7,8) | Occurrences in proteome N Rows | TCR II (-1,3,5,7,8) | Occurrences in proteome N Rows | TCR I (4,5,6,7,8) | Occurrences in proteome N Rows |
|---|---|---|---|---|---|
| SL~S~IA (SEQ ID NO: 32) | 80 | G~~V~T~TD (SEQ ID NO: 23) | 17 | ~~~LSYIF~ (SEQ ID NO: 27) | 14 |
| TG~I~DS (SEQ ID NO:33 ) | 23 | T~~V~S~TT (SEQ ID NO:24) | 61 | ~~~RSTAY~ (SEQ ID NO: 34) | 8 |
| VP~G~IV (SEQ ID NO: 17) | 20 | | | ~~~RTATL~ (SEQ ID NO: 29) | 28 |
| | | | | ~~~SSSTG~ (SEQ ID NO: 31) | 166 |

The identity of each protein with a match was determined and reviewed. The corresponding subset of this dataset for TCEM IIA only is shown in Table 9.

**Table 10.**

| TCR II (2,3,5,7,8) | protein_id | pos | peptide |
|---|---|---|---|
| SL~S~IA (SEQ ID NO: 32) | CTGEF_HUMAN cTAGE family member 15 OS=Homo sapiens | 179 | DESKSLKSQIAEAKI (SEQ ID NO: 35) |
| SL~S~IA (SEQ ID NO: 32) | CTGE9_HUMAN cTAGE family member 9 OS=Homo sapiens | 179 | DESKSLKSQIAEAKI (SEQ ID NO:35 ) |
| SL~S~IA (SEQ ID NO: 32) | A8MXH5_HUMAN Collagen alpha-6(IV) chain OS=Homo sa | 1245 | APGISLPSLIAGQPG (SEQ ID NO: 36) |
| SL~S~IA (SEQ ID NO: 32) | B4DDH2_HUMAN Serine/threonine-protein kinase 33 OS | 176 | WIIQSLASAIAYLHN (SEQ ID NO: 37) |
| SL~S~IA (SEQ ID NO: 32) | C9J319_HUMAN Serine/threonine-protein kinase 33 (F | 176 | WIIQSLASAIAYLHN (SEQ ID NO:37) |
| SL~S~IA (SEQ ID NO: 32) | C9J5X9_HUMAN Fanconi anemia group B protein (Fragm | 768 | VTLSSLSSAIAKHES (SEQ ID NO: 38) |
| SL~S~IA (SEQ ID NO: 32) | C9J6X7_HUMAN Serine/threonine-protein kinase 33 (F | 176 | WIIQSLASAIAYLHN (SEQ ID NO:37) |
| SL~S~IA (SEQ ID NO: 32) | D6RBI9_HUMAN Epsin-3 (Fragment) OS=Homo sapiens GN | 34 | GPPSSLMSEIADLTF (SEQ ID NO: 39) |
| SL~S~IA (SEQ ID NO: 32) | D6RBR6_HUMAN Epsin-3 (Fragment) OS=Homo sapiens GN | 34 | GPPSSLMSEIADLTF (SEQ ID NO:39) |
| SL~S~IA (SEQ ID NO: 32) | D6REL6_HUMAN Epsin-3 (Fragment) OS=Homo sapiens GN | 34 | GPPSSLMSEIADLTF (SEQ ID NO: 39) |
| SL~S~IA (SEQ ID NO: 32) | D6RFG3_HUMAN Epsin-3 (Fragment) OS=Homo sapiens GN | 34 | GPPSSLMSEIADLTF (SEQ ID NO: 39) |
| SL~S~IA (SEQ ID NO: 32) | E7EU13_HUMAN Arf-GAP with Rho-GAP domain | 55 | SLSLSLPSTIAAPHP (SEQ ID NO: 40) |
| SL~S~IA (SEQ ID NO: 32) | F5GZX4_HUMAN DNA-directed RNA polymerase OS=Homo s | 699 | LFPHSLLSVIANFIP (SEQ ID NO: 41) |
| SL~S~IA (SEQ ID NO: 32) | F5H3Q5_HUMAN Collagen alpha-6(IV) chain OS=Homo sa | 1220 | APGISLPSLIAGQPG (SEQ ID NO:42 ) |
| SL~S~IA (SEQ ID NO: 32) | F5H643_HUMAN DNA-directed RNA polymerase OS=Homo s | 450 | LFPHSLLSVIANFIP (SEQ ID NO: 43) |
| SL~S~IA (SEQ ID NO: 32) | F5H851_HUMAN Collagen alpha-6(IV) chain OS=Homo sa | 1220 | APGISLPSLIAGQPG (SEQ ID NO: 42) |
| SL~S~IA (SEQ ID NO: 32) | F8W898_HUMAN DNA-directed RNA polymerase OS=Homo s | 478 | LFPHSLLSVIANFIP (SEQ ID NO: 43) |
| SL~S~IA (SEQ ID NO: 32) | F8WAK5_HUMAN Serine/threonine-protein kinase 33 OS | 30 | WIIQSLASAIAYLHN (SEQ ID NO:44 ) |
| SL~S~IA (SEQ ID NO: 32) | HOY2S9_HUMAN Myosin phosphatase Rho-interacting pr | 939 | ALEASLISQIADSLK (SEQ ID NO: 45) |
| SL~S~IA (SEQ ID NO: 32) | H0Y7E2_HUMAN Myosin phosphatase Rho-interacting pr | 249 | ALEASLISQIADSLK (SEQ ID NO: 45) |
| SL~S~IA (SEQ ID NO: 32) | H0YEZ7_HUMAN Serine/threonine-protein kinase 33 (F | 129 | WIIQSLASAIAYLHN (SEQ ID NO: 44) |
| SL~S~IA (SEQ ID NO: 32) | H0YL06_HUMAN Membrane progestin receptor gamma (Fr | 79 | VNLFSLGSAIAYSAY (SEQ ID NO: 46) |
| SL~S~IA (SEQ ID NO: 32) | H7C4U7_HUMAN Membrane-associated guanylate kinase | 501 | NDTVSLASSIATQPE (SEQ ID NO: 47) |
| SL~S~IA (SEQ ID NO: 32) | H7C535_HUMAN Membrane-associated guanylate kinase | 507 | NDTVSLASSIATQPE (SEQ ID NO: 47) |
| SL~S~IA (SEQ ID NO: 32) | H7C5T8_HUMAN Membrane-associated guanylate kinase | 412 | NDTVSLASSIATQPE (SEQ ID NO: 47) |
| SL~S~IA (SEQ ID NO: 32) | I6L9I8_HUMAN EPN3 protein OS=Homo sapiens GN=EPN3 | 34 | GPPSSLMSEIADLTF (SEQ ID NO: 48) |
| SL~S~IA (SEQ ID NO: 32) | J3KTP8_HUMAN G-protein-coupled receptor family C g | 304 | LGEQSLQSRIAALTV (SEQ ID NO: 49) |
| SL~S~IA (SEQ ID NO: 32) | J3QSH4_HUMAN Vascular endothelial zinc finger 1 OS | 114 | SSRTSLVSTIAGILS (SEQ ID NO: 50) |
| SL~S~IA (SEQ ID NO: 32) | K7EJN9_HUMAN Protein YIPF2 (Fragment) OS=Homo sapi | 57 | PQITSLPSNIALSPT (SEQ ID NO: 51) |
| SL~S~IA (SEQ ID NO: 32) | K7ENM8_HUMAN Protein YIPF2 OS=Homo sapiens GN=YIPF | 255 | PQITSLPSNIALSPT (SEQ ID NO: 51) |
| SL~S~IA (SEQ ID NO: 32) | K7EQH3_HUMAN Protein YIPF2 (Fragment) OS=Homo sapi | 147 | PQITSLPSNIALSPT (SEQ ID NO: 51) |
| SL~S~IA (SEQ ID NO: 32) | ECM2_HUMAN Extracellular matrix protein 2 OS=Homo | 160 | TVSYSLLSGIALNDR (SEQ ID NO: 52) |
| SL~S~IA (SEQ ID NO: 32) | CTGE8_HUMAN cTAGE family member 8 OS=Homo sapiens | 179 | DESKSLKSQIAEAKI (SEQ ID NO: 53) |
| SL~S~IA (SEQ ID NO: 32) | CLD2_HUMAN Claudin-2 OS=Homo sapiens GN=CLDN2 PE=1 | 167 | GIISSLFSLIAGIIL (SEQ ID NO: 54) |
| SL~S~IA (SEQ ID NO: 32) | CO4A6_HUMAN Collagen alpha-6(IV) chain OS=Homo sap | 1245 | APGISLPSLIAGQPG (SEQ ID NO:55 ) |
| SL~S~IA (SEQ ID NO: 32) | CO4A6_HUMAN Isoform B of Collagen alpha-6(IV) chai | 1244 | APGISLPSLIAGQPG (SEQ ID NO: 55) |
| SL~S~IA (SEQ ID NO: 32) | VEZF1_HUMAN Vascular endothelial zinc finger 1 OS= | 123 | SSRTSLVSTIAGILS (SEQ ID NO: 56) |
| SL~S~IA (SEQ ID NO: 32) | K2022_HUMAN Uncharacterized protein KIAA2022 OS=Ho | 96 | VNAISLTSGIAKGLN (SEQ ID NO: 57) |
| SL~S~IA (SEQ ID NO: 32) | GP179_HUMAN Probable G-protein coupled receptor 17 | 1450 | ECSGSLGSGIAEVCL (SEQ ID NO:58 ) |
| SL~S~IA (SEQ ID NO: 32) | RFIP1_HUMAN Rab11 family-interacting protein 1 OS= | 593 | SVFSSLSSPIAAPIS (SEQ ID NO: 59) |
| SL~S~IA (SEQ ID NO: 32) | CTGE6_HUMAN cTAGE family member 6 OS=Homo sapiens | 179 | DESKSLKSQIAEAKI (SEQ ID NO:60 ) |
| SL~S~IA (SEQ ID NO: 32) | MTMRD_HUMAN Myotubularin-related protein 13 OS=Hom | 147 | SLNVSLESLIANLCA (SEQ ID NO: 61) |
| SL~S~IA (SEQ ID NO: 32) | MTMRD_HUMAN Isoform 3 of Myotubularin-related prot | 147 | SLNVSLESLIANLCA (SEQ ID NO: 61) |
| SL~S~IA (SEQ ID NO: 32) | TRI42_HUMAN Tripartite motif-containing protein 42 | 332 | ERAASLFSAIAKFKA (SEQ ID NO: 62) |
| SL~S~IA (SEQ ID NO: 32) | TRI42_HUMAN Isoform 2 of Tripartite motif-containi | 332 | ERAASLFSAIAKFKA (SEQ ID NO: 62) |
| SL~S~IA (SEQ ID NO: 32) | CTGE4_HUMAN cTAGE family member 4 OS=Homo sapiens | 179 | DESKSLKSQIAEAKI (SEQ ID NO: 63) |
| SL~S~IA (SEQ ID NO: 32) | FANCB_HUMAN Fanconi anemia group B protein OS=Homo | 768 | VTLSSLSSAIAKHES (SEQ ID NO:64 ) |
| SL~S~IA (SEQ ID NO: 32) | ARAP1_HUMAN Arf-GAP with Rho-GAP domain | 300 | SLSLSLPSTIAAPHP (SEQ ID NO: 65) |
| SL~S~IA (SEQ ID NO: 32) | ARAP1_HUMAN Isoform 1 of Arf-GAP with Rho-GAP doma | 60 | SLSLSLPSTIAAPHP (SEQ ID NO:65 ) |
| SL~S~IA (SEQ ID NO: 32) | ARAP1_HUMAN Isoform 2 of Arf-GAP with Rho-GAP doma | 60 | SLSLSLPSTIAAPHP (SEQ ID NO: 65) |
| SL~S~IA (SEQ ID NO: 32) | ARAP1_HUMAN Isoform 3 of Arf-GAP with Rho-GAP doma | 300 | SLSLSLPSTIAAPHP (SEQ ID NO: 65) |
| SL~S~IA (SEQ ID NO: 32) | ARAP1_HUMAN Isoform 4 of Arf-GAP with Rho-GAP doma | 55 | SLSLSLPSTIAAPHP (SEQ ID NO: 65) |
| SL~S~IA (SEQ ID NO: 32) | ARAP1_HUMAN Isoform 7 of Arf-GAP with Rho-GAP doma | 55 | SLSLSLPSTIAAPHP (SEQ ID NO: 65) |
| SL~S~IA (SEQ ID NO: 32) | MAGI1_HUMAN Membrane-associated guanylate kinase | 626 | NDTVSLASSIATQPE (SEQ ID NO: 66) |
| SL~S~IA (SEQ ID NO: 32) | MAGI1_HUMAN Isoform 2 of Membrane-associated guany | 626 | NDTVSLASSIATQPE (SEQ ID NO: 66) |
| SL~S~IA (SEQ ID NO: 32) | MAGI1_HUMAN Isoform 3 of Membrane-associated guany | 626 | NDTVSLASSIATQPE (SEQ ID NO: 66) |
| SL~S~IA (SEQ ID NO: 32) | MAGI1_HUMAN Isoform 4 of Membrane-associated guany | 626 | NDTVSLASSIATQPE (SEQ ID NO: 66) |
| SL~S~IA (SEQ ID NO: 32) | MAGI1_HUMAN Isoform 5 of Membrane-associated guany | 626 | NDTVSLASSIATQPE (SEQ ID NO: 66) |
| SL~S~IA (SEQ ID NO: 32) | MAGI1_HUMAN Isoform 6 of Membrane-associated guany | 626 | NDTVSLASSIATQPE (SEQ ID NO: 66) |
| SL~S~IA (SEQ ID NO: 32) | MAGI1_HUMAN Isoform 7 of Membrane-associated guany | 626 | NDTVSLASSIATQPE (SEQ ID NO: 66) |
| SL~S~IA (SEQ ID NO: 32) | YIPF2_HUMAN Protein YIPF2 OS=Homo sapiens GN=YIPF2 | 294 | PQITSLPSNIALSPT (SEQ ID NO: 67) |
| SL~S~IA (SEQ ID NO: 32) | STK33_HUMAN Serine/threonine-protein kinase 33 OS= | 217 | WIIQSLASAIAYLHN (SEQ ID NO: 68) |
| SL~S~IA (SEQ ID NO: 32) | STK33_HUMAN Isoform 2 of Serine/threonine-protein | 217 | WIIQSLASAIAYLHN (SEQ ID NO: 68) |
| SL~S~IA (SEQ ID NO: 32) | ZMY15_HUMAN Zinc finger MYND domain-containing pro | 468 | WRGLSLDSPIAVLLT (SEQ ID NO: 69) |
| SL~S~IA (SEQ ID NO: 32) | ZMY15_HUMAN Isoform 3 of Zinc finger MYND domain-c | 468 | WRGLSLDSPIAVLLT (SEQ ID NO: 69) |
| SL~S~IA (SEQ ID NO: 32) | EPN3_HUMAN Epsin-3 OS=Homo sapiens GN=EPN3 PE=2 SV | 34 | GPPSSLMSEIADLTF (SEQ ID NO: 70)(SEQ ID NO: 70) |
| SL~S~IA (SEQ ID NO: 32) | EPN3_HUMAN Isoform 2 of Epsin-3 OS=Homo sapiens GN | 34 | GPPSSLMSEIADLTF (SEQ ID NO: 70) |
| SL~S~IA (SEQ ID NO: 32) | 051V1_HUMAN Olfactory receptor 51V1 OS=Homo sapien | 98 | IREISLDSCIAQSYF (SEQ ID NO: 71) |
| SL~S~IA (SEQ ID NO: 32) | RPA2_HUMAN DNA-directed RNA polymerase I subunit R | 661 | LFPHSLLSVIANFIP (SEQ ID NO: 72) |
| SL~S~IA (SEQ ID NO: 32) | RPA2_HUMAN Isoform 2 of DNA-directed RNA polymeras | 605 | LFPHSLLSVIANFIP (SEQ ID NO: 72) |
| SL~S~IA (SEQ ID NO: 32) | T184C_HUMAN Transmembrane protein 184C OS=Homo sap | 90 | VPIYSLDSWIALKYP (SEQ ID NO: 73) |
| SL~S~IA (SEQ ID NO: 32) | T184C_HUMAN Isoform 2 of Transmembrane protein 184 | 90 | VPIYSLDSWIALKYP (SEQ ID NO: 73) |
| SL~S~IA (SEQ ID NO: 32) | MPRG_HUMAN Membrane progestin receptor gamma OS=Ho | 123 | VNLFSLGSAIAYSAY (SEQ ID NO: 74) |
| SL~S~IA (SEQ ID NO: 32) | SCN8A_HUMAN Sodium channel protein type 8 subunit | 1271 | IVAVSLVSLIANALG (SEQ ID NO: 75) |
| SL~S~IA (SEQ ID NO: 32) | SCN8A_HUMAN Isoform 2 of Sodium channel protein ty | 1271 | IVAVSLVSLIANALG (SEQ ID NO: 75) |
| SL~S~IA (SEQ ID NO: 32) | SCN8A_HUMAN Isoform 3 of Sodium channel protein ty | 1282 | IVAVSLVSLIANALG (SEQ ID NO: 75) |
| SL~S~IA (SEQ ID NO: 32) | PCDB7_HUMAN Protocadherin beta-7 OS=Homo sapiens G | 347 | LLLSSLTSPIAENSP (SEQ ID NO: 76) |
| SL~S~IA (SEQ ID NO: 32) | EPN1_HUMAN Epsin-1 OS=Homo sapiens GN=EPN1 PE=1 SV | 34 | GPSSSLMSEIADLTY (SEQ ID NO: 77) |
| SL~S~IA (SEQ ID NO: 32) | EPN1_HUMAN Isoform 2 of Epsin-1 OS=Homo sapiens GN | 145 | GPSSSLMSEIADLTY (SEQ ID NO: 77) |
| SL~S~IA (SEQ ID NO: 32) | EPN1_HUMAN Isoform 3 of Epsin-1 OS=Homo sapiens GN | 34 | GPSSSLMSEIADLTY (SEQ ID NO: 77) |
| VP~G~IV (SEQ ID NO: 17) | A8MT40_HUMAN Pyruvate dehydrogenase phosphatase re | 451 | NDLDVPVGHIVHTGM (SEQ ID NO: 86) |
| VP~G~IV (SEQ ID NO: 17) | D6RB49_HUMAN Death domain-containing protein 1 OS= | 658 | VVYKVPKGKIVPNLN (SEQ ID NO: 78) |
| VP~G~IV (SEQ ID NO: 17) | E9PD98_HUMAN Intersectin-1 OS=Homo sapiens GN=ITSN | 1700 | LLHEVPTGEIVVRLD (SEQ ID NO: 79) |
| VP~G~IV (SEQ ID NO: 17) | F8WDR2_HUMAN tRNA (adenine(58)-N(1))-methyltransfe | 183 | NWGAVPFGKIVGKFP (SEQ ID NO: 80) |
| VP~G~IV (SEQ ID NO: 17) | H0Y6Q5_HUMAN Uncharacterized protein OS=Homo sapie | 27 | CVPSVPVGPIVLTSA (SEQ ID NO: 81) |
| VP~G~IV (SEQ ID NO: 17) | USH2A_HUMAN Usherin OS=Homo sapiens GN=USH2A PE=1 | 1548 | FRTKVPEGLIVFAAS (SEQ ID NO: 82) |
| VP~G~IV (SEQ ID NO: 17) | USH2A_HUMAN Isoform 3 of Usherin OS=Homo sapiens G | 1548 | FRTKVPEGLIVFAAS (SEQ ID NO: 82) |
| VP~G~IV (SEQ ID NO: 17) | EF2_HUMAN Elongation factor 2 OS=Homo sapiens GN=E | 460 | PIEDVPCGNIVGLVG (SEQ ID NO: 83) |
| VP~G~IV (SEQ ID NO: 17) | ITSN1_HUMAN Intersectin-1 OS=Homo sapiens GN=ITSN1 | 1700 | LLHEVPTGEIVVRLD (SEQ ID NO: 84) |
| VP^{~}G^{~}IV (SEQ ID NO: 17) | ITSN1_HUMAN Isoform 4 of Intersectin-1 OS=Homo sap | 1629 | LLHEVPTGEIVVRLD (SEQ ID NO: 84) |
| VP^{~}G^{~}IV (SEQ ID NO: 17) | ITSN1_HUMAN Isoform 8 of Intersectin-1 OS=Homo sap | 1695 | LLHEVPTGEIVVRLD (SEQ ID NO: 84) |
| VP^{~}G^{~}IV (SEQ ID NO: 17) | ITSN1_HUMAN Isoform 9 of Intersectin-1 OS=Homo sap | 1639 | LLHEVPTGEIVVRLD (SEQ ID NO: 84) |
| VP^{~}G^{~}IV (SEQ ID NO: 17) | DTHD1_HUMAN Death domain-containing protein 1 OS=H | 618 | VVYKVPKGKIVPNLN (SEQ ID NO: 85) |
| VP^{~}G^{~}IV (SEQ ID NO: 17) | DTHD1_HUMAN Isoform 2 of Death domain-containing p | 453 | VVYKVPKGKIVPNLN (SEQ ID NO: 85) |
| VP^{~}G^{~}IV (SEQ ID NO: 17) | PDPR_HUMAN Pyruvate dehydrogenase phosphatase regu | 551 | NDLDVPVGHIVHTGM (SEQ ID NO: 86) |
| VP^{~}G^{~}IV (SEQ ID NO: 17) | SHCBP_HUMAN SHC SH2 domain-binding protein 1 OS=Ho | 235 | LEDRVPSGLIVDYHN (SEQ ID NO: 87) |
| VP^{~}G^{~}IV (SEQ ID NO: 17) | HASP_HUMAN Serine/threonine-protein kinase haspin | 314 | QEASVPKGRIVPRGI (SEQ ID NO: 88) |
| VP^{~}G^{~}IV (SEQ ID NO: 17) | FAP24_HUMAN Fanconi anemia-associated protein of 2 | 10 | GPVHVPLGHIVANEK (SEQ ID NO: 89) |
| VP^{~}G^{~}IV (SEQ ID NO: 17) | TR61B_HUMAN tRNA (adenine(58)-N(1))-methyltransfer | 183 | NWGAVPFGKIVGKFP (SEQ ID NO: 90) |
| VP^{~}G^{~}IV (SEQ ID NO: 17) | FNDC4_HUMAN Fibronectin type III domain-containing | 65 | VSWDVPEGNIVIGYS (SEQ ID NO: 91) |

### Example 9 Example of a join with human proteome

In Example 4 we describe certain TCEM motifs found in the hemagglutinin of a specific strain of influenza and having a high binding affinity to DQA1*01:02/DQB1*06:02 DQ.

As described in Example 1 we have extracted the TCEM motifs found in the human proteome. By matching (joining) motif datasets from influenza and the human proteome we demonstrated that the motifs AG~G~II (SEQ ID NO: 470) and NA~S~II (SEQ ID NO: 471) are found in proteins shown in **Figure 13****.** This illustrates the process by which we approach the search for a TCEM mimic in self-proteins.

### Example 10. Identification of probable Treg motifs in Tumor associated antigen proteins

We examined a number of tumor associated antigens (TAA) for the presence of TCEM motifs which might contribute to down regulation of the immune response to tumors thereby creating tolerance. Some TAA have distinct and highly probable Treg motifs (e.g. PMEL). In other cases the balance of immunostimulation and immunosuppression appears more subtle (e.g. MART, MAGE). In neoplasias a consideration which differs from healthy tissues is that there is a density of cells locally or metastasized, all expressing the same motifs, which can lead to a shift in the relative emphasis or frequency of a given motif locally or systemically, potentially starting a downward spiral of suppression and tolerance. Hence what appears as a FC14 relative to the normal frequency in a balanced database of IgV may locally become much more common and lead to an accumulation of cognate T cells. This is especially the case when the GEM binding is particularly high affinity, ensuring that the TCEM exposure to T cells is extended.

In this example we examine TCEM motifs in TAA, PMEL MART and MAGE-1. These are examples and should not therefore be considered limiting. Rather the intent is to show an approach that allows examination of the TCEMs in determining overall outcome of the tumor immune response.

The same approach is applicable to characterizing the TCEM in a tumor neo antigen created by a novel mutation and thus enabling characterization of the immune response to a neo antigen and enabling various intervention strategies. Thus the examples applied to well recognize tumor associated antigens should not be considered limiting.

### Identification of probable Treg peptides in PMEL

An evaluation of the TCEM in PMEL (gi 2507099 Melanocyte protein PMEL) was conducted based on comparison with the frequency of occurrence of TCEM motifs in the immunoglobulin variable region reference databases comprising both heavy and light chains. Two probable Treg motifs are identified (Figure 14). These both have binding to MHC I alleles. The peptide with index position of its 9 mer at 213 is a FC5 peptide found in light chain variable regions, ~~SVSVS~ (SEQ ID NO: 472); the 9-mer peptide with index position at 334 is a light chain FC 3 ~~PGQAP~ (SEQ ID NO: 473). When evaluated along with their corresponding GEM binding both have several high binding alleles and generate suppressive indices of 33,519 and 20,179 respectively. This indicates more high binding alleles for the peptide at 213. Either peptide would be expected to cause significant down regulation of a response to PMEL.

When two isoforms of PMEL (gi 318037593 melanocyte protein PMEL isoform 2 precursor and gi318037595 melanocyte protein PMEL isoform 1 precursor) are compared, the isoform 1 is found to contain both motifs whereas the isoform 2 only contains ~~PGQAP~. Potential Tregs in PMEL are recorded in Table 12

### MART-1

The distribution of TCEM motifs in MART presents a more subtle picture..

The MART-1 protein (Melanoma antigen recognized by T-cells 1) was recognized in 1994 to be overexpressed in melanoma (Coulie,[29] Kawakami [30,31]). It was subsequently found to be expressed in some other cancers, including but not limited to breast cancer. Specific peptides were identified which upregulated CD8+ responses in melanoma patients (Kawakami [31]), in particular a peptide AAGIGILTV (SEQ ID NO: 474) which comprises amino acid positions 27-35 was identified as an immunodominant CD8+ epitope restricted by HLA A 0201. This peptide has been deployed as therapeutic vaccines for melanoma [32,33]. Notably this work was with isolated peptides. Other peptides in MART-1 have further been described as having binding affinity for other MHC-I HLA alleles [34] and for certain MHC-II alleles [35]. It has further been shown that the co-administration of a drug which curtails Treg activity enhances the immune response to the peptide aa27-35 used as a therapeutic melanoma vaccine [36,37]. This suggests that Tregs may play a key role in modulating the response to MART.

In order to better understand the balance of up-regulating and down-regulating immune responses invoked by the MART-1 protein we first examined the predicted MHC binding affinity as determined by the methods previously described (PCT US2011/029192, ). This identified a cluster of peptides around 25-50 which have high binding affinity for various MHC-I alleles. This is shown in **Figure 15** and Table 11.

**Table 11** shows that a number of MHC-I alleles have high binding affinity for peptides with their index positions between 25 and 50. The binding affinity is shown in standard deviation units, with higher binding indicated by more negative numbers.

| index amino acid | 9-mer | nA_0201 |
|---|---|---|
| 25 | EEAAGIGIL (SEQ ID NO: 92) | 0.71 |
| 26 | EAAGIGILT(SEQ ID NO: 93) | 0.10 |
| **27** | **AAGIGILTV**(SEQ ID NO: 94) | **-1.17** |
| 28 | AGIGILTVI(SEQ ID NO: 95) | -1.25 |
| 29 | GIGILTVIL(SEQ ID NO: 96) | -1.42 |
| 30 | IGILTVILG(SEQ ID NO: 97) | -1.50 |
| 31 | GILTVILGV(SEQ ID NO: 98) | -2.23 |
| 32 | ILTVILGVL(SEQ ID NO: 99) | -2.02 |
| 33 | LTVILGVLL(SEQ ID NO: 100) | -1.61 |
| 34 | TVILGVLLL(SEQ ID NO: 101) | -1.59 |
| 35 | VILGVLLLI(SEQ ID NO: 102) | -2.17 |
| 36 | ILGVLLLIG(SEQ ID NO: 103) | -2.05 |
| 37 | LGVLLLIGC(SEQ ID NO: 104) | -1.41 |
| 38 | GVLLLIGCW(SEQ ID NO: 105) | 0.31 |
| 39 | VLLLIGCWY(SEQ ID NO: 106) | -1.12 |
| 40 | LLLIGCWYC(SEQ ID NO: 107) | -1.73 |
| 41 | LLIGCWYCR(SEQ ID NO: 108) | -0.55 |
| 42 | LIGCWYCRR(SEQ ID NO:109 ) | 0.47 |
| 43 | IGCWYCRRR(SEQ ID NO: 110) | 0.95 |
| 44 | GCWYCRRRN(SEQ ID NO: 111) | 0.95 |
| 45 | CWYCRRRNG(SEQ ID NO: 112) | -1.09 |
| 46 | WYCRRRNGY(SEQ ID NO: 113) | -0.27 |
| 47 | YCRRRNGYR(SEQ ID NO: 114) | 0.83 |
| 48 | CRRRNGYRA(SEQ ID NO: 115) | -0.08 |
| 49 | RRRNGYRAL(SEQ ID NO: 116) | -0.41 |
| 50 | RRNGYRALM(SEQ ID NO: 117) | -0.12 |

We further showed, by application of the methods described in PCT US2014/041525, that many of the high binding peptides are excised by cathepsin enabling their binding by MHC. Notably for A*0201 several peptides have higher predicted binding affinity than that which has its index position at aa 27 (AAGIGILTV), as seen in Table 3. However Kawakami [31] showed that only those with index at 27 and 29 stimulated a response. Index positon 28 was not tested by Kawakami.

We then conducted an analysis to identify whether TCEM motifs in MART-1 were present in the reference database of IGHV described in **Example 2.** The results are shown in **Figures 16** , **17** **and** 18.Figure 16- shows the comparative output for MHC-I motifs. Figures 17 and 18 shows output for TCEM IIa and TCEM IIb.

While several alleles of MHC-II have a strong binding affinity across the 25-50 positions, it is also noted that 15-mer peptides with index position at 37 are found to have high affinity TCEM IIa and TCEM IIb motifs for almost all MHC-II alleles, and these motifs are also in the IGHV reference database where they show consistently high predicted MHC binding affinity. The frequency of occurrence of the TCEM motif with index position 37 in the IgV database is TCEM IIA = FC 14 and TCEM IIB= FC 15. Thus the peptide with index at 37 is would find a preexisting cognate T cell population and would bind strongly to MHC II, for almost all MHC-II alleles. This peptide overlaps all those with MHC-I binding positions except those with index position 27 or lower. Specifically the Treg motifs in this instance are those contained in peptide LGVL**LL**I**G**C**WY**CRRR (SEQ ID NO: 475), where the underscores indicate TCEM IIa motif LLXGXWY (SEQ ID NO: 476) and LG**V**LL**L**I**G**C**WY**CRRR (SEQ ID NO: 477) where the underscores indicate TCEM IIb motif VXXLXGXWY (SEQ ID NO: 478). This is shown in **Figure 19**

The finding of a potential Treg in this position is consistent with the observations of Kawakami that peptide index 27 binding A*0201 is an active immune up-regulator, while those in adjacent higher amino acid positions are not. It is also consistent with the observations of Mahnke and Rasku that depletion of Tregs enhances the immune response. Identification of this Treg offers opportunities to design interventions in which the Treg function is eliminated by specific T-cell targeting and thus enhances the therapeutic vaccine effect. Potential Tregs are recorded in Table 5

### MAGE proteins

Melanoma antigen E (MAGE) family antigens belong to the so-called cancer/testis TAA subclass and are expressed in normal tissues but particularly overexpressed in melanoma and several other tumor types. Epitopes derived from MAGE and recognized by cytotoxic T cells have been identified and are the subject of efforts to build anti-cancer vaccines.

We analyzed 19 proteins which are members of the melanoma associated antigen protein family and determined the presence of potential Tregs in each. As one example in particular the MAGE-1 protein (gi 148276977) was analyzed. Potential Tregs are recorded in Table 5

**Table 12. Potential Tregs in Tumor associated antigens. In Motif column "X" indicates any amino acid.**

| Index amino acid | 9-mer or 15-mer peptide | TCEM register | Motif |
|---|---|---|---|
| *PMEL gi 2507099* | | | |
| 213 | SEQ ID NO: 118: PFSVSVSQ | TCEM I | SEQ ID NO: 119: XXSVSVSX |
| 334 | SEQ ID NO: 120: TTPGQAPT | TCEM I | SEQ ID NO: 121: XXPGQAPX |

| *MART-1 gi 2833278* | | | |
|---|---|---|---|
| 37 | SEQ ID NO: 122: LGVLLLIGCWYCRRR | TCEM I | SEQ ID NO: 123: LLXGXWY |
| | | TCEM I | SEQ ID NO: 124: VXXLXGXWY |

| *MAGE-1 gi 148276977* | | | |
|---|---|---|---|
| 33 | SEQ ID NO: 125: SSSSPLVLGTLEEVP | TCEM IIb | SEQ ID NO: 126: SXXLXLXTL |
| 34 | SEQ ID NO: 127: SSSPLVLGTLEEVPT | TCEM IIa | SEQ ID NO: 128: LVXGXLE |
| 36 | SEQ ID NO: 129: SPLVLGTLEEVPTAG | TCEM IIa | SEQ ID NO: 130: LGXLXEV |
| 192 | SEQ ID NO: 131: TGFLIIVLVMIAMEG | TCEM IIa | SEQ ID NO: 132: IIXLXMI |
| 193 | SEQ ID NO: 133: GFLIIVLVMIAMEGG | TCEM IIb | SEQ ID NO: 134: LXVXVXIA |

### Example 11 Identification of TCEM motifs in Myelin Basic protein

Myelin basic protein has been implicated as a target of autoimmune disease in which a demyelination occurs. We examined myelin basic protein including isotypes 1-4 and examined the occurrence of peptides with TCEMs that occur at high frequency in IGHV. Several TCEM IIa are present with Frequency Categories in IgV less than FC10 as shown in Figure 20. Three of these in particular, at index positions 131, (FC5),236 (FC3) and 283 (FC9) are also associated with high MHC binding affinity giving them a high suppressive index as shown in Figure 20. These are summarized in Table 13.

**Table 13**

| Index amino acid | 15-mer peptide | TCEM register | Motif |
|---|---|---|---|
| *Myelin basic protein gi 17378805* | | | |
| 131 | SEQ ID NO: 135: LDVMASQKRPSQRHG | TCEM IIa | SEQ ID NO: 136: ASXKXPS |
| 236 | SEQ ID NO: 137: SQGKGRGLSLSRFSW | TCEM IIa | SEQ ID NO: 138: GRXLXLS |
| 283 | SEQ ID NO: 139: TLSKIFKLGGRDSRS | TCEM IIa | SEQ ID NO: 140: IFXLXGR |

### Example 12: Identification of TCEM motifs viral infections

### Hepatitis B virus core protein

The association of chronic hepatitis B virus infection with incidence of liver cancer is well documented. There has not been a proven causal association, rather a strong association [38]. Recent reports have noted the relationship of increased Treg activity with progression of chronic hepatitis B. One report has linked increased Tregs directed to the core protein with chronic hepatitis B infection [39]. Vaccination using various formulations of hepatitis S antigen (HbsAg, surface protein) has been very effective at prevention of hepatitis B infection. However it has not been possible to design vaccines which aid the resolution of chronic infection. Hepatitis B has a particularly high incidence in China, although it is unclear whether this has a relationship with ethnicity or environmental factors enhancing transmission. There are however several reports of relationships between HLA alleles and incidence of hepatitis B [40,41]. Hepatitis B has been categorized into multiple genotypes (reviewed by Kramvis et al [42] ).

We examined all the proteins of multiple strains of hepatitis B virus in order to determine if TCEM motifs that are found at high frequency in the 40K IGHV database referenced elsewhere in this application are represented and if so whether they have predicted high binding affinity to IGHV as well as in the hepatitis protein of interest.

Examination of MHC-I TCEMs showed no notable features. In proteins X, polymerase, large S, PreS and S some high binding peptides were noted but all were of Frequency class 16, indicating the motifs occur only once in the 40,000 IGHV database. The observations of the core protein and the precore and core protein were notably different. In the core protein the 15mer peptide with index position 120, VSFGVWIRTPPAYRP (SEQ ID NO: 479), showed both high frequency and high predicted binding in both frameworks of TCEM-II. TCEM-IIa showed binding in Frequency class 12 (1 in every 4096 heavy chain variable regions) and binding of greater than 1σ for all DR and for all DP except DPA1_0103-DPB1_0402. No binding occurred to DQ alleles (except at low affinity to DQA1_0101_DQB1_0501. When TCEM-IIB was examined the same peptide with index position 120 was found to have a Frequency Class of 8, indicating this motif is resent in 1 of every 256 heavy chain variable regions. Furthermore binding occurred as above to all DR, almost all DP and no DQ alleles except DQA1_0101_DQB1_0501. For TCEM-IIA the motif is F~~W~R~PP (SEQ ID NO: 480) whereas for TCEM IIB the motif is VW~R~PP (SEQ ID NO: 481). The peptide with index position 120 is excised by cathepsin B.

Figures 21 and 22 show the binding patterns of peptides in the hepatitis core protein and motif matches with the IGHV database.

The peptide VSFGVWIRTPPAYRP (SEQ ID NO: 482) and the motifs it includes F~~W~R~PP (SEQ ID NO: 483) and VW~R~PP (SEQ ID NO: 484) are conserved across all genotypes A-H of hepatitis B. Other changes occur in the core protein between genotypes but this peptide is conserved.

These motifs have all the characteristics of T regulatory motifs and are present in all strains of hepatitis B virus. The presence of a strong conserved T regulatory motif in chronic cases of hepatitis B would inhibit the mounting of an immune response capable of eliminating dysplastic or neoplastic cells arising in the liver. Hence chronic hepatitis B would be permissive to the progression of cancer in the liver. It follows that it would be possible to target T regulatory cells specifically by using a synthetic construct of the identified peptide or the TCEM IIA or TCEM IIB motifs it encompasses and an MHC molecule to deliver a cytotoxin to eliminate the Treg cells.

### Human papillomavirus.

Human papillomavirus is a cause of squamous cell carcinoma of the cervix and oropharynx, and a cause of genital warts. A clear association of certain strains of HPV with progression of infection to cancer is reported, with strain HPV 16 being associated with 70% of reported cervical cancer cases. Strains 31, 33 and several others are known to be particularly aggressive in oncogenesis, whereas others such as type 6 and 11 are not associated with cancer [43-45]. While a preventive vaccine has been developed based on the surface proteins L1 and L2, there is as yet no vaccine approved for treatment of established infections. The oncogenic protein E7 has been the focus of efforts to develop such a vaccine [46,47]. Using the tools developed and described in the present invention we mapped the TCEMs in all proteins of selected strains (16, 18, 6, 11) and in the E7 proteins of 78 strains. For 31 strains we mapped, information was available documenting the degree of risk of progression to carcinoma. We documented the "immunosuppression index:" as a measure of the number and frequency (FC class) of TCEM found in the 40K set multiplied by the alleles binding these TCEM. The highest index of immunosuppression was observed in TCEM type II of strains 16, 31, 33, 18, 45, 58 and 68. These are all strains documented as being of higher risk of progression [48].

The TCEM motifs contributing most to the immunosuppression score were determined and also plotted on graphics. The cathepsin excision of the TCEM of interest was verified. Comparative plots of low risk strains again verified that these motifs were absent. Results are shown in Table 7. The TCEM shown are thus apparent Tregs associated with risk of progression.

Analysis was conducted of the E7 and E6 proteins of HPV strains from the Alpha papillomaviruses species Alpha 1, 3, 5, 6, 7, 8, 9, 10, 11 and 13 available at http://pave.niaid.nih.gov/. This identified additional TCEMs in E7 and added motifs in E6, which are occurring at high frequency and binding with high affinity in the reference database. These are predicted Treg motifs. Initially TCEMs were identified which correspond to high frequency motifs in the IGHV and IGLV databases. Each of these was then evaluated individually to assess the probability of cathepsin cleavage to enable presentation in a MHC molecule and to evaluate how many HLA alleles bind the peptide containing the TCEM. Those TCEMs which are not bound or predicted to be excised were eliminated from the list. Table 7 and Table 8 show the resulting motifs identified for E7 and E6 respectively. Figures 23 and 24 show the location of the motifs in the protein sequences.

**Table 14: E7 Critical motifs**

| In these tables the ~ character implies the presence of GEM sequences not seen by the T-cell The IARC risk category refers to the classification in Muñoz et al 2003 New Eng J Med 348, 518-527. | | | | | | | | | |
|---|---|---|---|---|---|---|---|---|---|
| ALPHA | Type | IARC Risk class | Motif based Classification | IIA | FCIIa | IIB | FCIIb | I | FC1 |
| 1 | 42 | L | H | | | N~~Q~L~GT (SEQ ID NO: 141) | 6 | | |
| 3 | 61 | L | LL | | | | | | |
| 3 | 72 | L | L | | | V~~C~R~YR(SEQ ID NO: 142) | 10 | | |
| 3 | 81 | L | LL | | | | | | |
| 5 | 26 | *H | H | | | Q~~Q~G~EV(SEQ ID NO:143) | 4 | | |
| 5 | 51 | H | LL | | | | | | |
| 5 | 82 | H | H | | | | | GDSLR (SEQ ID NO: 199) | 6 |
| 6 | 53 | *H | LL | | | | | | |
| 6 | 56 | H | LL | | | | | | |
| 6 | 66 | *H | LL | | | | | | |
| 7 | 18 | H | H | | | R~~L~V~SS(SEQ ID NO: 144) | 8 | | |
| 7 | 39 | H | HH | | | L~~L~M~SL(SEQ ID NO: 145) | 9 | SRDTL(SEQ ID NO: 200) | 10 |
| 7 | 45 | H | H | | | R~~L~V~SS(SEQ ID NO: 144) | 8 | | |
| 7 | 59 | H | H | | | L~~Y~Q~PD(SEQ ID NO: 146) | 10 | | |
| 7 | 68 | H | HH | | | L~~L~M~SL(SEQ ID NO: 147) | 9 | | |
| 7 | 70 | ? | LL | | | | 9 | | |
| 8 | 40 | L | LL | | | | | | |
| 8 | 43 | L | LL | | | | | | |
| 9 | 16 | H | HH | PE^{~}T^{~}LY(SEQ ID NO: 148) | 8 | L~~E~T~LY(SEQ ID NO: 149) | 5 | | |
| 9 | 31 | H | HH | | | L~~Y~Q~PD(SEQ ID NO: 146) | 10 | | |
| 9 | 33 | H | HH | PE^{~}T^{~}LY(SEQ ID NO: 148) | 8 | L~~E~T~LY(SEQ ID NO: 149) | 5 | | |
| 9 | 35 | H | HH | SS~E~ED(SEQ ID NO: ) | 5 | S~~K~K~TL(SEQ ID NO: 150) | 9 | | |
| | | | | | 8 | | 5 | | |
| | | | | PE~T~LY(SEQ ID NO: 148) | | L~~E~T~L~Y(SEQ ID NO: 149) | | | |
| 9 | 52 | H | HH | YC~S~DS(SEQ ID NO: 151) | 4 | | | | |
| 9 | 58 | H | HH | | | T~~Y~CTT(SEQ ID NO: 152) | *10* | | |
| 10 | 6 | L | LL | | | | | | |
| 10 | 11 | L | LL | | | | | | |
| 10 | 44 | L | LL | | | | | | |
| 11 | 73 | ? | LL | | | | | | |
| 13 | 54 | L | HH | PE~F~LY(SEQ ID NO: 153) | 9 | L~~E~F~LY(SEQ ID NO: 154) | 9 | | |

**Table 15: E6 Critical Motifs**

| ALPHA | Type | IARC Risk class | Motif based Classification | IIA | FCII a | IIB | FCII b | I | FC 1 |
|---|---|---|---|---|---|---|---|---|---|
| 1 | 42 | L | H | KE~G~TL(SE Q ID NO: 155) | 9 | K~~S~S~KN(SE Q ID NO: 156) | 9 | | |
| | | | | | 10 | | | | |
| | | | | LT~R~LQ(SE Q ID NO: 157) | | | | | |
| 3 | 61 | L | H | RG~C~AR(SEQ ID NO: 158) | 8 | | | | |
| 3 | 72 | L | HH | RG~C~AR(SE | 8 | | | | |
| | | | | Q ID NO:158) | 8 | | | | |
| | | | | | 10 | | | | |
| | | | | IF~K~EL(SEQ ID NO: 159) | | | | | |
| | | | | SG~G~TV(SE Q ID NO: 160) | | | | | |
| 3 | 81 | L | HH | RG~C~AR(SE | 8 | | | | |
| | | | | Q ID NO: 158) | 8 | | | | |
| | | | | IF~K~EL(SEQ ID NO: 159) | | | | | |
| 5 | 26 | *H | HH | TC~V~GA(SE Q ID NO: 161) | 8 | L~~V~R~RS(SE Q ID NO: 162) | 9 | | |
| 5 | 51 | H | HH | YS~S~YG(SE Q ID NO: 163) | 10 | S~~V~G~TL(SE Q ID NO: 164) | 9 | ~~KKSLY~(SE Q ID NO: 165) | 8 |
| 5 | 82 | H | HH | YS~S~YG(SE Q ID NO: 163) | 10 | S^{~~}V^{~}G^{~}TL(SE Q ID NO: 164) | 9 | | |
| 6 | 53 | *H | HH | | | T~~R~T~AT(SE Q ID NO: 166) | 10 | ~~VYGAS~(SE Q ID NO: 167) | 9 |
| 6 | 56 | H | HHH | | | S~~R~Y~YY(SE Q ID NO: 168) | 10 | ~~LRLSC~(SEQ ID NO: 169) | 1 |
| | | | | | | | | | 6 |
| | | | | | | | | ~~RLSCV~(SE Q ID NO: 170) | |
| 6 | 66 | *H | HHH | LL~L~LS(SEQ ID NO: 171) | 10 | Y~~T~S~LQ(SE Q ID NO: 172) | 8 | ~~LRLSC~(SEQ ID NO: 173) | 1 |
| | | | | | | | 9 | | 6 |
| | | | | | | S~~R~Y~YY(SE Q ID NO: 168) | | ~~RLSCV~(SE Q ID NO: 174) | 10 |
| | | | | | | | | ~~RNNWP~(S EQ ID NO: 175) | |
| 7 | 18 | H | HH | IE~T~VY(SE Q ID NO: 176) | 8 | K~~F~V~RD(S EQ ID NO: 178) | 6 | | |
| | | | | | 10 | | 9 | | |
| | | | | RQ~R~QR(S EQ ID NO: 177) | | S~~V~G~TL(SE Q ID NO: 179) | | | |
| 7 | 39 | H | HH | LT~R~TQ(SE Q ID NO: 180) | 9 | | | ~~YSDSV~(SE Q ID NO: 181) | 8 |
| | | | | | | | | | 9 |
| | | | | | | | | ~~YYSDS~(SE Q ID NO: 182) | 9 |
| | | | | | | | | ~~AGSYT~(SE Q ID NO: 183) | |
| 7 | 45 | H | HH | LE~T~VY(SE Q ID NO: 184) | 10 | S^{~~}V^{~}G^{~}TL(SE Q ID NO: 185) | 9 | | |
| 7 | 59 | H | L | | | | | | |
| 7 | 68 | H | L | | | | | | |
| 7 | 70 | ? | L | | | | | | |
| 8 | 40 | L | H | DS~T~YC(SE Q ID NO: 186) | 8 | | | | |
| 8 | 43 | L | L | | | | | | |
| 9 | 16 | H | H | | | Q~~P~K~PQ(S EQ ID NO: 187) | 8 | | |
| 9 | 31 | H | H | TD~T~VY(SE Q ID NO: 188) | 9 | | | | |
| 9 | 33 | H | H | AD~T~VY(SE Q ID NO: 189) | 6 | | | | |
| 9 | 35 | H | H | YC~Q~LQ(SE Q ID NO: 190) | 6 | E~~E~I~EI(SEQ ID NO: 191) | 10 | | |
| 9 | 52 | H | L | | | | | | |
| 9 | 58 | H | H | | | H~~S~R~TG(S EQ ID NO: 192) | 8 | | |
| 10 | 6 | L | H | | | T~~L~M~TL(SE Q ID NO: 193) | 8 | | |
| 10 | 11 | L | HH | AS~S~TS(SE Q ID NO: 194) | 7 | | | | |
| | | | | | 9 | | | | |
| | | | | DA~T~AT(SE Q ID NO: 195) | | | | | |
| 10 | 44 | L | H | | | V~~R~T~ST(SE Q ID NO: 196) | 10 | | |
| 11 | 73 | ? | L | | | | | | |
| 13 | 54 | L | H | SL~L~CA(SE Q ID NO: 197) | 2 | E~~G~I~YR(SE Q ID NO: 198) | 10 | | |

Tables 14 and 15 show critical motifs which were derived initially based on reference strains for HPV (http://pave.niaid.nih.gov/). We then evaluated up to ten strains of each type. The TCEMs tabulated in Tables 7 and 8 are well conserved. When multiple strains of HPV type 16 were evaluated we observed complete conservation between strains in these peptides, however in some types, for example type 31, there was more diversity of E7 between isolates. In addition, there are differences in individual amino acids which comprise the GEM motif ( ~ )s within the corresponding peptide, while retaining the conserved TCEM. Other isolates of HPV may contain additional TCEMs with properties likely to confer Treg stimulation. In the case of the Alpha 6 species, high frequency TCEM motifs are found for MHC I in protein E6, rather than for MHC II motifs in E7 as for the other Alpha species. We further evaluated the predicted binding affinity to 28 HLA Type II and 35 MHC Type I alleles and determined which alleles had the highest binding affinity for each motif.

The probable Treg motifs in HPV were identified (Tables 14 and 15) and the proteome database was searched to identify other human proteins which have these motifs. Relatively few such proteins were found. The proteins in which the motifs occurred are of interest as they could be the target of adverse targeting by immunotherapeutics; such proteins have now been identified. The summary of numbers of matches is shown in Table 16.

**Table 16 Proteome matches of critical HPV TCEM motifs**

| **E7** | | | Motif | FC | Proteome matches |
|---|---|---|---|---|---|
| Alpha 9 | 16,33 | TCEM IIA | PE~T~LY(SEQ ID NO: 201) | 8 | 16 |
| Alpha 9 | 35 | TCEM IIA | SS~E~ED(SEQ ID NO: 202) | 5 | 167 |
| Alpha 9 | 52 | TCEM IIA | YC~S~DS(SEQ ID NO: 203) | 4 | 0 |
| | | | | | |
| Alpha 7 | 39,68 | TCEM IIB | L~~L~M~SL(SEQ ID NO: 204) | 9 | 38 |
| Alpha 7 | 18,45 | TCEM IIB | R~~L~V~SS(SEQ ID NO: 205) | 8 | 56 |
| Alpha 9&7 | 59,31 | TCEM IIB | L~~Y~Q~PD(SEQ ID NO: 206) | 10 | 2 |
| Alpha 9 | 16,33 | TCEM IIB | L~~E~T~LY(SEQ ID NO: 207) | 5 | 36 |
| Alpha 9 | 58 | TCEM IIB | T~~Y~C~TT(SEQ ID NO: 208) | 10 | 0 |
| Alpha 9 | 35 | TCEM IIB | S~~K~K~TL(SEQ ID NO: 209) | 9 | 50 |

| E6 | | | Motif | FC | Proteome matches |
|---|---|---|---|---|---|
| Alpha 6 | 66 | TCEM IIA | ID NO: 210) | 10 | 436 |
| Alpha 7 | 18 | TCEM IIA | IE~T~VY(SEQ ID NO: 211) | 8 | 12 |
| Alpha 7 | 45 | TCEM IIA | LE~T~VY(SEQ ID NO: 212) | 10 | 1 |
| Alpha 7 | 39 | TCEM IIA | LT~R~TQ(SEQ ID NO: 213) | 9 | 3 |
| Alpha 7 | 18 | TCEM IIA | RQ~R~QR(SEQ ID NO: 214) | 10 | 27 |
| Alpha 9 | 31 | TCEM IIA | TD~T~VY(SEQ ID NO: 215) | 9 | 15 |
| Alpha 9 | 35 | TCEM IIA | YC~Q~LQ (SEQ ID NO: 216) | 6 | 8 |
| Alpha 9 | 33 | TCEM IIA | AD~T~VY(SEQ ID NO: 217) | 6 | 0 |
| Alpha 10 | 11 | TCEM IIA | AS~S~TS(SEQ ID NO: 218) | 7 | 127 |
| Alpha 10 | 11 | TCEM IIA | DA~T~AT(SEQ ID NO: 219) | 9 | 12 |
| | | | | | |
| Alpha 6 | 66 | TCEM IIB | Y~~T~S~LQ(SEQ ID NO: 220) | 8 | 24 |
| Alpha 6 | 56,66 | TCEM IIB | S~~R~Y~YY(SEQ ID NO: 221) | 9 | 0 |
| Alpha 6 | 53 | TCEM IIB | T~~R~T~AT(SEQ ID NO: 222) | 10 | 17 |
| Alpha 7 | 18 | TCEM IIB | K~~F~V~RD(SEQ ID NO: 223) | 6 | 14 |
| Alpha 7 | 18,45 | TCEM IIB | S~~V~G~TL(SEQ ID NO:224 ) | 9 | 59 |
| Alpha 9 | 35 | TCEM IIB | E~~E~I~EI(SEQ ID NO: 225) | 10 | 48 |
| Alpha 9 | 16 | TCEM IIB | Q~~P~K~PQ(SEQ ID NO: 226) | 8 | 11 |
| Alpha 9 | 58 | TCEM IIB | H~~S~R~TG(SEQ ID NO: 227) | 8 | 16 |
| Alpha 10 | 6 | TCEM IIB | T~~L~M~TL(SEQ ID NO: 228) | 8 | 43 |
| Alpha 10 | 44 | TCEM IIB | V~~R~T~ST(SEQ ID NO: 229) | 10 | 32 |
| | | | | | |
| Alpha 6 | 53 | TCEM I | ~~~VYGAS~(SEQ ID NO: 230) | 9 | 19 |
| Alpha 6 | 56,66 | TCEM I | ~~~LRLSC~(SEQ ID NO: 231) | 1 | 0 |
| Alpha 6 | 56,66 | TCEM I | ~~~RLSCV~(SEQ ID NO: 232) | 6 | 0 |
| Alpha 6 | 66 | TCEM I | ~~~RNNWP~(SEQ ID NO:233 ) | 10 | 0 |
| Alpha 7 | 39 | TCEM I | ~~~ YSDSV~(SEQ ID NO: 234) | 8 | 0 |
| Alpha 7 | 39 | TCEM I | ~~~YYSDS~(SEQ ID NO: 235) | 9 | 0 |
| Alpha 7 | 39 | TCEM I | ~~~AGSYT~(SEQ ID NO: 236) | 9 | 0 |

**Table 17 Viral target sequences of interest**

| Based on the above analyses of viral motifs from hepatitis b virus and human papillomavirus, the following sequences are identified as being of relevance to interventions. Amino acids indicated by X may be any amino acid. | | | |
|---|---|---|---|
| Source | 9-mer or 15-mer peptide | TCEM register | Motif |
| *Hepatitis B* | | | |
| Core protein | SEQ ID NO: 46724: VSFGVWIRTPPAYRP | TCEM IIa | SEQ ID NO: 237: VWXRXPP |
| | | TCEM IIb | SEQ ID 238: FXXWXRXPP |

| *Human Papilloma virus* | | | |
|---|---|---|---|
| E6 protein | | TCEM IIa | SEQ ID NO: 239: TCXVXGA |
| | | | SEQ ID NO: 240: YSXSXYG |
| | | | SEQ ID NO: 241: YSXSXYG |
| | | | SEQ ID NO:242 LLXLXLS |
| | | | SEQ ID NO:243 IEXTXVY |
| | | | SEQ ID NO:244 RQXRXQR |
| | | | SEQ ID NO:245 LTXRXTQ |
| | | | SEQ ID NO:246 LEXTXVY |
| | | | SEQ ID NO:247 TDXTXVY |
| | | | SEQ ID NO:248 ADXTXVY |
| | | | SEQ ID NO:249 YCXQXLQ |
| | | TCEM IIb | SEQ ID NO:250 LXXVXRXRS |
| | | | SEQ ID NO:251 SXXVXGXTL |
| | | | SEQ ID NO:252 TXXRXTXAT |
| | | | SEQ ID NO:253 SXXRXYXYY |
| | | | SEQ ID NO:254: YXXTXSXLQ |
| | | | SEQ ID NO:255 KXXFXVXRD |
| | | | SEQ ID NO:256 QXXPXKXPQ |
| | | | SEQ ID NO:257 EXXEXIXEI |
| | | | SEQ ID NO:258 HXXSXRXTG |
| | | | SEQ ID NO: TXXLXMXTL |
| | SEQ ID NO:259 LIDLRLSCV (HPV56) | TCEM I | SEQ ID NO:263 XXKKSLYX |
| | SEQ ID NO:260 IDLRLSCVY (HPV56) | | |
| | SEQ ID NO:261 LLDLRLSCV (HPV 66) | | SEQ ID NO:264 XXVYGASX |
| | SEQ ID NO:262 LDLRLSCVY (HPV 66) | | |
| | | | SEQ ID NO:265 XXLRLSCX |
| | | | SEQ ID NO:266 XXRLSCVX |
| | | | SEQ ID NO:267 XXRNNWPX |
| | | | SEQ ID NO:268 XXYSDSVX |
| | | | SEQ ID NO:269 XXYYSDSX |
| | | | SEQ ID NO:270 XXAGSYTX |
| E7 protein | SEQ ID NO:271 LDLQPETTDLYCYEQ (HPV 16) | TCEM IIa | SEQ ID NO:274 SSXEXED |
| | SEQ ID NO:272 LDLQPEATDLYCYEQ (HPV 31) | | SEQ ID NO:275 PEXTXLY |
| | SEQ ID NO:273 LDLYPEPTDLYCYEQ (HPV 33) | | SEQ ID NO:276 YCXSXDS |
| | SEQ ID NO:277 EARIELVVESSADDL (HPV 18) | TCEM IIb | SEQ ID NO:279 RXXLXVXSS |
| | SEQ ID NO:278 IVTCCYTCGTTVRLC (HPV 58) | | SEQ ID NO:280 LXXLXMXSL |
| | | | SEQ ID NO:281 LXXYXQXPD |
| | | | SEQ ID NO:282 LXXEXTXLY |
| | | | SEQ ID NO:283 SXXKXKXTL |
| | | | SEQ ID NO:284 TXXYXCTT |
| | | TCEM I | SEQ ID NO:285 XXSRDTLX |

### Example 13 Selection of immunogenic epitopes in Ebola virus (EBOV) membrane associated proteins.

In light of the 2014 outbreak of EBOV, we elected to examine the epitopes in the principal proteins of EBOV. Included in our evaluation were the following reference strains of the virus.

**Table 18.**

| Reference strain name | Date of isolation | Representative proteins identifiers/ accession numbers |
|---|---|---|
| Zaire Mayinga | 1976 | Q05320.1 GI:465411 |
| Sudan ebolavirus Boniface | 1976 | Q66814.1 GI:8479515 |
| Musoke Marburg | 1980 | P35253.1 GI:465412 |
| Uganda Bundibugyo | 2007 | YP_003815435.1 GI:302371218 |
| Cote D'Ivoire Tai Forest | 1994 | Q66810.1 GI:8479513 |

Also included in the analysis were the 99 field isolates in 2014 from Guinea and Sierra Leone published by Gire et al (Science. 2014 Sep 12;345(6202):1369-72. doi: 10.1126/science.1259657. Epub 2014 Aug 28. PMID: 25214632). The sequences available from these isolates are deposited at ncbi.nln.nih.gov/bioproject/PRJNA257197 and comprise 891 individual proteins. Many of the proteins of the 2014 isolates were identical having been isolated within a short time span and geographical area. The identical sequences were identified and a single representative of each identity group analyzed. For both 2014 and reference strains particular attention was paid to analysis of the envelope spike glycoprotein GP and the two other membrane associated proteins VP24, and VP40. Analysis of B-cell epitope sequences and MHC I and II binding, cathepsin cleavage and topology was conducted as previously described on all non-identical proteins (see PCT US2011/029192, PCT US2012/055038, and PCT US2014/041523). Analysis of TCEM was then conducted as described elsewhere in the present invention. The TCEM motifs found in the EBOV proteins of interest were categorized by reference to the frequency categories in a database of 56,000 heavy and light chain variable regions.

Peptides were identified which spanned contiguous epitope group regions (CEGs) where high affinity MHC I and MHC II binding peptides overlap and where a B-cell linear epitope occurs within 3-10 amino acids of said MHC binding. These were further analyzed for the presence of potential immunosuppressive peptides which were likely, based on a combination of (a) their high frequency occurrence in the immunoglobulin reference database and (b) their binding at high affinity (≤1 σ below the mean binding affinity for the protein) to multiple MHC alleles. High value peptides were identified by applying the above two criteria. A cross evaluation between the multiple strains of virus was made to determine the degree of conservation of the high value peptides.

A number of T-independent epitopes, comprising B cell binding regions but very little MHC binding, were identified especially in the GP1 mucin like region of the envelope spike glycoprotein (Fields' Virology, Fifth Edition, Volume 1, Chapter 40, 2004). These correspond to sequences previously identified as capable of producing antibody mediated enhancement of virus replication (Takada A, et al. J Infect Dis. 2007 Nov 15;196 Suppl 2:5347-56). Given the absence of MHC binding these epitopes are considered unlikely to be capable of generating a recall memory. These are sequences which are not selected for vaccine or immunogen inclusion.

The identification of both high value target peptides, and those peptides which should be avoided, leads to the ability to design a vaccine based on single, or in a preferred embodiment multiple subunit immunogen peptides. Said immunogen peptides may be expressed singly or as antibody Fc fusions or as fusions to other peptides or polypeptides. The peptides of interest may be further incorporated into a number of different vaccine configurations known to the art including but not limited to virus like particles, DNA, and virus vectored vaccines.

Two strategies were developed for generating antibodies and vaccines to EBOV envelope glycoprotein:
A. Focus on the N terminal of GP1: this is the region 1-375 located to the N terminal side of the mucin like region of GP0 which is also shared by sGP and ssGP, which are secreted and form part of an "antigenic subversion" by binding antibodies (Mohan et al 2012, Plos Path 8(12) e1003065). In this case the focus is on small highly immunogenic epitope dense sequences which avoid potential Tregs. Such proteins may have utility as vaccine components but are unlikely to be good targets for targeting antibody based therapeutics given the epitope overlap with sGP and ssGP.
B. The second strategy is to focus on the GP2 which forms the membrane inserted core and assembles as trimers. This region can be used in a near complete sequence or the most immunogenic epitope dense regions can be expressed individually. One identified potential immunosuppressive motif is located in the peptide with index position 503 and comprising the TCEM IIB motif V~~Q~K~NP (SEQ ID NO: 485) which is a FC6 motif found in 1 in every 64 antibody variable regions. This may be modified to reduce the immunosuppressive effect. As the transmembrane domain may inhibit secretion truncation of the C terminal end facilitates expression.

The TCEM motifs were compared to the database of frequency categories determined by reference to the database of 56,000 immunoglobulin variable regions. Based on this the following potential immunosuppressive or Treg motifs were identified

**Table 19. Envelope glycoprotein**

| Amino acid position | TCEM IIA Frequency category | Motif | TCEM IIB Frequency category | Motif | peptide |
|---|---|---|---|---|---|
| **2014 isolates 98 represented by gi 667853357** | | | | | |
| 363 | 10 | TL~T~ST (SEQ ID NO: 186) | | | SHLTTLATISTSPQP (SEQ ID NO: 287) |
| 391 | 9 | KL~I~EA (SEQ ID NO: 288) | | | TPVYKLDISEATQVG (SEQ ID NO: 289) |
| 209 | 7 | YY~T~IR (SEQ ID NO: 290) | | | PSSGYYSTTIRYQAT (SEQ ID NO: 291) |
| 247 | 5 | QF~L~LN (SEQ ID NO:292) | | | RFTPQFLLQLNETIY (SEQ ID NO:293) |
| 365 | 5 | AT~S~SP (SEQ ID NO: 294) | | | LTTLATISTSPQPPT (SEQ ID NO: 295) |
| 438 | | | 10 | S~~S~D~AT (SEQ ID NO: 296) | SKSADSLDLATTTSP (SEQ ID NO: 297) |
| 217 | | | 9 | R~~A~G~GT (SEQ ID NO: 298) | TIRYQATGFGTNETE (SEQ ID NO: 299) |
| 209 | | | 8 | S~~Y~T~IR (SEQ ID NO: 300) | PSSGYYSTTIRYQAT (SEQ ID NO: 301) |
| 503 | | | 6 | V~~Q~K~NP (SEQ ID NO: 302) | VIVNAQPKCNPNLHY (SEQ ID NO: 303) |

| **EBOV Zaire Mayinga gi 465411** | | | | | |
|---|---|---|---|---|---|
| 363 | 10 | TL~T~ST (SEQ ID NO: 304) | | | SHLTTLATISTSPQS (SEQ ID NO: 305) |
| 391 | 9 | KL~I~EA (SEQ ID NO: 306) | | | TPVYKLDISEATQVE (SEQ ID NO: 307) |
| 209 | 7 | YY~T~IR (SEQ ID NO: 308) | | | PSSGYYSTTIRYQAT (SEQ ID NO: 309) |
| 434 | 6 | SK~T~FL (SEQ ID NO: 310) | | | NTNTSKSTDFLDPAT (SEQ ID NO: 311) |
| 247 | 5 | QF~L~LN (SEQ ID NO: 312) | | | RFTPQFLLQLNETIY (SEQ ID NO: 313) |
| 365 | 5 | AT~S~SP (SEQ ID NO: 314) | | | LTTLATISTSPQSLT (SEQ ID NO: 315) |
| 217 | | | 9 | R~~A~G~GT (SEQ ID NO: 316) | TIRYQATGFGTNETE (SEQ ID NO: 317) |
| 209 | | | 8 | S~~Y~T~IR (SEQ ID NO: 318) | PSSGYYSTTIRYQAT (SEQ ID NO: 319) |
| 503 | | | 6 | V~~Q~K~NP (SEQ ID NO: 320) | AIVNAQPKCNPNLHY (SEQ ID NO:321) |

| **EBOV Sudan Boniface gi 8479515** | | | | | |
|---|---|---|---|---|---|
| 69 | 10 | SG~S~DI (SEQ ID NO: 322) | | | PSQNSTEGRRVDVNT (SEQ ID NO: 323) |
| 363 | 10 | ST~G~RV (SEQ ID NO: 324) | | | NLEGSGVSTDIPSAT (SEQ ID NO: 325) |
| 209 | 9 | YA~S~LE (SEQ ID NO: 326) | | | GLSSSQILSSSPTMA (SEQ ID NO: 327) |
| 404 | 9 | SQ~L~SS (SEQ ID NO: 328) | | | TSSYYATSYLEYEIE (SEQ ID NO: 329) |
| 378 | 4 | TE~T~TI (SEQ ID NO: 330) | | | QETITETTATIIGTN (SEQ ID NO: 331) |
| 375 | | | 10 | T~~T~T~TT (SEQ ID NO: 332) | VNTQETITETTATII (SEQ ID NO: 333) |
| 405 | | | 10 | S~~I~S~SP (SEQ ID NO: 334) | LSSSQILSSSPTMAP (SEQ ID NO: 335) |
| 365 | | | 10 | S~~G~R~DV (SEQ ID NO:336) | QNSTEGRRVDVNTQE (SEQ ID NO: 337) |
| 264 | | | 10 | L~~T~G~LI (SEQ ID NO: 338) | QQLSNTTGKLIWTLD (SEQ ID NO: 339) |
| 88 | | | 9 | S~~P~Q~VS (SEQ ID NO: 340) | FRSGVPPQVVSYEAG (SEQ ID NO: 341) |
| 577 | | | 9 | L~~Y~I~NR (SEQ ID NO: 342) | TELRTYTILNRKAID (SEQ ID NO: 343) |
| 136 | | | 7 | V~~A~G~GP (SEQ ID NO: 344) | RYVHKAQGTGPCPGD (SEQ ID NO: 345) |

From this analysis and examination of the MHC binding and proximity to linear B cell epitopes the following peptides were selected for expression of immunogens from the envelope glycoprotein of Mayinga. It is noted that Seq 503-534 contains a FC6 TCEM IIA motif but that this is absent from the other selected peptides. It is further noted that position 503 contains an A503V mutation in the 2014 isolates, this conservative change is not likely to cause a significant change in recognition. It was further established by reference to cathepsin cleavage probabilities that each peptide contains appropriate excision sites to permit binding. As an initial application is to immunize mice for antibody production, the predicted MHC binding of Balb/c and C57 mice was determined.

### Region A

72 GNGVATDVPSATKRWGFRSGVPPKVVNYEA 101 (SEQ ID NO: 346)
139 HKVSGTGPCAGDFAFHKEGAFFLYDRLASTVI 169 (SEQ ID NO: 347)
263 SGKRSNTTGKLIWKVNPEIDTTIGEWAFWE 292 (SEQ ID NO: 348)

### Region B

503 AIVNAQPKCNPNLHYWTTQDEGAAIGLAWIPY 534 (SEQ ID NO: 349)
518 WTTQDEGAAIGLAWIPYFGPAAEGIYIEGL 546 (SEQ ID NO: 350)
559 RQLANETTQALQLFLRATTELRTFSILNRKA 588 (SEQ ID NO: 351)
636 PDQGDNDNWWTGWRQWIPAG 655 (SEQ ID NO: 352)
505 ....

VNAQPKCNPNLHYWTTQDEGAAIGLAWIPYFGPAAEGIYIEGLMHNQDGLICGLRQLAN
ETTQALQLFLRATTELRTFSILNRKAIDFLLQRWGGTCHILGPDCCIEPHDWTKNITDKIDQIIHDF
VDKTLPDQGDNDNWWTGWRQWIPAG 655 (SEQ ID NO: 353)

Other EBOV membrane proteins were examined using a similar approach. TCEM analysis was conducted as described above for VP24 and VP 40. The potentially immunosuppressive peptides based on TCEMs found with high frequency in heavy and light chain immunoglobulin variable regions are as shown in Tables 13 and 14.

**Table 20. VP40**

| Amino acid position | TCEM IIA Frequency category | Motif | TCEM IIB Frequency category | Motif | Peptide |
|---|---|---|---|---|---|
| **2014 isolates 98 represented by gi 667853356** | | | | | |
| 141 | 10 | HP~R~LR (SEQ ID NO: 354) | | | GIPDHPLRLLRIGNQ (SEQ ID NO: 355) |
| 74 | 8 | MV~V~SG (SEQ ID NO: 356) | | | ILEAMVNVISGPKVL (SEQ ID NO: 357) |
| 222 | 3 | GN~A~LT (SEQ ID NO: 358) | | | SGKKGNSADLTSPEK (SEQ ID NO: 359) |
| 192 | | | 10 | D~~T~S~GA (SEQ ID NO: 360) | TDDTPTGSNGALRPG (SEQ ID NO: 361) |
| 105 | | | 9 | S~~S~T~AI (SEQ ID NO: 362) | TYSFDSTTAAIMLAS (SEQ ID NO: 363) |
| 174 | | | 9 | L~~L~L~TQ (SEQ ID NO: 364) | FDLTALKLITQPLPA (SEQ ID NO: 365) |
| 60 | | | 8 | A~~T~G~VS (SEQ ID NO: 366) | DHASHTPGSVSSAFI (SEQ ID NO: 367) |
| 190 | | | 8 | T~~T~T~SN (SEQ ID NO: 368) | TWTDDTPTGSNGALR (SEQ ID NO: 369) |
| 270 | | | 7 | T~~K~T~KN (SEQ ID NO: 370) | KLTGKKVTSKNGQPI (SEQ ID NO: 371) |
| 222 | | | 4 | K~~N~A~LT (SEQ ID NO: 372) | SGKKGNSADLTSPEK (SEQ ID NO: 373) |

| **EBOV Zaire Mayinga gi 10313993** | | | | | |
|---|---|---|---|---|---|
| 141 | 10 | HP~R~LR (SEQ ID NO: 374) | | | GIPDHPLRLLRIGNQ (SEQ ID NO: 375) |
| 74 | 8 | MV~V~SG (SEQ ID NO: 376) | | | ILEAMVNVISGPKVL (SEQ ID NO: 377) |
| 222 | 3 | GN~A~LT (SEQ ID NO: 378) | | | SGKKGNSADLTSPEK (SEQ ID NO: 379) |
| 192 | | | 10 | D~~T~S~GA (SEQ ID NO: 380) | TDDTPTGSNGALRPG (SEQ ID NO: 381) |
| 105 | | | 9 | S~~S~T~AI (SEQ ID NO: 382) | TYSFDSTTAAIMLAS (SEQ ID NO: 383) |
| 174 | | | 9 | L~~L~L~TQ (SEQ ID NO: 384) | FDLTALKLITQPLPA (SEQ ID NO: 385) |
| 60 | | | 8 | A~~T~G~VS (SEQ ID NO: 386) | DHASHTPGSVSSAFI (SEQ ID NO: 387) |
| 190 | | | 8 | T~~T~T~SN (SEQ ID NO: 388) | TWTDDTPTGSNGALR (SEQ ID NO: 389) |
| 270 | | | 7 | T~~K~T~KN (SEQ ID NO:390) | KLTGKKVTSKNGQPI (SEQ ID NO: 391) |
| 222 | | | 4 | K~~N~A~LT (SEQ ID NO: 392) | SGKKGNSADLTSPEK (SEQ ID NO: 393) |

**Table 21. VP24**

| Amino acid position | TCEM IIA Frequency category | Motif | TCEM IIB Frequency category | Motif | Peptide |
|---|---|---|---|---|---|
| **2014 isolates 98 represented by gi 667853361** | | | | | |
| 8 | 6 | SP~K~LE (SEQ ID NO: 394) | | | YNLISPKKDLEKGVV (SEQ ID NO: 395) |
| 142 | 6 | SL~M~SL (SEQ ID NO: 396) | | | KEQLSLKMLSLIRSN (SEQ ID NO: 397) |

| **EBOV Zaire Mayinga gi 10313998** | | | | | |
|---|---|---|---|---|---|
| 8 | 6 | SP~K~LE (SEQ ID NO: 398) | | | YNLISPKKDLEKGVV (SEQ ID NO: 399) |
| 142 | 6 | SL~M~SL (SEQ ID NO: 400) | | | KEQLSLKMLSLIRSN (SEQ ID NO: 401) |

Following a review of the MHC binding and CEG identification the following peptides were selected from VP40 of gi 667853361 and VP 24 of gi 667853361as immunogens
VP40
   63 SHTPGSVSSAFILEAMVNVISGPKVLMKQIPIWLPLG 99 (SEQ ID NO: 402)
   273 GKKVTSKNGQPIIPVLLPKYIGLDPVAPGDLTMVITQDCDTC 314 (SEQ ID NO: 403)
VP24
   81 PNSTIESPLWALRVILAAGIQDQLID 108 (SEQ ID NO: 404)

A number of strategies were developed for constructing immunogens. These are illustrated in Figure 25.
A. Immunoglobulin Fc carrier, one construct design: human or mouse Fc + hinge region, GP2 or 10 different peptides from the GP, VP24 and VP40 proteins. All peptides are attached to the N-terminus of the hinge region via cleavable linker (Lin10).
B. Immunoglobulin constant region carrier: This is a 2-construct design: *Construct 1:* Make a human or mouse constant heavy chain using the CH(1-3) region with the Ebola protein GP2 (505-655) fused to the N-terminus of the CH(1-3), no linker is used for this design. *Construct 2:* Light chain kappa (CLkappa) constant region with the Ebola protein GP2 (505-655) fused to the N-terminus of CLkappa. The two constructs are co-transduced into CHO expression cells. They should self-assemble similar to full size antibody heavy and light chain.
C. One construct design anti-parallel folding of GP2: human or mouse constant heavy chain using the CH(1-3) region with N-terminal fusion to 3 repeats of the Ebola protein GP2 (505-655). The GP2 repeats are linked together via short linkers (GGGGS (SEQ ID NO: 486)). This design should allow anti-parallel folding of the 3 GP protein repeat onto each other.
D. One construct design to encourage parallel folding of GP2: human or mouse constant heavy chain using the CH(1-3) region with N-terminal fusion to 3 repeats of the Ebola protein GP2 (505-655). The GP2 repeats are linked together via long linker (Lin10). This design should allow parallel folding of the 3 GP2 protein.

The sequences listed are for the following constructs corresponding to the description above.

**Table 22.**

| **Sequence** | **Construct** | **Description** |
|---|---|---|
| SEQ ID NO: 405 | A1 | GP2-Lin10-hFc |
| SEQ ID NO: 406 | A2 | GP2-Lin10-mFc |
| SEQ ID NO: 407 | A3 | GP72-101-Lin10-hFc |
| SEQ ID NO: 408 | A4 | GP72-101-Lin10-mFc |
| SEQ ID NO: 409 | A5 | GP139-169-Lin10-hFc |
| SEQ ID NO: 410 | A6 | GP139-169-Lin10-mFc |
| SEQ ID NO: 411 | A7 | GP263-292-Lin10-hFc |
| SEQ ID NO: 412 | A8 | GP263-292-Lin10-mFc |
| SEQ ID NO: 413 | A9 | GP503-534-Lin10-hFc |
| SEQ ID NO: 414 | A10 | GP503-534-Lin10-mFc |
| SEQ ID NO: 415 | A11 | GP518-546-Lin10-hFc |
| SEQ ID NO: 416 | A12 | GP518-546-Lin10-mFc |
| SEQ ID NO: 417 | A13 | GP559-588-Lin10-hFc |
| SEQ ID NO: 418 | A14 | GP559-588-Lin10-mFc |
| SEQ ID NO: 419 | A15 | GP636-655-Lin10-hFc |
| SEQ ID NO: 420 | A16 | GP636-655-Lin10-mFc |
| SEQ ID NO: 421 | A17 | VP40-63-99-Lin10-hFc |
| SEQ ID NO: 422 | A18 | VP40-63-99-Lin10-mFc |
| SEQ ID NO: 423 | A19 | VP40-273-314-Lin10-hFc |
| SEQ ID NO: 424 | A20 | VP40-273-314-Lin10-mFc |
| SEQ ID NO: 425 | A21 | VP24-81-108-Lin10-hFc |
| SEQ ID NO: 426 | A22 | VP24-81-108--Lin10-mFc |
| SEQ ID NO: 427 | B1 | GP2-hCH1-3 |
| SEQ ID NO: 428 | B2 | GP2-hCL |
| SEQ ID NO: 429 | B3 | GP2-mCH1-3 |
| SEQ ID NO: 430 | B4 | GP2-mCL |
| SEQ ID NO: 431 | C1 | GP2-(G4S)-GP2-(G4S)-GP2-hCH1-3 |
| SEQ ID NO: 432 | C2 | GP2-(G4S)-GP2-(G4S)-GP2-mCH1-3 |
| SEQ ID NO: 433 | D1 | G P2-(Lin 10)-GP2-(Lin 10)-GP2-hCH 1-3 |
| SEQ ID NO: 434 | D2 | GP2-(Lin10)-GP2-(Lin10)-GP2-mCH1-3 |

**SEQ ID NO 405: A1. GP2-Lin10-hFc, amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRVNAQPKCNPNLHYWTTQDEGAAIGLAWIPYF
GPAAEGIYIEGLMHNQDGLICGLRQLANETTQALQLFLRATTELRTFSILNRKA
IDFLLQRWGGTCHILGPDCCIEPHDWTKNITDKIDQIIHDFVDKTLPDQGDNDN
WWTGWRQWIPAGGGGGSGGGGSGGPGSGGGGSGGGGSASTEPKSCDKTHTCPPC
PAPELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKTIS
KAKGQPREPQVYTLPPSREEMTKNQVSLTCLVKGFYPSDIAVEWESNGQPENNY
KTTPPVLDSDGSFFLYSKLTVDKSRWQQGNVFSCSVMHEALHNHYTQKSLSLSP
GK.

| | |
|---|---|
| 1-23 | Signal peptide |
| 24-174 | GP2 |
| 175-199 | Linker Variant 10 |
| 200-434 | hG1(CH2-3) |

**SEQ ID NO 406: Seq.A2. GP2-Lin10-mFc, amino acid sequence,**

. . . . . . . . .○. . . . . . . . .○. . . . . . . . .○. . . . . . . . .○. . . . . . . . .○

METDTLLLWVLLLWVPGSTGDTRVNAQPKCNPNLHYWTTQDEGAAIGLAWIPYF

GPAAEGIYIEGLMHNQDGLICGLRQLANETTQALQLFLRATTELRTFSILNRKA

IDFLLQRWGGTCHILGPDCCIEPHDWTKNITDKIDQIIHDFVDKTLPDQGDNDN

WWTGWRQWIPAGGGGGSGGGGSGGPGSGGGGSGGGGSVDEPRGPTIKPCPPCKC

PAPNLLGGPSVFIFPPKIKDVLMISLSPIVTCVVVDVSEDDPDVQISWFVNNVE

VHTAQTQTHREDYNSTLRVVSALPIQHQDWMSGKEFKCKVNNKDLPAPIERTIS

KPKGSVRAPQVYVLPPPEEEMTKKQVTLTCMVTDFMPEDIYVEWTNNGKTELNY

KNTEPVLDSDGSYFMYSKLRVEKKNWVERNSYSCSVVHEGLHNHHTTKSFSRTP

GK.

| | |
|---|---|
| 1-23 | Signal peptide |
| 24-174 | GP2 |
| 175-199 | Linker Variant 10 |
| 200-434 | mG2a(CH2-3) |

**SEQ ID NO 407: A3. GP(72-101)-Lin10-hFc, amino acid sequence,**

.........○.........○.........○.........○.........○

METDTLLLWVLLLWVPGSTGDTRGNGVATDVPSATKRWGFRSGVPPKVVNYEAG

GGGSGGGGSGGPSGGGGSGGGGSASTEPKSCDKTHTCPPCPAPELLGGPSVFL

FPPKPKDTLMISRTPEVTCVVVDVSHEDPEVKFNWYVDGVEVHNAKTKPREEQY

NSTYRVVSVLTVLHQDWLNGKEYKCKVSNKALPAPIEKTISKAKGQPREPQVYT

LPPSREEMTKNQVSLTCLVKGFYPSDIAVEWESNGQPENNYKTTPPVLDSDGSF

FLYSKLTVDKSRWQQGNVFSCSVMHEALHNHYTQKSLSLSPGK.

| | |
|---|---|
| 1-23 | Signal peptide |
| 24-53 | GP (72-101) |
| 54-78 | Linker Variant 10 |
| 79-313 | hG1(CH2-3) |

**SEQ ID NO 408 : A4. GP(72-101)-Lin10-mFc, amino acid sequence,**

.........o.........o.........o.........o.........o

METDTLLLWVLLLWVPGSTGDTRGNGVATDVPSATKRWGFRSGVPPKVVNYEAG

GGGSGGGGSGGPGSGGGGSGGGGSVDEPRGPTIKPCPPCKCPAPNLLGGPSVFI

FPPKIKDVLMISLSPIVTCVVVDVSEDDPDVQISWFVNNVEVHTAQTQTHREDY

NSTLRVVSALPIQHQDWMSGKEFKCKVNNKDLPAPIERTISKPKGSVRAPQVYV

LPPPEEEMTKKQVTLTCMVTDFMPEDIYVEWTNNGKTELNYKNTEPVLDSDGSY

FMYSKLRVEKKNWVERNSYSCSVVHEGLHNHHTTKSFSRTPGK.

| | |
|---|---|
| 1-23 | Signal peptide |
| 24-53 | GP(72-101) |
| 54-78 | Linker Variant 10 |
| 79-313 | mG2a(CH2-3) |

**SEQ ID NO 409 : A5. GP(139-169)-Lin10-hFc, amino acid sequence,**

.........○.........○.........○.........○.........○

METDTLLLWVLLLWVPGSTGDTRHKVSGTGPCAGDFAFHKEGAFFLYDRLASTV

IGGGGSGGGGSGGPGSGGGGSGGGGSASTEPKSCDKTHTCPPCPAPELLGGPSV

FLFPPKPKDTLMISRTPEVTCVVVDVSHEDPEVKFNWYVDGVEVHNAKTKPREE

QYNSTYRVVSVLTVLHQDWLNGKEYKCKVSNKALPAPIEKTISKAKGQPREPQV

YTLPPSREEMTKNQVSLTCLVKGFYPSDIAVEWESNGQPENNYKTTPPVLDSDG

SFFLYSKLTVDKSRWQQGNVFSCSVMHEALHNHYTQKSLSLSPGK.

| | |
|---|---|
| 1-23 | Signal peptide |
| 24-55 | GP(139-169) |
| 56-80 | Linker Variant 10 |
| 81-315 | hG1(CH2-3) |

**SEQ ID NO 410 : A6. GP(139-169)-Lin10-mFc, amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRHKVSGTGPCAGDFAFHKEGAFFLYDRLASTV

IGGGGSGGGGSGGPGSGGGGSGGGGSVDEPRGPTIKPCPPCKCPAPNLLGGPSV

FIFPPKIKDVLMISLSPIVTCVVVDVSEDDPDVQISWFVNNVEVHTAQTQTHRE

DYNSTLRVVSALPIQHQDWMSGKEFKCKVNNKDLPAPIERTISKPKGSVRAPQV

YVLPPPEEEMTKKQVTLTCMVTDFMPEDIYVEWTNNGKTELNYKNTEPVLDSDG

SYFMYSKLRVEKKNWVERNSYSCSVVHEGLHNHHTTKSFSRTPGK.

| | |
|---|---|
| 1-23 | Signal peptide |
| 24-55 | GP(139-169) |
| 56-80 | Linker Variant 10 |
| 81-315 | mG2a(CH2-3) |

**SEQ ID NO 411: A7. GP(263-292)Lin10-hFc, amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRSGKRSNTTGKLIWKVNPEIDTTIGEWAFWEG
GGGSGGGGSGGPGSGGGGSGGGGSASTEPKSCDKTHTCPPCPAPELLGGPSVFL
FPPKPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQVYT
LPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK.

| | |
|---|---|
| 1-23 | Signal peptide |
| 24-53 | GP(263-292) |
| 54-78 | Linker Variant 10 |
| 79-313 | hG1(CH2-3) |

**SEQ ID NO 412. GP(263-292)-Lin10-mFc, amino acid sequence,**

.........○.........○.........○.........○.........○
METDTLLLWVLLLWVPGSTGDTRSGKRSNTTGKLIWKVNPEIDTTIGEWAFWEG
GGGSGGGGSGGPGSGGGGSGGGGSVDEPRGPTIKPCPPCKCPAPNLLGGPSVFI
FPPKIKDVLMISLSPIVTCVVVDVSEDDPDVQISWFVNNVEVHTAQTQTHREDY
NSTLRVVSALPIQHQDWMSGKEFKCKVNNKDLPAPIERTISKPKGSVRAPQVYV
LPPPEEEMTKKQVTLTCMVTDFMPEDIYVEWTNNGKTELNYKNTEPVLDSDGSY
FMYSKLRVEKKNWVERNSYSCSVVHEGLHNHHTTKSFSRTPGK.

| | |
|---|---|
| 1-23 | Signal peptide |
| 24-53 | GP(263-292) |
| 54-78 | Linker Variant 10 |
| 79-313 | mG2a(CH2-3) |

**SEQ ID NO 413. GP(503-534)-Lin10-hFc, amino acid sequence,**

.........○.........○.........○.........○.........○

METDTLLLWVLLLWVPGSTGDTRAIVNAQPKCNPNLHYWTTQDEGAAIGLAWIP

YGGGGSGGGGSGGPGSGGGGSGGGGSASTEPKSCDKTHTCPPCPAPELLGGPSV

FLFPPKPKDTLMISRTPEVTCVVVDVSHEDPEVKFNWYVDGVEVHNAKTKPREE

QYNSTYRVVSVLTVLHQDWLNGKEYKCKVSNKALPAPIEKTISKAKGQPREPQV

YTLPPSREEMTKNQVSLTCLVKGFYPSDIAVEWESNGQPENNYKTTPPVLDSDG

SFFLYSKLTVDKSRWQQGNVFSCSVMHEALHNHYTQKSLSLSPGK.

| | |
|---|---|
| 1-23 | Signal peptide |
| 24-55 | GP (503-534) |
| 56-80 | Linker Variant 10 |
| 81-315 | hG1(CH2-3) |

**SEQ ID NO 414 :A10. GP(503-534)-Lin10-mFc, amino acid sequence,**

.........o.........o.........o.........o.........o

METDTLLLWVLLLWVPGSTGDTRAIVNAQPKCNPNLHYWTTQDEGAAIGLAWIP

YGGGGSGGGGSGGPGSGGGGSGGGGSVDEPRGPTIKPCPPCKCPAPNLLGGPSV

FIFPPKIKDVLMISLSPIVTCVVVDVSEDDPDVQISWFVNNVEVHTAQTQTHRE

DYNSTLRVVSALPIQHQDWMSGKEFKCKVNNKDLPAPIERTISKPKGSVRAPQV

YVLPPPEEEMTKKQVTLTCMVTDFMPEDIYVEWTNNGKTELNYKNTEPVLDSDG

SYFMYSKLRVEKKNWVERNSYSCSVVHEGLHNHHTTKSFSRTPGK.

| | |
|---|---|
| 1-23 | Signal peptide |
| 24-55 | GP(503-534) |
| 56-80 | Linker Variant 10 |
| 81-315 | mG2a(CH2-3) |

**SEQ ID NO 415 A11. GP(518-546)-Lin10-hFc, amino acid sequence,**

.........○.........○.........○.........○.........○

METDTLLLWVLLLWVPGSTGDTRWTTQDEGAAIGLAWIPYFGPAAEGIYIEGLG

GGGSGGGGSGGPGSGGGGSGGGGSASTEPKSCDKTHTCPPCPAPELLGGPSVFL

FPPKPKDTLMISRTPEVTCVVVDVSHEDPEVKFNWYVDGVEVHNAKTKPREEQY

NSTYRVVSVLTVLHQDWLNGKEYKCKVSNKALPAPIEKTISKAKGQPREPQVYT

LPPSREEMTKNQVSLTCLVKGFYPSDIAVEWESNGQPENNYKTTPPVLDSDGSF

FLYSKLTVDKSRWQQGNVFSCSVMHEALHNHYTQKSLSLSPGK.

| | |
|---|---|
| 1-23 | Signal peptide |
| 24-53 | GP(518-546) |
| 54-78 | Linker Variant 10 |
| 79-313 | hG1(CH2-3) |

**SEQ ID NO 416 A12. GP(518-546)-Lin10-mFc, amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRWTTQDEGAAIGLAWIPYFGPAAEGIYIEGLG
GGGSGGGGSGGPGSGGGGSGGGGSVDEPRGPTIKPCPPCKCPAPNLLGGPSVFI
FPPKIKDVLMISLSPIVTCVVVDVSEDDPDVQISWFVNNVEVHTAQTQTHREDY
NSTLRVVSALPIQHQDWMSGKEFKCKVNNKDLPAPIERTISKPKGSVRAPQVYV
LPPPEEEMTKKQVTLTCMVTDFMPEDIYVEWTNNGKTELNYKNTEPVLDSDGSY
FMYSKLRVEKKNWVERNSYSCSVVHEGLHNHHTTKSFSRTPGK.

| | |
|---|---|
| 1-23 | Signal peptide |
| 24-53 | GP(518-546) |
| 54-78 | Linker Variant 10 |
| 79-313 | mG2a(CH2-3) |

**SEQ ID NO 417 A13. GP(559-588)-Lin10-hFc, amino acid sequence,**

.........o.........o.........o.........o.........o

METDTLLLWVLLLWVPGSTGDTRRQLANETTQALQLFLRATTELRTFSILNRKA

GGGGSGGGGSGGPGSGGGGSGGGGSASTEPKSCDKTHTCPPCPAPELLGGPSVF

LFPPKPKDTLMISRTPEVTCVVVDVSHEDPEVKFNWYVDGVEVHNAKTKPREEQ

YNSTYRVVSVLTVLHQDWLNGKEYKCKVSNKALPAPIEKTISKAKGQPREPQVY

TLPPSREEMTKNQVSLTCLVKGFYPSDIAVEWESNGQPENNYKTTPPVLDSDGS

FFLYSKLTVDKSRWQQGNVFSCSVMHEALHNHYTQKSLSLSPGK.

| | |
|---|---|
| 1-23 | Signal peptide |
| 24-54 | GP(559-588) |
| 55-79 | Linker Variant 10 |
| 80-314 | hG1(CH2-3) |

**SEQ ID NO 418 A14. GP(559-588)-Lin10-mFc, amino acid sequence,**

.........○.........○.........○.........○.........○

METDTLLLWVLLLWVPGSTGDTRRQLANETTQALQLFLRATTELRTFSILNRKA

GGGGSGGGGSGGPGSGGGGSGGGGSVDEPRGPTIKPCPPCKCPAPNLLGGPSVF

IFPPKIKDVLMISLSPIVTCVVVDVSEDDPDVQISWFVNNVEVHTAQTQTHRED

YNSTLRVVSALPIQHQDWMSGKEFKCKVNNKDLPAPIERTISKPKGSVRAPQVY

VLPPPEEEMTKKQVTLTCMVTDFMPEDIYVEWTNNGKTELNYKNTEPVLDSDGS

YFMYSKLRVEKKNWVERNSYSCSVVHEGLHNHHTTKSFSRTPGK.

| | |
|---|---|
| 1-23 | Signal peptide |
| 24-54 | GP(559-588) |
| 55-79 | Linker Variant 10 |
| 80-314 | mG2a(CH2-3) |

**SEQ ID NO 419 A15. GP(636-655)-Lin10-hFc, amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRPDQGDNDNWWTGWRQWIPAGGGGGSGGGGSG

GPGSGGGGSGGGGSASTEPKSCDKTHTCPPCPAPELLGGPSVFLFPPKPKDTLM

ISRTPEVTCVVVDVSHEDPEVKFNWYVDGVEVHNAKTKPREEQYNSTYRVVSVL

TVLHQDWLNGKEYKCKVSNKALPAPIEKTISKAKGQPREPQVYTLPPSREEMTK

NQVSLTCLVKGFYPSDIAVEWESNGQPENNYKTTPPVLDSDGSFFLYSKLTVDK

SRWQQGNVFSCSVMHEALHNHYTQKSLSLSPGK.

| | |
|---|---|
| 1-23 | Signal peptide |
| 24-43 | GP(636-655) |
| 44-68 | Linker Variant 10 |
| 69-303 | hG1(CH2-3) |

**SEQ ID NO 420 A16. GP(636-655)-Lin10-mFc, amino acid sequence,**

. . . . . . . . . o . . . . . . . . . o . . . . . . . . . o . . . . . . . . . o . . . . . . . . . o

METDTLLLWVLLLWVPGSTGDTRPDQGDNDNWWTGWRQWIPAGGGGGSGGGGSG

GPGSGGGGSGGGGSVDEPRGPTIKPCPPCKCPAPNLLGGPSVFIFPPKIKDVLM

ISLSPIVTCVVVDVSEDDPDVQISWFVNNVEVHTAQTQTHREDYNSTLRVVSAL

PIQHQDWMSGKEFKCKVNNKDLPAPIERTISKPKGSVRAPQVYVLPPPEEEMTK

KQVTLTCMVTDFMPEDIYVEWTNNGKTELNYKNTEPVLDSDGSYFMYSKLRVEK

KNWVERNSYSCSVVHEGLHNHHTTKSFSRTPGK.

| | |
|---|---|
| 1-23 | Signal peptide |
| 24-43 | GP(636-655) |
| 44-68 | Linker Variant 10 |
| 69-303 | mG2a(CH2-3) |

**SEQ ID NO 421 A17. VP40 (63-99) -Lin10-hFc, amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRSHTPGSVSSAFILEAMVNVISGPKVLMKQIP
IWLPLGGGGGSGGGGSGGPGSGGGGSGGGGSASTEPKSCDKTHTCPPCPAPELL
GGPSVFLFPPKPKDTLMISRTPEVTCVVVDVSHEDPEVKFNWYVDGVEVHNAKT
KPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPV
LDSDGSFFLYSKLTVDKSRWQQGNVFSCSVMHEALHNHYTQKSLSLSPGK.

| | |
|---|---|
| 1-23 | Signal peptide |
| 24-60 | VP40(63-99) |
| 61-85 | Linker Variant 10 |
| 86-320 | hG1(CH2-3) |

**SEQ ID NO 422 A18. VP40(63-99)-Lin10-mFc, amino acid sequence,**

.........○.........○.........○.........○.........○
METDTLLLWVLLLWVPGSTGDTRSHTPGSVSSAFILEAMVNVISGPKVLMKQIP
IWLPLGGGGGSGGGGSGGPGSGGGGSGGGGSVDEPRGPTIKPCPPCKCPAPNLL
GGPSVFIFPPKIKDVLMISLSPIVTCVVVDVSEDDPDVQISWFVNNVEVHTAQT
QTHREDYNSTLRVVSALPIQHQDWMSGKEFKCKVNNKDLPAPIERTISKPKGSV
RAPQVYVLPPPEEEMTKKQVTLTCMVTDFMPEDIYVEWTNNGKTELNYKNTEPV
LDSDGSYFMYSKLRVEKKNWVERNSYSCSVVHEGLHNHHTTKSFSRTPGK.

| | |
|---|---|
| 1-23 | Signal peptide |
| 24-60 | VP40(63-99) |
| 61-85 | Linker Variant 10 |
| 86-320 | mG2a(CH2-3) |

**SEQ ID NO 423 A19. VP40(273-314)-Lin10-hFc, amino acid sequence,**

.........o.........o.........o.........o.........o
METDTLLLWVLLLWVPGSTGDTRGKKVTSKNGQPIIPVLLPKYIGLDPVAPGDL
TMVITQDCDTCGGGGSGGGGSGGPGSGGGGSGGGGSASTEPKSCDKTHTCPPCP
APELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISK
AKGQPREPQVYTLPPSREEMTKNQVSLTCLVKGFYPSDIAVEWESNGQPENNYK
TTPPVLDSDGSFFLYSKLTVDKSRWQQGNVFSCSVMHEALHNHYTQKSLSLSPG
K.

| | |
|---|---|
| 1-23 | Signal peptide |
| 24-65 | VP40(273-314) |
| 66-90 | Linker Variant 10 |
| 91-325 | hG1(CH2-3) |

**SEQ ID NO 424 A20. VP40(273-314)-Lin10-mFc, amino acid sequence,**

........○.........○.........○.........○.........○
METDTLLLWVLLLWVPGSTGDTRGKKVTSKNGQPIIPVLLPKYIGLDPVAPGDL
TMVITQDCDTCGGGGSGGGGSGGPGSGGGGSGGGGSVDEPRGPTIKPCPPCKCP
APNLLGGPSVFIFPPKIKDVLMISLSPIVTCVVVDVSEDDPDVQISWFVNNVEV
HTAQTQTHREDYNSTLRVVSALPIQHQDWMSGKEFKCKVNNKDLPAPIERTISK
PKGSVRAPQVYVLPPPEEEMTKKQVTLTCMVTDFMPEDIYVEWTNNGKTELNYK
NTEPVLDSDGSYFMYSKLRVEKKNWVERNSYSCSVVHEGLHNHHTTKSFSRTPG
K.

| | |
|---|---|
| 1-23 | Signal peptide |
| 24-65 | VP40(273-314) |
| 66-90 | Linker Variant 10 |
| 91-325 | mG2a(CH2-3) |

**SEQ ID NO 425 A21. VP24 (81-108) -Lin10-hFc, amino acid sequence,**

........○.........○.........○.........○.........○
METDTLLLWVLLLWVPGSTGDTRPNSTIESPLWALRVILAAGIQDQLIDGGGGS
GGGGSGGPGSGGGGSGGGGSASTEPKSCDKTHTCPPCPAPELLGGPSVFLFPPK
PKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYS
KLTVDKSRWQQGNVFSCSVMHEALHNHYTQKSLSLSPGK.

| | |
|---|---|
| 1-23 | Signal peptide |
| 24-49 | VP24(81-108) |
| 50-74 | Linker Variant 10 |
| 75-309 | hG1(CH2-3) |

**SEQ ID NO 426 A22. VP24(81-108)-Lin10-mFc, amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRPNSTIESPLWALRVILAAGIQDQLIDGGGGS
GGGGSGGPGSGGGGSGGGGSVDEPRGPTIKPCPPCKCPAPNLLGGPSVFIFPPK
IKDVLMISLSPIVTCVVVDVSEDDPDVQISWFVNNVEVHTAQTQTHREDYNSTL
RVVSALPIQHQDWMSGKEFKCKVNNKDLPAPIERTISKPKGSVRAPQVYVLPPP
EEEMTKKQVTLTCMVTDFMPEDIYVEWTNNGKTELNYKNTEPVLDSDGSYFMYS
KLRVEKKNWVERNSYSCSVVHEGLHNHHTTKSFSRTPGK.

| | |
|---|---|
| 1-23 | Signal peptide |
| 24-49 | VP24(81-108) |
| 50-74 | Linker Variant 10 |
| 75-309 | mG2a(CH2-3) |

**SEQ ID NO 427 B1. GP2-h(CH1-3), amino acid sequence,**

. . . . . . . . .○. . . . . . . . .○. . . . . . . . .○. . . . . . . . .○. . . . . . . . .○

METDTLLLWVLLLWVPGSTGDTRVNAQPKCNPNLHYWTTQDEGAAIGLAWIPYF

GPAAEGIYIEGLMHNQDGLICGLRQLANETTQALQLFLRATTELRTFSILNRKA

IDFLLQRWGGTCHILGPDCCIEPHDWTKNITDKIDQIIHDFVDKTLPDQGDNDN

WWTGWRQWIPAGASTKGPSVFPLAPSSKSTSGGTAALGCLVKDYFPEPVTVSWN

SGALTSGVHTFPAVLQSSGLYSLSSVVTVPSSSLGTQTYICNVNHKPSNTKVDK

RVEPKSCDKTHTCPPCPAPELLGGPSVFLFPPKPKDTLMISRTPEVTCVVVDVS

HEDPEVKFNWYVDGVEVHNAKTKPREEQYNSTYRVVSVLTVLHQDWLNGKEYKC

KVSNKALPAPIEKTISKAKGQPREPQVYTLPPSREEMTKNQVSLTCLVKGFYPS

DIAVEWESNGQPENNYKTTPPVLDSDGSFFLYSKLTVDKSRWQQGNVFSCSVMH

EALHNHYTQKSLSLSPGK.

| | |
|---|---|
| 1-23 | Signal peptide |
| 24-174 | GP2 |
| 175-504 | hG1(CH1-3) |

**SEQ ID NO 428 B2. GP2-hCL, amino acid sequence,**

.........o.........o.........o.........o.........o

METDTLLLWVLLLWVPGSTGDTRVNAQPKCNPNLHYWTTQDEGAAIGLAWIPYF

GPAAEGIYIEGLMHNQDGLICGLRQLANETTQALQLFLRATTELRTFSILNRKA

IDFLLQRWGGTCHILGPDCCIEPHDWTKNITDKIDQIIHDFVDKTLPDQGDNDN

WWTGWRQWIPAGTVAAPSVFIFPPSDEQLKSGTASVVCLLNNFYPREAKVQWKV

DNALQSGNSQESVTEQDSKDSTYSLSSTLTLSKADYEKHKVYACEVTHQGLSSP

VTKSFNRGEC.

| | |
|---|---|
| 1-23 | Signal peptide |
| 24-174 | GP2 |
| 175-280 | human constant light region |

**SEQ ID NO 429 B3. GP2-m(CH1-3), amino acid sequence,**

.........o.........o.........o.........o.........o

METDTLLLWVLLLWVPGSTGDTRVNAQPKCNPNLHYWTTQDEGAAIGLAWIPYF

GPAAEGIYIEGLMHNQDGLICGLRQLANETTQALQLFLRATTELRTFSILNRKA

IDFLLQRWGGTCHILGPDCCIEPHDWTKNITDKIDQIIHDFVDKTLPDQGDNDN

WWTGWRQWIPAGAKTTAPSVYPLAPVCGDTTGSSVTLGCLVKGYFPEPVTLTWN

SGSLSSGVHTFPAVLQSDLYTLSSSVTVTSSTWPSQSITCNVAHPASSTKVDKK

IEPRGPTIKPCPPCKCPAPNLLGGPSVFIFPPKIKDVLMISLSPIVTCVVVDVS

EDDPDVQISWFVNNVEVHTAQTQTHREDYNSTLRVVSALPIQHQDWMSGKEFKC

KVNNKDLPAPIERTISKPKGSVRAPQVYVLPPPEEEMTKKQVTLTCMVTDFMPE

DIYVEWTNNGKTELNYKNTEPVLDSDGSYFMYSKLRVEKKNWVERNSYSCSVVH

EGLHNHHTTKSFSRTPGK.

| | |
|---|---|
| 1-23 | Signal peptide |
| 24-174 | GP2 |
| 175-504 | mG2a(CH1-3) |

**SEQ ID NO 430 B4. GP2-mCL, amino acid sequence,**

.........○.........○.........○.........○.........○

METDTLLLWVLLLWVPGSTGDTRVNAQPKCNPNLHYWTTQDEGAAIGLAWIPYF

GPAAEGIYIEGLMHNQDGLICGLRQLANETTQALQLFLRATTELRTFSILNRKA

IDFLLQRWGGTCHILGPDCCIEPHDWTKNITDKIDQIIHDFVDKTLPDQGDNDN

WWTGWRQWIPAGKRADAAPTVSIFPPSSEQLTSGGASVVCFLNNFYPKDINVKW

KIDGSERQNGVLNSWTDQDSKDSTYSMSSTLTLTKDEYERHNSYTCEATHKTST

SPIVKSFNRNEC.

| | |
|---|---|
| 1-23 | Signal peptide |
| 24-174 | GP2 |
| 175-282 | murine constant light region |

**SEQ ID NO 431 C1. GP2-(G4S)-GP2-(G4S)-GP2-h(CH1-3), amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRVNAQPKCNPNLHYWTTQDEGAAIGLAWIPYF

GPAAEGIYIEGLMHNQDGLICGLRQLANETTQALQLFLRATTELRTFSILNRKA

IDFLLQRWGGTCHILGPDCCIEPHDWTKNITDKIDQIIHDFVDKTLPDQGDNDN

WWTGWRQWIPAGGGGGSVNAQPKCNPNLHYWTTQDEGAAIGLAWIPYFGPAAEG

IYIEGLMHNQDGLICGLRQLANETTQALQLFLRATTELRTFSILNRKAIDFLLQ

RWGGTCHILGPDCCIEPHDWTKNITDKIDQIIHDFVDKTLPDQGDNDNWWTGWR

QWIPAGGGGGSVNAQPKCNPNLHYWTTQDEGAAIGLAWIPYFGPAAEGIYIEGL

MHNQDGLICGLRQLANETTQALQLFLRATTELRTFSILNRKAIDFLLQRWGGTC
HILGPDCCIEPHDWTKNITDKIDQIIHDFVDKTLPDQGDNDNWWTGWRQWIPAG
ASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHT
CPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSL
SLSPGK.

| | |
|---|---|
| 1-23 | Signal peptide |
| 24-174 | GP2 |
| 175-179 | G4S Linker |
| 180-330 | GP2 |
| 331-335 | G4S Linker |
| 336-486 | GP2 |
| 487-816 | hG1(CH1-3) |

**SEQ ID NO 432 C2. GP2-(G4S)-GP2-(G4S)-GP2-m(CH1-3), amino acid sequence,**

. . . . . . . . .○. . . . . . . . .○. . . . . . . . .○. . . . . . . . .○. . . . . . . . .○
METDTLLLWVLLLWVPGSTGDTRVNAQPKCNPNLHYWTTQDEGAAIGLAWIPYF
GPAAEGIYIEGLMHNQDGLICGLRQLANETTQALQLFLRATTELRTFSILNRKA
IDFLLQRWGGTCHILGPDCCIEPHDWTKNITDKIDQIIHDFVDKTLPDQGDNDN
WWTGWRQWIPAGGGGGSVNAQPKCNPNLHYWTTQDEGAAIGLAWIPYFGPAAEG
IYIEGLMHNQDGLICGLRQLANETTQALQLFLRATTELRTFSILNRKAIDFLLQ
RWGGTCHILGPDCCIEPHDWTKNITDKIDQIIHDFVDKTLPDQGDNDNWWTGWR
QWIPAGGGGGSVNAQPKCNPNLHYWTTQDEGAAIGLAWIPYFGPAAEGIYIEGL
MHNQDGLICGLRQLANETTQALQLFLRATTELRTFSILNRKAIDFLLQRWGGTC
HILGPDCCIEPHDWTKNITDKIDQIIHDFVDKTLPDQGDNDNWWTGWRQWIPAG
AKTTAPSVYPLAPVCGDTTGSSVTLGCLVKGYFPEPVTLTWNSGSLSSGVHTFP
AVLQSDLYTLSSSVTVTSSTWPSQSITCNVAHPASSTKVDKKIEPRGPTIKPCP
PCKCPAPNLLGGPSVFIFPPKIKDVLMISLSPIVTCVVVDVSEDDPDVQISWFV
NNVEVHTAQTQTHREDYNSTLRVVSALPIQHQDWMSGKEFKCKVNNKDLPAPIE
RTISKPKGSVRAPQVYVLPPPEEEMTKKQVTLTCMVTDFMPEDIYVEWTNNGKT
ELNYKNTEPVLDSDGSYFMYSKLRVEKKNWVERNSYSCSVVHEGLHNHHTTKSF
SRTPGK.

| | |
|---|---|
| 1-23 | Signal peptide |
| 24-174 | GP2 |
| 175-179 | G4S Linker |
| 180-330 | GP2 |
| 331-335 | G4S Linker |
| 336-486 | GP2 |
| 487-816 | mG2a(CH1-3) |

**SEQ ID NO 433 D1. GP2-(Lin10)-GP2-(Lin10)-GP2-h(CH1-3), amino acid sequence,**

. . . . . . . . . ○ . . . . . . . . . ○ . . . . . . . . . ○ . . . . . . . . . ○ . . . . . . . . . ○

METDTLLLWVLLLWVPGSTGDTRVNAQPKCNPNLHYWTTQDEGAAIGLAWIPYF

GPAAEGIYIEGLMHNQDGLICGLRQLANETTQALQLFLRATTELRTFSILNRKA

IDFLLQRWGGTCHILGPDCCIEPHDWTKNITDKIDQIIHDFVDKTLPDQGDNDN

WWTGWRQWIPAGGGGGSGGGGSGGPGSGGGGSGGGGSVNAQPKCNPNLHYWTTQ

DEGAAIGLAWIPYFGPAAEGIYIEGLMHNQDGLICGLRQLANETTQALQLFLRA

TTELRTFSILNRKAIDFLLQRWGGTCHILGPDCCIEPHDWTKNITDKIDQIIHD

FVDKTLPDQGDNDNWWTGWRQWIPAGGGGGSGGGGSGGPGSGGGGSGGGGSVNA

QPKCNPNLHYWTTQDEGAAIGLAWIPYFGPAAEGIYIEGLMHNQDGLICGLRQL

ANETTQALQLFLRATTELRTFSILNRKAIDFLLQRWGGTCHILGPDCCIEPHDW

TKNITDKIDQIIHDFVDKTLPDQGDNDNWWTGWRQWIPAGASTKGPSVFPLAPS

SKSTSGGTAALGCLVKDYFPEPVTVSWNSGALTSGVHTFPAVLQSSGLYSLSSV

VTVPSSSLGTQTYICNVNHKPSNTKVDKRVEPKSCDKTHTCPPCPAPELLGGPS

VFLFPPKPKDTLMISRTPEVTCVVVDVSHEDPEVKFNWYVDGVEVHNAKTKPRE

EQYNSTYRVVSVLTVLHQDWLNGKEYKCKVSNKALPAPIEKTISKAKGQPREPQ

VYTLPPSREEMTKNQVSLTCLVKGFYPSDIAVEWESNGQPENNYKTTPPVLDSD

GSFFLYSKLTVDKSRWQQGNVFSCSVMHEALHNHYTQKSLSLSPGK.

| | |
|---|---|
| 1-23 | Signal peptide |
| 24-174 | GP2 |
| 175-199 | Linker Variant 10 |
| 200-350 | GP2 |
| 351-375 | Linker Variant 10 |
| 376-526 | GP2 |
| 527-856 | hG1(CH1-3) |

**SEQ ID NO: 434 D2. GP2-(Lin10)-GP2-(Lin10)-GP2-m(CH1-3), amino acid sequence,**

.........o.........o.........o.........o.........o

METDTLLLWVLLLWVPGSTGDTRVNAQPKCNPNLHYWTTQDEGAAIGLAWIPYF

GPAAEGIYIEGLMHNQDGLICGLRQLANETTQALQLFLRATTELRTFSILNRKA

IDFLLQRWGGTCHILGPDCCIEPHDWTKNITDKIDQIIHDFVDKTLPDQGDNDN

WWTGWRQWIPAGGGGGSGGGGSGGPGSGGGGSGGGGSVNAQPKCNPNLHYWTTQ
DEGAAIGLAWIPYFGPAAEGIYIEGLMHNQDGLICGLRQLANETTQALQLFLRA
TTELRTFSILNRKAIDFLLQRWGGTCHILGPDCCIEPHDWTKNITDKIDQIIHD
FVDKTLPDQGDNDNWWTGWRQWIPAGGGGGSGGGGSGGPGSGGGGSGGGGSVNA
QPKCNPNLHYWTTQDEGAAIGLAWIPYFGPAAEGIYIEGLMHNQDGLICGLRQL
ANETTQALQLFLRATTELRTFSILNRKAIDFLLQRWGGTCHILGPDCCIEPHDW
TKNITDKIDQIIHDFVDKTLPDQGDNDNWWTGWRQWIPAGAKTTAPSVYPLAPV
CGDTTGSSVTLGCLVKGYFPEPVTLTWNSGSLSSGVHTFPAVLQSDLYTLSSSV
TVTSSTWPSQSITCNVAHPASSTKVDKKIEPRGPTIKPCPPCKCPAPNLLGGPS
VFIFPPKIKDVLMISLSPIVTCVVVDVSEDDPDVQISWFVNNVEVHTAQTQTHR
EDYNSTLRVVSALPIQHQDWMSGKEFKCKVNNKDLPAPIERTISKPKGSVRAPQ
VYVLPPPEEEMTKKQVTLTCMVTDFMPEDIYVEWTNNGKTELNYKNTEPVLDSD
GSYFMYSKLRVEKKNWVERNSYSCSVVHEGLHNHHTTKSFSRTPGK.

| | |
|---|---|
| 1-23 | Signal peptide |
| 24-174 | GP2 |
| 175-199 | Linker Variant 10 |
| 200-350 | GP2 |
| 351-375 | Linker Variant 10 |
| 376-526 | GP2 |
| 527-856 | mG2a(CH1-3) |

**SEQ ID NO:435**
Linker Variant 10 
GGTGGTGGCGGTTCAGGCGGAGGTGGCTCTGGCGGTCCCGGATCCGGCGGAGGT
GGCTCTGGCGGAGGAGGGTCT
**SEQ ID NO:436**
Linker Variant 10
GGGGSGGGGSGGPGSGGGGSGGGGS

The constructs described above are expressed in CHO cells. This is accomplished using a retrovector based gene transfer system described elsewhere (see, e.g., US Pat. No. 6,852,510).

In one application of the immunogens thus designed they are incorporated into a vaccine and for this purpose comprise immunoglobulin components. In yet another specific application the Fc fusion is constructed using a murine Fc. The fusion protein is then used to immunize mouse for the reparation of hybridomas. This follows protocols well known to the art (Kohler and Milstein, Nature, 1975, 256(5517):495-7). Immunoglobulin variable regions are extracted from the hybridomas as previously described (see, e.g., US Pat. No. 8,703,134) and constructed with human Fc and transfected into CHO cells, or other mammalian cell lines, using a retrovector system as previously described. The resultant recombinant immunoglobulin is evaluated for its neutralizing capability may be used as an immunotherapeutic. In a further embodiment the recombinant antibody is expressed as a fusion with a microbiocide which is virucidal for enveloped viruses. The specific antibody binding targets the virucide to conserved epitopes on the surface of the virus. Specific virucides which may be incorporated include, but are not limited to, cathelicidins, defensins, and phospholipase A2 (see, e.g., Bezzine, et al 2002. J Biol.Chem. 277:48523-48534; Bezzine et al. 2000. J Biol.Chem. 275:3179-3191; Buck et al 2006. Proc.Natl.Acad.Sci.U.S.A 103:1516-1521; Daher et al. J.Virol. 60:1068-1074; Klotman, M. E. and T. L. Chang. 2006. Nat.Rev.Immunol. 6:447-456; Koduri et al 2002. J.Biol.Chem. 277:5849-5857; Ganz, T. 2004. J Leukoc.Biol. 75:34-38.).

### Example 14 Design of optimized peptides

The ability to specifically identify probable Treg motifs in HPV, or indeed in other oncoproteins or tumor associated antigens of interest, opens the way to developing immunotherapies targeted to such pMHC complexes, or developing diagnostic tools to track the appearance of Tregs binding such pMHC. The TCR recognizes the exposed motif (TCEM) in the context of the MHC molecule of the patient's allele. To develop diagnostic or therapeutic reagents, it may be beneficial to select TCEM combined with intercalated GEM motifs which provide high binding affinity and hence a stable MHC-peptide relationship.

As an example we therefore generated *in silico* high binding affinity peptides comprising two of the conserved high frequency motifs in Alpha 9 species HPV E7, PE~T~LY (SEQ ID NO: 487) and the overlapping L~~E~T~LY (SEQ ID NO: 488). We selected two of the highest binding alleles for these two motifs, Dr1*09:01 and DR1*01:01 and two lower binders DQA1*0102:DQB1*0602 and DQA1*0101:DQB1*0501 and generated peptides which would have at least ten fold higher affinity. Monte Carlo simulation was used to generate 50,000 unique peptides with modified GEM sequences, while holding the TCEM sequence constant across two frames, a hexamer corresponding to the -1,2,3,5,7,8 positions in the naturally occurring 13-mer with index position at amino acid 13 (in HPV33 reference strain gi 218931424). The 50,000 peptides generated *in silico* were predicted to have a range of affinities, as shown in Figure 26 with mean In(ic50) at 7-9. For each allele of interest up to ten top binding peptides were selected, as shown in Table 10. As shown in Figure 27, the predicted affinity of the top peptides created for each allele was In(ic50) 0.08 to 5.87, depending on the allele. The selected peptides have a higher affinity and therefore a higher residence time in the binding groove as compared to the original peptide while keeping the same amino acid side chains exposed for T-cell recognition and binding.

These are illustrative examples and other different peptides with similar binding properties could be derived by applying this approach for the same or other predicted Treg TCEM motifs, and are thus embodied in this description. The examples are not considered limiting.

**Table 23. Example of high affinity peptides for specific alleles which will present HPV E7 TCEM motifs PE~T~LY (SEQ ID NO: 487)and L~~E~T~LY (SEQ ID NO: 488). The TCEM motifs are boldfaced and underlined in the first line of each group. Random generation results in some repeated peptides.**

| DRB1*01:01 Hi 10 | DRB1*09:01 Hi 10 |
|---|---|
| RW**L**Y**PE**S**T**V**LY**FTFH **(SEQ ID NO:437)** | LF**L**M**PE**M**T**F**LY**GAII **(SEQ ID NO: 438)** |
| DDLWPELTSLYVVFT(SEQ **ID NO:439)** | IQLLPEGTVLYVCPT **(SEQ ID NO: 440)** |
| MSLMPELTGLYISYV **(SEQ ID NO: 441)** | LLLFPEATNLYTIEK **(SEQ ID NO: 442)** |
| MSLMPELTGLYISYV **(SEQ ID NO: 441)** | FVLFPEATKLYLHGV **(SEQ ID NO: 444)** |
| KRLYPESTLLYVSLC **(SEQ ID NO:445)** | LELLPEGTLLYVCPI **(SEQ ID NO: 446)** |
| RWLYPESTVLYFTFH **(SEQ ID NO: 447)** | LELLPEGTLLYVCPI **(SEQ ID NO: 446)** |
| RWLYPESTVLYFTFH **(SEQ ID NO: 447)** | LQLVPEGTALYLYEL **(SEQ ID NO:448)** |
| FWLQPEYTALYAMLD **(SEQ ID NO: 449)** | LQLMPEGTLLYVCPG **(SEQ ID NO: 450)** |
| QMLLPELTVLYPGCA **(SEQ ID NO: 451)** | LLLKPECTSLYSMLI **(SEQ ID NO: 452)** |
| LSLLPEITALYMLIQ **(SEQ ID NO: 453)** | IVLVPEITQLYLRLC **(SEQ ID NO:454)** |

| DQA1*01:02-DQB1*06:02 | DQA1*01:01-DQB1*05:01 |
|---|---|
| ML**L**N**PE**G**T**V**LY**TCAQ **(SEQ ID NO: 455)** | CH**L**C**PE**T**T**R**LY**IHST **(SEQ ID NO: 456)** |
| LLLGPEGTVLYICSQ **(SEQ ID NO: 457)** | ICLRPECTQLYKRHV **(SEQ ID NO:458)** |
| LLLGPEGTVLYICSQ **(SEQ ID NO: 457)** | CCLCPETTRLYVQST **(SEQ ID NO: 459)** |
| LLLGPEGTVLYICSQ **(SEQ ID NO: 457)** | CHLCPETTRLYIHST **(SEQ ID NO:460)** |
| LLLGPEGTVLYNCST **(SEQ ID NO: 461)** | LHLEPEDTFLYEQLC **(SEQ ID NO: 462)** |
| LLLGPEGTVLYICSQ **(SEQ ID NO: 457)** | CHLCPETTRLYIHST **(SEQ ID NO: 463)** |
| LLLGPEGTVLYICSQ **(SEQ ID NO: 457)** | CCLCPETTRLYVQST **(SEQ ID NO: 464)** |
| LLLGPEGTVLYICSQ **(SEQ ID NO: 457)** | CCLCPETTRLYVQST **(SEQ ID NO: 464)** |
| LQLMPEGTLLYVCPG **(SEQ ID NO:450)** | RCLKPEDTGLYRDIV **(SEQ ID NO: 465)** |
| LLLGPEGTVLYNCST **(SEQ ID NO: 461)** | CCLCPETTRLYVQST **(SEQ ID NO: 464)** |

### Example 15 Selective T-cell targeting for in vivo and in vitro use.

Two strategies are envisioned in which a TCEM attached to an MHC is combined with a third peptide which may be a cytotoxin or a cytokine. In the first strategy the combination is configured as TCEM-MHC-Peptide in the second it is configured as MHC- TCEM- peptide

The invention described herein identifies Treg binding peptides which are specific to tumor proteins. In one example described above these are TCEM motifs identified in HPV oncoproteins, in another they are PMEL, MART or HBV associated motifs. By identifying TCEMs which bind specifically to Tregs, the possibility is created of attaching or incorporating a cytotoxin to such a peptide which, when mounted in an MHC molecule as a pMHC, binds specifically to a T cell. This allows for the very specific depletion of T cells and, depending on the choice of TCEM, specifically depleting Tregs. The stability of the pMHC complex can be increased by adjusting the GEM amino acids to provide increased binding affinity. The MHC is selected to match that of a particular patient. The selection of cytotoxin depends on the particular disease. One group of cytotoxins are radionuclides. Among the radionuclides are alpha emitters, Auger electron emitters or positron emitters. For labeling peptides containing tyrosine, or to which tyrosine is added iodine 125 has particular ease of use and as an Auger emitter offers a very focused cytotoxic effect on the binding cell.

In the case of HPV cancers, the peptides listed in Tables 9 are examples of motifs which may be incorporated into a peptide to target Tregs.

As a particular example of this in the case of HPV type 16 and type 33, the peptide which in the natural sequence of E7 initiates at index position 13 as shown underlined below and contains the high frequency TCEMs PE~T~LY (SEQ ID NO: 487) and L~~E~T~LY (SEQ ID NO: 488). The same process can be applied to other peptides in Table 9, as well as those occurring on other strains or isolates of HPV, so this example is not limiting.
>gi|333031.E7|genpept|HPV16-E7.1| Human papillomavirus 16 (HPV16), E7 protein 
MHGDTPTLHEYMLDLQPETTDLYCYEQLNDSSEEEDEIDGPAGQAEPDRAHYNIVTFCCKCDSTLRLCV
QSTHVDIRTLEDLLMGTLGIVCPICSQKP (SEQ ID NO:465)
>gi|333049.E7|genpept|HPV33-E7.1| Human papillomavirus 33 (HPV33), E7 protein

MRGHKPTLKEYVLDLYPEPTDLYCYEQLSDSSDEDEGLDRPDGQAQPATADYYIVTCCHTCNTTVRLCV
NSTASDLRTIQQLLMGTVNIVCPTCAQQ (SEQ ID NO:466)

The peptide (for HPV 16) LDLQPETTDLYCYEQ (SEQ ID NO: 271) or (for HPV 33) LDLYPEPTDLYCYEQ (SEQ ID NO: 273) is selected for use as a T-cell target. In some instances a short extension of a few amino acids, 1-4 amino-acids, may be retained on one or both ends of this peptide to facilitate addition of labels or cytotoxins. The intercalated amino acids may be changed to enhance binding affinity for a patient allele of choice as shown above in Table 10. Such optimization has the added advantage of removing by mutation the pRB binding site motif LXCXE, thereby reducing any perceived risk associated with administration.

In one particular case iodination with I125 allows the delivery of Auger electrons. The methods of iodination are well known to those skilled in the art (See Hunter, R.M. & Greenwood, F.C. Preparation of iodine-131 labelled human growth hormone of high specific activity. Nature 194, 495-496 (1962).; Bolton, A.E. & Hunter, W.M. The labeling of proteins to high specific radioactivities by conjugation to a 125I-containing acylating agent. Biochem. J. 133, 529-539 (1973).; Garg, P.K., Archer, G.E., Bigner, D.D. & Zalutsky, M.R. Synthesis of radioiodinated N-succinimidyl iodobenzoate: optimization for use in antibody labelling. Appl. Radiat. Isot. 40, 485-490 (1989).; Wilbur, D.S. Radiohalogenation of proteins: an overview of radionuclides, labeling methods, and reagents for conjugate labeling. Bioconjugate Chem. 3, 433-470 (1992).; Preparation of N-succinimidyl 3-[*I]iodobenzoate: an agent for the indirect radioiodination of proteins. Vaidyanathan G1, Zalutsky MR. Nat Protoc. 2006;1(2):707-13.

The most common method for the radioiodination of proteins involves direct electrophilic substitution on tyrosine residues. This method has several problems with some molecules and to counter these problems, conjugation-labeling methods were developed, wherein a labeled prosthetic group was conjugated to mAbs and other proteins under mild conditions through modification of lysine e-amino groups8. N-succinimidyl 3-(4- hydroxy-3-iodophenyl) propionate, popularly known as Bolton-Hunter reagent, is perhaps the best known conjugation-labeling agent. Although the Bolton-Hunter reagent does not create a labeled tyrosine on the protein, the iodine is positioned ortho to a phenolic hydroxyl group, making it susceptible to deiodination. An alternative procedure is the synthesis of N-succinimidyl 3-iodobenzoate labeled with any iodine isotope ([*I]SIB), which is an agent used in the radioiodination of proteins and peptides, from its tin precursor N-succinimidyl 3-(tri-n-butylstannyl)benzoate. Radioiododestannylation of STB using tert-butylhydroperoxide as the oxidant gives [*I]SIB in 80% radiochemical yields. Use of [*I]SIB yields radioiodinated proteins that are more stable in vivo than those radioiodinated by the direct electrophilic method

A MHC molecule is expressed by retrovector transfection into CHO cells as both alpha and beta subunits. In the case of DR1-0101 the alpha and beta subunits are each constructed into a retrovector and alpha and beta subunits transfected sequentially into dividing CHO cells when transfected with both sub chains the CHO cells express the assembled DRB1. The retrovector transfection methods have been described elsewhere (see, e.g., US 8,703,134; 8,394,379; 7,566,447; and 20130230516). Following a recovery period the cells are subject to single cell dilution and allowed to grow back as clonal populations. Yield of the secreted MHC molecules is determined using an immunoassay and antibody to the alphachain and MHC molecules are harvested and purified. The cytotoxin-labeled affinity-optimized peptide is combined with the MHC molecule to generate a pMHC.

Patients are HLA allele matched to the MHC delivering the labeled peptide. Administration of the pMHC to the patient delivers the cytotoxin directly to the binding reaction with the T-cell.

In a variant of this method, instead of a cytotoxin the peptide is labelled with streptavidin and the allele matched patent cells added to the pMHC in vitro. T cells recognizing the TCEM in the pMHC will bind and other cells may be separated, allowing the bound cells to be enumerated.

### Example 16 Generation of a generic immunosuppressive peptide combination

In order to examine the immunosuppressive content of a randomly generated peptide mixture we proceeded as follows. In order to select four amino acids, we examined the IGHV variable region amino acid positions 37 and 38. These positions are located in the CDR1 and are a region characterized by high binding. In the 40K reference database of IGHV sequences we determined that the commonest occurring amino acids were alanine (A) Glutamine (Q), Valine (V) and Tryptophan (W). Further we determined that the ration of frequency of these at positions 37 and 38 is A(12):Q(22):V(17):W(10).

Using this ratio of amino acids we created 10,000 random 15-mer peptides. We then compared the TCEM motifs in the randomly generated set to those occurring in the naturally derived 40K database of IGHV. Approximately 6% of our random peptides were also found in the 40K database. In addition 42 also matched motifs found in heavy chain constant regions.

Table 6 shows the total occurrence of each type of motif and the frequency class to which they belong.

**Table 24 Shows the frequency class (level) and count of IGHV TCEM motifs found in 10,000 randomly generated 15mer peptides comprised of 4 amino acids.**

| TCEM IIA | | | TCEM IIB | | | TCEM I | | |
|---|---|---|---|---|---|---|---|---|
| **Frequencies** | | | **Frequencies** | | | **Frequencies** | | |
| **Level** | **Count** | **Prob** | **Level** | **Count** | **Prob** | **Level** | **Count** | **Prob** |
| 10 | 28 | 0.04643 | 12 | 24 | 0.03954 | 9 | 5 | 0.00828 |
| 12 | 11 | 0.01824 | 13 | 82 | 0.13509 | 10 | 25 | 0.04139 |
| 13 | 22 | 0.03648 | 14 | 15 | 0.02471 | 11 | 14 | 0.02318 |
| 14 | 60 | 0.09950 | 15 | 108 | 0.17792 | 12 | 25 | 0.04139 |
| 15 | 87 | 0.14428 | 16 | 378 | 0.62273 | 13 | 47 | 0.07781 |
| 16 | 395 | 0.65506 | Total | 607 | 1.00000 | 14 | 128 | 0.21192 |
| Total | 603 | 1.00000 | | | | 15 | 84 | 0.13907 |
| | | | | | | 16 | 276 | 0.45695 |
| | | | | | | Total | 604 | 1.00000 |

Therefore a total of 39 TCEM IIA (28 x FC 10+11 x FC12), 24 TCEM IIB and 69 TCEM I (comprising FC 9-12) plus 42 HC motifs were identified which would be expected to elicit immunosuppression. The sequences of these were recorded. The peptides comprising these motifs, singly or collectively could be administered as a general immunosuppressive.

It should be noted that although 4 specific amino acids were selected to generate the peptides in this example, the same approach could be applied to any small set of amino acids selected by other means and to any selected ratio of amino acids. Hence the method described in the example is not limited to the four amino acids or the particular ratio demonstrated here.

### Example 17 Modified HLA secretion

In a further variation on the expression of the HLA molecules the alpha chain or beta chain is made as a fusion with an immunoglobulin Fc region. The Fc region may be derived from IgG or IgA or IgM. In some instances the cysteine cross bridges are removed to prevent assembly of the Fc regions as dimers. The presence of the Fc region enables detection using standard immunoassays (e.g. using an anti Fc detection antibody). It further provides a site for addition of tags, cytotoxins and labels. To express Fc fusion HLA, in this example the DR1 the alpha chain is constructed as a fusion to IgG Fc and transfected into CHO cells as described above. An exemplary sequence of such a fusion is shown in SEQ ID NOs: 467 and 468.
SEQ ID NO:467 Theoretical p500X01 (eSP-DR1alpha'-G1-mVhC HC) nucleotide: 
atggccataagtggagtccctgtgctaggatttttcatcatagctgtgctgatgagcgctcaggaatcatgggctatcaaagaagaac
atgtgatcatccaggccgagttctatctgaatcctgaccaatcaggcgagtttatgtttgactttgatggtgatgagattttccatgtgg
atatggcaaagaaggagacggtctggcggcttgaagaatttggacgatttgccagctttgaggctcaaggtgcattggccaacatag
ctgtggacaaagccaacctggaaatcatgacaaagcgctccaactatactccgatcaccaatgtacctccagaggtaactgtgctca
cGaacagccctgtggaactgagagagcccaacgtcctcatctgtttcatCgacaagttcaccccaccagtggtcaatgtcacgtggc
ttcgaaatggaaaacctgtcaccacaggagtgtcagagacagtcttcctgcccagggaagaccaccttttccgcaagttccactatct
ccccttcctgccctcaactgaggacgtttacgactgcagggtggagcactggggcttggatgagcctcttctcaagcactgggagttt
gatgtgTCgaccaagggcccatcggtcttccccctggcaccctctagcaagagcacctctgggggcacagcggccctgggctgcctg
gtcaaggactacttccccgaaccggtgacggtgtcgtggaactcaggcgccctgaccagcggcgtgcacaccttcccggctgtccta
cagtcctcaggactctactccctcagcagcgtggtgaccgtgccctccagcagcttgggcacccagacctacatctgcaacgtgaatc
acaagcccagcaacaccaaggtggacaagagagttgagcccaaatcttgtgacaaaactcacacatgcccaCCGTGCCCAgc
acctgaactcctggggggaccgtcagtcttcctcttccccccaaaacccaaggacaccctcatgatctcccggacccctgaggtcaca
tgcgtggtggtggacgtgagccacgaagaccctgaggtcaagttcaactggtacgtggacggcgtggaggtgcataatgccaagac
aaagccgcgggaggagcagtacaacagcacgtaccgtgtggtcagcgtcctcaccgtcctgcaccaggactggctgaatggcaag
gagtacaagtgcaaggtctccaacaaagccctcccagcccccatcgagaaaaccatctccaaagccaaagggcagccccgagaac
cacaggtgtacaccctgcccccatcccgggaggagatgaccaagaaccaggtcagcctgacctgcctggtcaaaggcttctatccca
gcgacatcgccgtggagtgggagagcaatgggcagccggagaacaactacaagaccacgcctcccgtgctggactccgacggctc
cttcttcctctatagcaagctcaccgtggacaagagcaggtggcagcaggggaacgtcttctcatgctccgtgatgcatgaggctctg
cacaaccactacacgcagaagagcctctccctgtctccgggtaaatga
SEQ ID NO:468 Theoretical p500X01 (eSP-DR1alpha'-G1-mVhC HC) protein:

MAISGVPVLGFFIIAVLMSAQESWAIKEEHVIIQAEFYLNPDQSGEFMFDFDGDEIFHVDMAKKETVWRL
EEFGRFASFEAQGALANIAVDKANLEIMTKRSNYTPITNVPPEVTVLTNSPVELREPNVLICFIDKFTPPVVN
VTWLRNGKPVTTGVSETVFLPREDHLFRKFHYLPFLPSTEDVYDCRVEHWGLDEPLLKHWEFDVSTKGPS
VFPLAPSSKSTSGGTAALGCLVKDYFPEPVTVSWNSGALTSGVHTFPAVLQSSGLYSLSSVVTVPSSSLGTQ
TYICNVNHKPSNTKVDKRVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPIEKTI
SKAKGQPREPQVYTLPPSREEMTKNQVSLTCLVKGFYPSDIAVEWESNGQPENNYKTTPPVLDSDGSFFL
YSKLTVDKSRWQQGNVFSCSVMHEALHNHYTQKSLSLSPGK.

### Reference List

1. De Groot AS, Moise L, McMurry JA, Wambre E, Van Overtvelt L, et al. (2008) Activation of natural regulatory T cells by IgG Fc-derived peptide "Tregitopes". Blood 112: 3303-3311.
2. Rudolph MG, Stanfield RL, Wilson IA (2006) How TCRs bind MHCs, peptides, and coreceptors. Annu Rev Immunol 24: 419-466.
3. Lefranc MP, Giudicelli V, Ginestoux C, Jabado-Michaloud J, Folch G, et al. (2009) IMGT, the international ImMunoGeneTics information system. Nucleic Acids Res 37: D1006-1012.
4. Weiss S, Bogen B (1989) B-lymphoma cells process and present their endogenous immunoglobulin to major histocompatibility complex-restricted T cells. Proc Natl Acad Sci U S A 86: 282-286.
5. Chakrabarti D, Ghosh SK (1992) Induction of syngeneic cytotoxic T lymphocytes against a B cell tumor. III. MHC class I-restricted CTL recognizes the processed form(s) of idiotype. Cell Immunol 144: 455-464.
6. De Groot AS, Moise L, McMurry JA, Wambre E, Van OL, et al. (2008) Activation of natural regulatory T cells by IgG Fc-derived peptide "Tregitopes". Blood 112: 3303-3311.
7. Ahmed SS, Volkmuth W, Duca J, Corti L, Pallaoro M, et al. (2015) Antibodies to influenza nucleoprotein cross-react with human hypocretin receptor 2. Sci Transl Med 7: 294ra105.
8. Lalive PH, Neuhaus O, Benkhoucha M, Burger D, Hohlfeld R, et al. (2011) Glatiramer acetate in the treatment of multiple sclerosis: emerging concepts regarding its mechanism of action. CNS Drugs 25: 401-414.
9. Kerzel S, Rogosch T, Struecker B, Maier RF, Zemlin M (2010) IgE transcripts in the circulation of allergic children reflect a classical antigen-driven B cell response and not a superantigen-like activation. J Immunol 185: 2253-2260.
10. Davies JM, O'Hehir RE (2004) VH gene usage in immunoglobulin E responses of seasonal rhinitis patients allergic to grass pollen is oligoclonal and antigen driven. Clin Exp Allergy 34: 429-436.
11. Snow RE, Djukanovic R, Stevenson FK (1999) Analysis of immunoglobulin E VH transcripts in a bronchial biopsy of an asthmatic patient confirms bias towards VH5, and indicates local clonal expansion, somatic mutation and isotype switch events. Immunology 98: 646-651.
12. Bowers E, Scamurra RW, Asrani A, Beniguel L, MaWhinney S, et al. (2014) Decreased mutation frequencies among immunoglobulin G variable region genes during viremic HIV-1 infection. PLoS One 9: e81913.
13. Bremel RD, Homan EJ (2013) Recognition of higher order patterns in proteins: immunologic kernels. PLoS One 8: e70115.
14. Greenbaum J, Sidney J, Chung J, Brander C, Peters B, et al. (2011) Functional classification of class II human leukocyte antigen (HLA) molecules reveals seven different supertypes and a surprising degree of repertoire sharing across supertypes. Immunogenetics 63: 325-335.
15. Wang P, Sidney J, Dow C, Mothe B, Sette A, et al. (2008) A systematic assessment of MHC class II peptide binding predictions and evaluation of a consensus approach. PLoSComputBiol 4: e1000048.
16. Tholen S, Biniossek ML, Gessler AL, Muller S, Weisser J, et al. (2011) Contribution of cathepsin L to secretome composition and cleavage pattern of mouse embryonic fibroblasts. BiolChem 392: 961-971.
17. Biniossek ML, Nagler DK, Becker-Pauly C, Schilling O (2011) Proteomic identification of protease cleavage sites characterizes prime and non-prime specificity of cysteine cathepsins B, L, and S. JProteomeRes 10: 5363-5373.
18. Impens F, Colaert N, Helsens K, Ghesquiere B, Timmerman E, et al. (2010) A quantitative proteomics design for systematic identification of protease cleavage events. MolCell Proteomics 9: 2327-2333.
19. Chicz RM, Urban RG, Gorga JC, Vignali DA, Lane WS, et al. (1993) Specificity and promiscuity among naturally processed peptides bound to HLA-DR alleles. J Exp Med 178: 27-47.
20. Costantino CM, Spooner E, Ploegh HL, Hafler DA (2012) Class II MHC self-antigen presentation in human B and T lymphocytes. PLoS One 7: e29805.
21. Wattam AR, Abraham D, Dalay O, Disz TL, Driscoll T, et al. (2014) PATRIC, the bacterial bioinformatics database and analysis resource. Nucleic Acids Res 42: D581-591.
22. Bremel RD, Homan EJ (2014) Frequency Patterns of T-Cell Exposed Amino Acid Motifs in Immunoglobulin Heavy Chain Peptides Presented by MHCs. Front Immunol 5: 541.
23. Han F, Lin L, Li J, Dong SX, An P, et al. (2012) HLA-DQ association and allele competition in Chinese narcolepsy. Tissue Antigens 80: 328-335.
24. Mignot E, Lin L, Rogers W, Honda Y, Qiu X, et al. (2001) Complex HLA-DR and -DQ interactions confer risk of narcolepsy-cataplexy in three ethnic groups. Am J Hum Genet 68: 686-699.
25. Han F, Faraco J, Dong XS, Ollila HM, Lin L, et al. (2013) Genome wide analysis of narcolepsy in China implicates novel immune loci and reveals changes in association prior to versus after the 2009 H1N1 influenza pandemic. PLoS Genet 9: e1003880.
26. Drysdale SB (2013) Narcolepsy risk after A/H1N1 2009 influenza vaccination. Thorax.
27. Corti D, Voss J, Gamblin SJ, Codoni G, Macagno A, et al. (2011) A neutralizing antibody selected from plasma cells that binds to group 1 and group 2 influenza A hemagglutinins. Science 333: 850-856.
28. Ekiert DC, Friesen RH, Bhabha G, Kwaks T, Jongeneelen M, et al. (2011) A highly conserved neutralizing epitope on group 2 influenza A viruses. Science 333: 843-850.
29. Coulie PG, Brichard V, Van Pel A, Wolfel T, Schneider J, et al. (1994) A new gene coding for a differentiation antigen recognized by autologous cytolytic T lymphocytes on HLA-A2 melanomas. J Exp Med 180: 35-42.
30. Kawakami Y, Eliyahu S, Delgado CH, Robbins PF, Sakaguchi K, et al. (1994) Identification of a human melanoma antigen recognized by tumor-infiltrating lymphocytes associated with in vivo tumor rejection. Proc Natl Acad Sci U S A 91: 6458-6462.
31. Kawakami Y, Eliyahu S, Sakaguchi K, Robbins PF, Rivoltini L, et al. (1994) Identification of the immunodominant peptides of the MART-1 human melanoma antigen recognized by the majority of HLA-A2-restricted tumor infiltrating lymphocytes. J Exp Med 180: 347-352.
32. Cormier JN, Salgaller ML, Prevette T, Barracchini KC, Rivoltini L, et al. (1997) Enhancement of cellular immunity in melanoma patients immunized with a peptide from MART-1/Melan A. Cancer J Sci Am 3: 37-44.
33. Reynolds SR, Oratz R, Shapiro RL, Hao P, Yun Z, et al. (1997) Stimulation of CD8+ T cell responses to MAGE-3 and Melan A/MART-1 by immunization to a polyvalent melanoma vaccine. Int J Cancer 72: 972-976.
34. Schneider J, Brichard V, Boon T, Meyer zum Buschenfelde KH, Wolfel T (1998) Overlapping peptides of melanocyte differentiation antigen Melan-A/MART-1 recognized by autologous cytolytic T lymphocytes in association with HLA-B45.1 and HLA-A2.1. Int J Cancer 75: 451-458.
35. Bioley G, Jandus C, Tuyaerts S, Rimoldi D, Kwok WW, et al. (2006) Melan-A/MART-1-specific CD4 T cells in melanoma patients: identification of new epitopes and ex vivo visualization of specific T cells by MHC class II tetramers. J Immunol 177: 6769-6779.
36. Mahnke K, Schonfeld K, Fondel S, Ring S, Karakhanova S, et al. (2007) Depletion of CD4+CD25+ human regulatory T cells in vivo: kinetics of Treg depletion and alterations in immune functions in vivo and in vitro. Int J Cancer 120: 2723-2733.
37. Rasku MA, Clem AL, Telang S, Taft B, Gettings K, et al. (2008) Transient T cell depletion causes regression of melanoma metastases. J Transl Med 6: 12.
38. Rehermann B, Nascimbeni M (2005) Immunology of hepatitis B virus and hepatitis C virus infection. Nat Rev Immunol 5: 215-229.
39. Li J, Shi J, Ren W, Wu W, Chen Z (2014) Regulatory Role of CD4CD25 Foxp3 Regulatory T Cells on IL-17-Secreting T Cells in Chronic Hepatitis B Patients. Dig Dis Sci.
40. Han Y, Jiang ZY, Jiao LX, Yao C, Lin QF, et al. (2012) Association of human leukocyte antigen-DRB1 alleles with chronic hepatitis B virus infection in the Han Chinese of Northeast China. Mol Med Rep 5: 1347-1351.
41. Huang YW, Hu CY, Chen CL, Liao YT, Liu CJ, et al. (2009) Human leukocyte antigen-DRB1*1101 correlates with less severe hepatitis in Taiwanese male carriers of hepatitis B virus. J Med Virol 81: 588-593.
42. Kramvis A, Kew M, Francois G (2005) Hepatitis B virus genotypes. Vaccine 23: 2409-2423.
43. Burk RD, Chen Z, Van DK (2009) Human papillomaviruses: genetic basis of carcinogenicity. Public Health Genomics 12: 281-290.
44. Clifford G, Franceschi S, Diaz M, Munoz N, Villa LL (2006) Chapter 3: HPV type-distribution in women with and without cervical neoplastic diseases. Vaccine 24 Suppl 3: S3/26-34.
45. Munoz N, Castellsague X, de Gonzalez AB, Gissmann L (2006) Chapter 1: HPV in the etiology of human cancer. Vaccine 24 Suppl 3: S3/1-10.
46. Whitehead M, Ohlschlager P, Almajhdi FN, Alloza L, Marzabal P, et al. (2014) Human papillomavirus (HPV) type 16 E7 protein bodies cause tumour regression in mice. BMC Cancer 14: 367.
47. Bahrami AA, Ghaemi A, Tabarraei A, Sajadian A, Gorji A, et al. (2014) DNA vaccine encoding HPV-16 E7 with mutation in L-Y-C-Y-E pRb-binding motif induces potent anti-tumor responses in mice. J Virol Methods 206C: 12-18.
48. Munoz N, Castellsague X, de Gonzalez AB, Gissmann L (2006) Chapter 1: HPV in the etiology of human cancer. Vaccine 24 Suppl 3: S3/1-S310.

## Claims

1. A method to predict the immune response to a peptide in a target protein comprising:
establishing a reference database of T cell exposed motifs by assembling a database of reference proteins selected from a human proteome other than immunoglobulins and microorganism proteins,
wherein said T cell exposed motifs are those subsets of amino acids in a peptide which, if bound in an MHC molecule, are directed outwards and exposed to a T-cell binding to the complex of the peptide bound to an MHC molecule (pMHC),
extracting all sequential 9-mer and 15-mer peptide sequences from said reference proteins,
identifying T-cell exposed motifs in said peptide sequences by a computer implemented algorithm, wherein the configuration of amino acids in the T-cell exposed motifs comprise the 2,3,5,7,8 positions of a 15 amino acid peptide binding in a MHC-II groove which is numbered from -3 to +3, the -1,3,5,7,8 positions of a 15 amino acid peptide binding in a MHC-II groove which is numbered from -3 to +3, and the 4,5,6,7,8 positions of a 9 amino acid peptide binding in a MHC-I groove; and
categorizing said T-cell exposed motifs based on the frequency of occurrence of said T-cell exposed motifs in said reference database of reference proteins, and
determining the number of occurrences of each T-cell exposed motif in said reference database;
extracting peptide sequences from the target protein, identifying T-cell exposed motifs in said peptide sequences from the target protein by a computer implemented algorithm; and
comparing T cell exposed motifs from the target protein to the T cell exposed motifs from the reference database to determine the number of occurrence of the same T cell exposed motifs in said reference database.

2. The method of claim 1, wherein the database of reference comprises proteins from the human proteome other than immunoglobulins.

3. The method of claim 1, wherein the database of reference proteins comprises proteins from the gastrointestinal microbiome.

4. The method of claim 1, wherein said categorizing further comprises determining if a T cell exposed motif in said target protein is absent from the reference database.

5. The method of claim 1, wherein said categorizing further comprises determining whether said T-cell exposed motifs in said target protein are T cell exposed motifs which occur with less than the mean frequency of occurrence in said reference database.

6. The method of claim 1, wherein said categorizing further comprises determining whether said T-cell exposed motifs in said target protein are T cell exposed motifs which occur with greater than the mean frequency of occurrence in said reference database.

7. The method of claim 1, further comprising the step of characterizing the overall immunogenicity of said target protein by ranking of the frequency of occurrence of a plurality of said T-cell exposed motifs in said target protein.

8. The method of claim 1, further comprising determining the predicted MHC binding affinity of peptide sequences comprising T-cell exposed motifs derived from the target protein by determining the MHC allele-specific binding of the groove exposed motifs flanking each T-cell exposed motif.

9. The method of claim 1, further comprising identifying peptide sequences within said target protein that are predicted to lead to upregulation of the immune response.

10. The method of claim 1, further comprising identifying peptide sequences within said target protein that are predicted immunosuppressive T-cell epitopes.

11. The method of claim 1 further comprising designing modifications in a peptide selected from said target protein wherein said modifications are selected to produce a result selected from the group consisting of (a) a lower or higher frequency of representation of the T cell exposed motif as compared to the frequency in said reference database of reference proteins, and (b) increased or decreased MHC binding affinity of the selected peptide, and expressing a synthetic polypeptide comprising said modified peptide and purifying said synthetic polypeptide.

12. The method of claim 1, further comprising identifying peptide sequences within said target protein which have T-cell exposed motifs with determined frequency of occurrence in the reference database, and determining the predicted binding affinity to one or more MHC alleles of such peptides carrying the same T cell exposed motifs in the context of where the T-cell exposed motifs occur in the reference proteins in the reference database.

## Patentansprüche

1. Verfahren zum Vorhersagen der Immunantwort auf ein Peptid in einem Zielprotein, umfassend:
Anlegen einer Referenzdatenbank T-Zellen ausgesetzter Motive durch Zusammenstellen einer Datenbank von Referenzproteinen, die aus einem menschlichen Proteom außer Immunglobulinen und Mikroorganismusproteinen ausgewählt sind,
wobei die T-Zellen ausgesetzten Motive diejenigen Untergruppen von Aminosäuren in einem Peptid sind, die, wenn sie in einem MHC-Molekül gebunden sind, nach außen gerichtet sind und einer T-Zelle, die an den Komplex des an ein MHC-Molekül gebundenen Peptids (pMHC) bindet, ausgesetzt sind,
Extrahieren aller sequentiellen 9-mer- und 15-mer-Peptidsequenzen aus den Referenzproteinen,
Identifizieren T-Zellen ausgesetzter Motive in den Peptidsequenzen durch einen computerimplementierten Algorithmus, wobei die Konfiguration der Aminosäuren in den T-Zellen ausgesetzten Motiven die Positionen 2, 3, 5, 7, 8 eines 15-Aminosäuren-Peptids, das in einer MHC-II-Furche bindet, die von -3 bis +3 nummeriert ist, die Positionen -1, 3, 5, 7, 8 eines 15-Aminosäuren-Peptids, das in einer MHC-II-Furche bindet, die von -3 bis +3 nummeriert ist, und die Positionen 4, 5, 6, 7, 8 eines 9-Aminosäuren-Peptids, das in einer MHC-I-Furche bindet, umfasst; und
Kategorisieren der T-Zellen ausgesetzten Motive basierend auf der Häufigkeit des Vorkommens der T-Zellen ausgesetzten Motive in der Referenzdatenbank von Referenzproteinen, und
Bestimmen der Anzahl von Vorkommen jedes T-Zellen ausgesetzten Motivs in der Referenzdatenbank;
Extrahieren von Peptidsequenzen aus dem Zielprotein, Identifizieren von T-Zellen ausgesetzten Motiven in den Peptidsequenzen aus dem Zielprotein durch einen computerimplementierten Algorithmus; und
Vergleichen T-Zellen ausgesetzter Motive aus dem Zielprotein mit den T-Zellen ausgesetzten Motiven aus der Referenzdatenbank, um die Anzahl des Vorkommens der gleichen T-Zellen ausgesetzten Motive in der Referenzdatenbank zu bestimmen.

2. Verfahren nach Anspruch 1, wobei die Referenzdatenbank Proteine aus dem menschlichen Proteom, die sich von Immunglobulinen unterscheiden, umfasst.

3. Verfahren nach Anspruch 1, wobei die Datenbank von Referenzproteinen Proteine aus dem gastrointestinalen Mikrobiom umfasst.

4. Verfahren nach Anspruch 1, wobei das Kategorisieren ferner Bestimmen umfasst, ob ein T-Zellen ausgesetztes Motiv in dem Zielprotein in der Referenzdatenbank fehlt.

5. Verfahren nach Anspruch 1, wobei das Kategorisieren ferner Bestimmen umfasst, ob die T-Zellen ausgesetzten Motive in dem Zielprotein T-Zellen ausgesetzte Motive sind, die mit weniger als der mittleren Häufigkeit des Vorkommens in der Referenzdatenbank vorkommen.

6. Verfahren nach Anspruch 1, wobei das Kategorisieren ferner Bestimmen umfasst, ob die T-Zellen ausgesetzten Motive in dem Zielprotein T-Zellen ausgesetzte Motive sind, die mit mehr als der mittleren Häufigkeit des Vorkommens in der Referenzdatenbank vorkommen.

7. Verfahren nach Anspruch 1, ferner umfassend den Schritt eines Charakterisierens der Gesamtimmunogenität des Zielproteins durch Klassifizieren der Häufigkeit des Vorkommens einer Vielzahl der T-Zellen ausgesetzten Motive in dem Zielprotein.

8. Verfahren nach Anspruch 1, ferner umfassend Bestimmen der vorhergesagten MHC-Bindungsaffinität von Peptidsequenzen, die T-Zellen ausgesetzte Motive umfassen, die von dem Zielprotein abgeleitet sind, durch Bestimmen der MHC-Allel-spezifischen Bindung der furchenausgesetzten Motive, die an jedes T-Zellen ausgesetzte Motiv angrenzen.

9. Verfahren nach Anspruch 1, ferner umfassend Identifizieren von Peptidsequenzen innerhalb des Zielproteins, von denen vorhergesagt wird, dass sie zu einer Hochregulation der Immunantwort führen.

10. Verfahren nach Anspruch 1, ferner umfassend Identifizieren von Peptidsequenzen innerhalb des Zielproteins, die vorhergesagte immunsuppressive T-Zell-Epitope sind.

11. Verfahren nach Anspruch 1, ferner umfassend Entwerfen von Modifikationen in einem Peptid, das aus dem Zielprotein ausgewählt ist, wobei die Modifikationen ausgewählt sind, um ein Ergebnis zu erzeugen, das aus der Gruppe ausgewählt ist, die aus (a) einer niedrigeren oder höheren Häufigkeit der Darstellung des T-Zellen ausgesetzten Motivs im Vergleich zu der Häufigkeit in der Referenzdatenbank von Referenzproteinen und (b) einer erhöhten oder verringerten MHC-Bindungsaffinität des ausgewählten Peptids besteht, und Exprimieren eines synthetischen Polypeptids, das das modifizierte Peptid umfasst, und Reinigen des synthetischen Polypeptids.

12. Verfahren nach Anspruch 1, ferner umfassend Identifizieren von Peptidsequenzen innerhalb des Zielproteins, die T-Zellen ausgesetzte Motive mit bestimmter Häufigkeit des Vorkommens in der Referenzdatenbank aufweisen, und Bestimmen der vorhergesagten Bindungsaffinität zu einem oder mehreren MHC-Allelen solcher Peptide, die die gleichen T-Zellen ausgesetzten Motive tragen, im Zusammenhang damit, wo die T-Zellen ausgesetzten Motive in den Referenzproteinen in der Referenzdatenbank vorkommen.

## Revendications

1. Procédé de prédiction de la réponse immunitaire à un peptide dans une protéine cible comprenant :
l'établissement d'une base de données de référence de motifs exposés aux lymphocytes T en assemblant une base de données de protéines de référence sélectionnées à partir d'un protéome humain autre que les immunoglobulines et les protéines de micro-organismes,
dans lequel lesdits motifs exposés aux lymphocytes T sont ces sous-ensembles d'acides aminés dans un peptide qui, s'ils sont liés dans une molécule de CMH, sont dirigés vers l'extérieur et exposés à une liaison des lymphocytes T au complexe du peptide lié à une molécule de CMH (CMHp),
l'extraction de toutes les séquences peptidiques 9-mer et 15-mer séquentielles desdites protéines de référence,
l'identification de motifs exposés aux lymphocytes T dans lesdites séquences peptidiques par un algorithme mis en œuvre par ordinateur, dans lequel la configuration des acides aminés dans les motifs exposés aux lymphocytes T comprend les positions 2, 3, 5, 7, 8 d'un peptide de 15 acides aminés se liant dans un sillon du CMH-II qui est numéroté de -3 à +3, les positions -1, 3, 5, 7, 8 d'un peptide de 15 acides aminés se liant dans un sillon du CMH-II qui est numéroté de -3 à +3, et les positions 4, 5, 6, 7, 8 d'un peptide de 9 acides aminés se liant dans un sillon du CMH-I ; et
la catégorisation desdits motifs exposés aux lymphocytes T sur la base de la fréquence d'occurrence desdits motifs exposés aux lymphocytes T dans ladite base de données de référence de protéines de référence, et
la détermination du nombre d'occurrences de chaque motif exposé aux lymphocytes T dans ladite base de données de référence ;
l'extraction de séquences peptidiques de la protéine cible, l'identification de motifs exposés aux lymphocytes T dans lesdites séquences peptidiques de la protéine cible par un algorithme mis en œuvre par ordinateur ; et
la comparaison des motifs exposés aux lymphocytes T de la protéine cible aux motifs exposés aux lymphocytes T de la base de données de référence pour déterminer le nombre d'occurrences des mêmes motifs exposés aux lymphocytes T dans ladite base de données de référence.

2. Procédé de la revendication 1, dans lequel la base de données de référence comprend des protéines du protéome humain autres que les immunoglobulines.

3. Procédé de la revendication 1, dans lequel la base de données de protéines de référence comprend des protéines du microbiome gastro-intestinal.

4. Procédé de la revendication 1, dans lequel ladite catégorisation comprend en outre le fait de déterminer si un motif exposé aux lymphocytes T dans ladite protéine cible est absent de la base de données de référence.

5. Procédé de la revendication 1, dans lequel ladite catégorisation comprend en outre la détermination du fait que lesdits motifs exposés aux lymphocytes T dans ladite protéine cible sont des motifs exposés aux lymphocytes T qui se produisent avec une fréquence d'occurrence inférieure à la moyenne dans ladite base de données de référence.

6. Procédé de la revendication 1, dans lequel ladite catégorisation comprend en outre la détermination du fait que lesdits motifs exposés aux lymphocytes T dans ladite protéine cible sont des motifs exposés aux lymphocytes T qui se produisent avec une fréquence d'occurrence supérieure à la moyenne dans ladite base de données de référence.

7. Procédé de la revendication 1, comprenant en outre l'étape de caractérisation de l'immunogénicité globale de ladite protéine cible en classant la fréquence d'occurrence d'une pluralité desdits motifs exposés aux lymphocytes T dans ladite protéine cible.

8. Procédé de la revendication 1, comprenant en outre la détermination de l'affinité de liaison au CMH prédite de séquences peptidiques comprenant des motifs exposés aux lymphocytes T dérivés de la protéine cible en déterminant la liaison spécifique aux allèles du CMH des motifs exposés au sillon qui flanquent chaque motif exposé aux lymphocytes T.

9. Procédé de la revendication 1, comprenant en outre l'identification de séquences peptidiques au sein de ladite protéine cible qui sont prédites comme devant conduire à une régulation positive de la réponse immunitaire.

10. Procédé de la revendication 1, comprenant en outre l'identification de séquences peptidiques au sein de ladite protéine cible qui sont des épitopes de lymphocytes T immunosuppresseurs prédits.

11. Procédé de la revendication 1, comprenant en outre la conception de modifications dans un peptide sélectionné à partir de ladite protéine cible, dans lequel les modifications sont sélectionnées pour produire un résultat sélectionné dans le groupe constitué par (a) une fréquence inférieure ou supérieure de représentation du motif exposé aux lymphocytes T par rapport à la fréquence dans ladite base de données de référence de protéines de référence, et (b) une affinité de liaison au CMH accrue ou réduite du peptide sélectionné, et l'expression d'un polypeptide synthétique comprenant ledit peptide modifié et la purification dudit polypeptide synthétique.

12. Procédé de la revendication 1, comprenant en outre l'identification de séquences peptidiques au sein de ladite protéine cible qui ont des motifs exposés aux lymphocytes T avec une fréquence d'occurrence déterminée dans la base de données de référence, et la détermination de l'affinité de liaison à un ou plusieurs allèles CMH prédite de tels peptides portant les mêmes motifs exposés aux lymphocytes T dans le contexte de l'endroit où les motifs exposés aux lymphocytes T se produisent dans les protéines de référence dans la base de données de référence.
